# EUROPÄISCHE PATENTSCHRIFT

(11) **EP 3 223 788 B1**
(45) Veröffentlichungstag und Bekanntmachung des Hinweises auf die Patenterteilung: **24.04.2019**
(21) Anmeldenummer: 15780858.5
(22) Anmeldetag: 14.10.2015
(51) Int. Cl.: A61K 8/49, A61Q 5/06

(54) **MITTEL ZUR FARBVERÄNDERUNG VON KERATINISCHEN FASERN, ENTHALTEND MINDESTENS EINEN DIMEREN, RINGVERGBRÜCKTEN AZOFARBSTOFF**
AGENTS FOR DYEING KERATIN FIBRES, CONTAINING AT LEAST ONE DIMERIC, RING-BRIDGED AZO DYE
AGENT DE COLORATION DE FIBRES KÉRATINIQUES, CONTENANT AU MOINS UN COLORANT AZOÏQUE DIMÈRE À PONTAGE PAR COMPOSÉ CYCLIQUE

(30) Priorität: 25.11.2014 DE 102014223937
(43) Veröffentlichungstag der Anmeldung: 04.10.2017
(73) Patentinhaber: Henkel AG & Co. KGaA, 40589 Düsseldorf (DE)
(72) Erfinder: GEBERT-SCHWARZWÄLDER, Antje, 41469 Neuss (DE); GIESA, Helmut, 40670 Meerbusch (DE); MOCH, Melanie, 41542 Dormagen (DE)
(86) Internationale Anmeldenummer: PCT/EP2015/073776
(87) Internationale Veröffentlichungsnummer: WO 2016/083013

(56) Entgegenhaltungen:
- EP-A1- 0 970 687
- EP-A1- 1 609 456
- FR-A1- 2 825 622
- GB-A- 910 121
- GB-A- 1 186 753

## Beschreibung

Die vorliegende Erfindung betrifft Mittel zur Farbveränderung von menschlichen Haaren, die (a) mindestens einen dimeren, ringverbrückten Azofarbstoff einer speziellen Formel (I) und (b) mindestens ein Tensid.enthalten.

Für das Färben von keratinischen Fasern kommen im allgemeinen entweder direktziehende Farbstoffe oder Oxidationsfarbstoffe zur Anwendung. Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. H₂O₂, was in einigen Fällen Schädigungen der Faser zur Folge haben kann. Des Weiteren können einige Oxidationsfarbstoffvorprodukte bzw. bestimmte Mischungen von Oxidationsfarbstoffvorprodukten bei Personen mit empfindlicher Haut sensibilisierend wirken. Direktziehende Farbstoffe werden unter schonenderen Bedingungen appliziert. Ihr Nachteil liegt jedoch darin, dass die Färbungen häufig nur über unzureichende Echtheitseigenschaften verfügen.

Abhängig vom gewünschten Farbergebnis setzt der Fachmann direktziehende Farbstoffe unterschiedlicher Farbstoffklassen ein. Die aus dem Stand der Technik bekannten direktziehenden Farbstoffe gehören beispielsweise zur Klasse der Nitrofarbstoffe, der Anthrachinonfarbstoffe, der Azofarbstoffe, der Triarylmethanfarbstoffe oder der Methinfarbstoffe. Alle diese Farbstoffklassen sollten für die Anwendung im Kosmetikbereich ein bestimmtes Anforderungsprofil erfüllen. So sollten direktziehende Farbstoffe ein intensives Färbeergebnis liefern und möglichst gute Echtheitseigenschaften besitzen. Durch Umwelteinflüsse sollte das mit direktziehenden Farbstoffen erhaltene Farbergebnis möglichst wenig beeinflusst werden, d.h. die Farbstoffe sollten beispielsweise eine gute Waschechtheit, Lichtechtheit und Reibechtheit besitzen. Auch chemische Einflüsse, welchen die keratinischen Fasern nach dem Färbeprozess ausgesetzt sein können (wie beispielsweise Dauerwellen), sollten das Farbergebnis möglichst wenig verändern.

Um gleichzeitig mit der Färbung auch eine Aufhellung zu erreichen, sollten die direktziehenden Farbstoffe nach Möglichkeit auch mit den bei Blondierverfahren üblicherweise eingesetzten Oxidationsmitteln (wie z.B. Wasserstoffperoxid und/oder Persulfate) kompatibel sein.

Für die starke Aufhellung von dunklen Haaren wird nicht nur Wasserstoffperoxid allein, sondern eine Kombination aus Wasserstoffperoxid und Persulfaten (z.B. Ammoniumpersulfat, Kaliumpersulfat und/oder Natriumpersulfat) eingesetzt. Soll also dunkles Haar in einem Schritt stark aufgehellt und gleichzeitig in einem leuchtenden Farbton gefärbt werden, ist der Einsatz einer Mischung aus Wasserstoffperoxid, Persulfaten und einem direktziehenden Farbstoff von Vorteil. Obwohl dem Fachmann zum Färben von Haaren viele intensiv färbende direktziehende Farbstoffe bekannt sind, so kennt er doch nur eine sehr begrenzte Auswahl an Farbstoffen, die die starken oxidativen Bedingungen, wie sie eine Mischung der oben genannten Oxidationsmittel darstellt, ohne Zersetzung überstehen. Zudem weisen die aus dem Stand der Technik bekannten oxidationsstabilen Farbstoffe im Hinblick auf ihre übrigen Echtheitseigenschaften gravierende Nachteile auf.

Für das gleichzeitige Färben und starke Aufhellen von Haaren besteht damit nach wie vor ein Bedarf an Farbstoffen mit hoher Stabilität gegenüber starken Oxidationsmitteln. Auch unter diesen extremen Anwendungsbedingungen sollen diese Farbstoffe ihre positiven Echtheits- und Färbeeigenschaften nicht verlieren.

Es hat sich gezeigt, dass leuchtende und intensive Färbungen insbesondere mit kationischen direktziehenden Farbstoffen erzeugt werden können. Kationische Farbstoffe zeichnen sich oft durch eine besonders hohe Affinität zu Keratinfasern aus, was auf die Wechselwirkungen der positiven Ladungen der Farbstoffe mit negativ geladenen Strukturbestandteilen der keratinischen Fasern zurückgeführt werden kann. Daher lassen sich mit kationischen Farbstoffen oftmals besonders intensive Färbungen erzielen.

Aus dem Stand der Technik hinlänglich bekannte monomere kationische Azofarbstoffe sind beispielsweise die Vertreter Basic Orange 31 (Alternativname: 2-[(4-Aminophenyl)azo]-1,3-dimethyl-1H-imidazolium chloride, CAS-Nr. 97404-02-9) und Basic Red 51 (Alternativname: 2-[((4-Dimethylamino)phenyl)azo]-1,3-dimethyl-1H-imidazolium chlorid, CAS-Nr. 77061-58-6).

Beide Farbstoffe färben keratinischen Fasern mit herausragender Farbintensität in orange bis roten Nuancenbereich. Es besteht weiterhin auch noch Bedarf an direktziehenden Blaufarbstoffen, welche mit diesen beiden Farbstoffen in optimaler Weise kompatibel sind.

GB 910121 A offenbart dimere, kationische Azofarbstoffe, unter auch heterozyklisch verbrückt sein können, zur Färbung von Textilmaterialien.

GB 1186753 A betrifft dimere, heterozyklisch verbrückte Azofarbstoffe zur Färbung von Materialien auf Basis von Polyacrylonitrilen.

FR 2825622 A1 beansprucht Färbemittel zu Färbung von keratinischen Fasern, die bestimmte dimere Farbstoffe enthalten.

EP 1609456 A1 beschäftigt sich mit dimeren Farbstoffen des Typs A-L-B, wobei die beiden Einheiten A und B jeweils eine Arylazoimidazoliumgruppe darstellen und der Linker L mindestens eine kationische Ladung trägt.

Aufgabe der vorliegenden Anmeldung ist es daher, Färbemittel für keratinische Fasern, insbesondere menschliche Haare, bereitzustellen, die hinsichtlich der Farbtiefe und der Echtheitseigenschaften, wie insbesondere Licht-, Reib- und Waschechtheit sowie Schweiß- und Kaltwellechtheit, gute anwendungstechnische Eigenschaften besitzen. Im Falle der gleichzeitigen Anwendung mit Oxidationsfarbstoffen und/oder Oxidationsmitteln sollen die direktziehenden Farbstoffe eine hohe Stabilität gegen Wasserstoffperoxid und andere Oxidationsmittel aufweisen und ihre positiven Echtheits- und Färbeeigenschaften nicht verlieren. Zudem sollten möglichst leuchtende und intensive Färbungen erzielt werden.

Darüber hinaus sollen die vorgenannten Farbstoffe auch besonders gut mit den kationischen Azofarbstoffen Basic Orange 31 und Basic Red 51 kompatibel sein.

Es konnte nun gefunden werden, dass die Anwendung von dimeren dikationischen Azofarbstoffen der allgemeinen Formel (I) in kosmetischen Farbveränderungsmitteln auf keratinischen Fasern zu besonders intensiven und leuchtenden Färbungen führt.

Ein erster Gegenstand der vorliegenden Erfindung ist ein Mittel zur Farbveränderung von menschlichen Haaren, enthaltend in einem kosmetischen Träger
(a) mindestens einen direktziehenden Farbstoff der Formel (I), 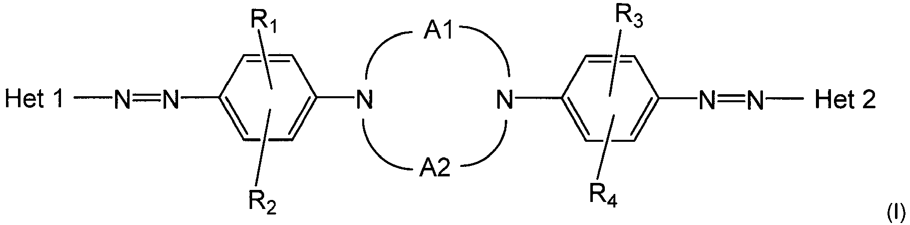 wobei
   - Het1, Het2: unabhängig voneinander für eine der Strukturen (II), (III), (IV), (V), (VI) oder (VII) stehen,

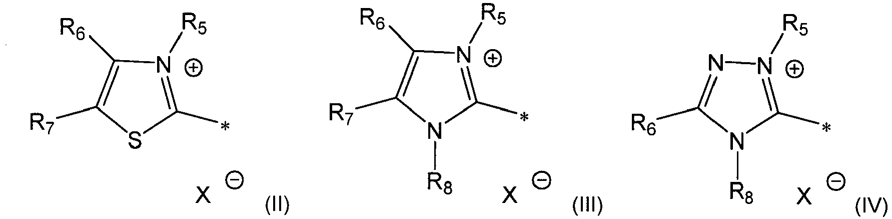

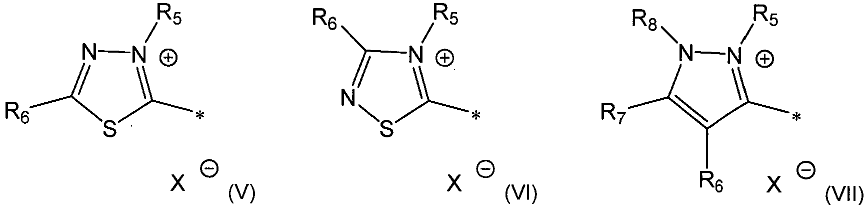

   - R1, R3: unabhängig voneinander für ein Wasserstoffatom, eine C₁-C₆-Alkylgruppe, ein Halogenatom aus der Gruppe Chlor, Brom, Fluor oder Jod, für eine C₁-C₆-Alkoxygruppe oder für eine Nitrogruppe stehen,
   - R2, R4: unabhängig voneinander für ein Wasserstoffatom, eine C₁-C₆-Alkylgruppe, ein Halogenatom aus der Gruppe Chlor, Brom, Fluor oder Jod, für eine C₁-C₆-Alkoxygruppe oder für eine Nitrogruppe stehen,
   - R5, R8: jeweils unabhängig voneinander für eine C₁-C₆-Alkylgruppe oder für eine C₂-C₆-Alkenylgruppe stehen,
   - R6, R7: jeweils unabhängig voneinander für ein Wasserstoffatom, eine C₁-C₆-Alkyl-gruppe, ein Halogenatom aus der Gruppe Chlor, Brom, Fluor oder Jod, für eine C₁-C₆-Alkoxygruppe oder für eine Nitrogruppe stehen,
   - A1, A2: unabhängig voneinander für eine Gruppierung der Formeln (VIII) oder (IX) stehen,

   ∗-(CH₂)n-∗ _{(VIII)}

   ∗-(CH₂)m-O-(CH₂)p-∗ _{(IX)}
   - n: für eine ganze Zahl von 2 bis 6 steht,
   - m, p: jeweils unabhängig voneinander für die Zahlen 2 oder 3 stehen,
   - X-: für ein physiologisch verträgliches Anion, bevorzugt aus der Gruppe Chlorid, Bromid, Iodid, Methylsulfat, Methylsulfonat, p-Toluolsulfonat, Acetat, Hydrogen-sulfat, ½ Sulfat oder ½ Tetrachlorozinkat, steht und
(b) mindestens ein Tensid.

Unter keratinischen Fasern, keratinhaltigen Fasern oder Keratinfasern sind Pelze, Wolle, Federn und insbesondere menschliche Haare zu verstehen.

Der erfindungsgemäß verwendete Begriff "Farbveränderung von Keratinfasern" umfasst jedwede Form der Farbveränderung der Fasern. Umfasst sind insbesondere die unter den Begriffen Tönung, Blondierung, Mattierung, oxidativer Färbung, semipermanenter Färbung, permanenter Färbung sowie temporärer Färbung umfassten Farbveränderungen. Explizit mit umfasst sind erfindungsgemäß Farbveränderungen, die ein helleres Farbergebnis im Vergleich zur Ausgangsfarbe aufweisen, wie beispielsweise färbende Blondierungen. Unter einer färbenden Blondierung wird die simultane Aufhellung und Färbung der keratinischen Fasern verstanden, die durch den gleichzeitigen Einsatz von Oxidationsmittel und Farbstoff im Farbänderungsmittel erzielt werden kann.

Die erfindungsgemäßen Mittel enthalten die direktziehenden Farbstoffe der Formel (I) und die Tenside (b) in einem kosmetischen Träger. Dieser kosmetische Träger ist bevorzugt wässrig, alkoholisch oder wässrig-alkoholisch. Zum Zwecke der Haarbehandlung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch möglich, zur Lagerung eine pulverförmige oder auch tablettenförmige Formulierung bereitzustellen. Diese wird dann vor der Anwendung in einem wässrigen Lösungsmittel oder mit organischen Lösungsmitteln bzw. mit Gemischen aus Wasser und organischen Lösungsmitteln unter Erhalt der Anwendungsmischung vermengt. Ein wässriger Träger enthält im Sinne der Erfindung mindestens 40 Gew.-%, insbesondere mindestens 50 Gew.-% Wasser. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Bevorzugte erfindungsgemäße Mittel sind dadurch gekennzeichnet, dass sie zusätzlich ein nichtwässriges Lösungsmittel enthalten, wobei bevorzugte erfindungsgemäße Mittel das Lösungsmittel in einer Konzentration von 0,1 bis 30 Gew.-%, bevorzugt in einer Konzentration von 1 bis 20 Gew.-%, ganz besonders bevorzugt in einer Konzentration von 2 bis 10 Gew.-%, jeweils bezogen auf das Mittel, enthalten.

Als wesentlichen Inhaltsstoff (a) enthalten die erfindungsgemäßen Mittel mindestens einen direktziehenden Farbstoff der Formel (I).

Die Substituenten R1 bis R8 der Verbindungen der Formel (I) sind nachstehend beispielhaft erläutert: Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine C₂-C₆-Alkenylgruppe sind Vinyl, Allyl, But-2-enyl, But-3-enyl sowie Isobutenyl, bevorzugte C₂-C₆-Alkenylreste sind Vinyl und Allyl. Halogenatome sind ausgewählt aus der Gruppe Chlor, Brom, Fluor und/oder Jod, hierbei sind Chlor und Brom besonders bevorzugt. Beispielhaft für eine C₁-C₆-Alkoxygruppe können die Methoxy-, die Ethoxy- und die Propoxygruppe genannt werden. Unter einer Nitrogruppe ist eine Gruppierung -NO₂ zu verstehen.

Jeder direktziehende Farbstoff der Formel (I) trägt zwei kationische, heterozyklische Endgruppen Het 1 und Het 2, die unabhängig voneinander aus den Gruppierungen der Formeln (II) bis (VII) ausgewählt sein können

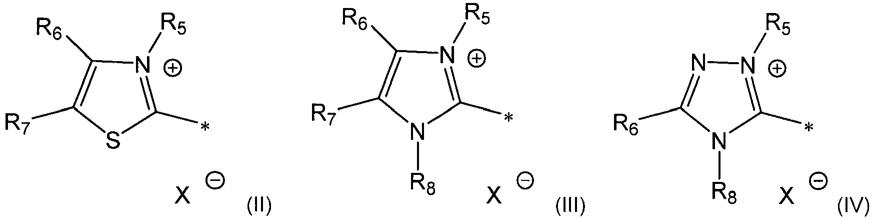

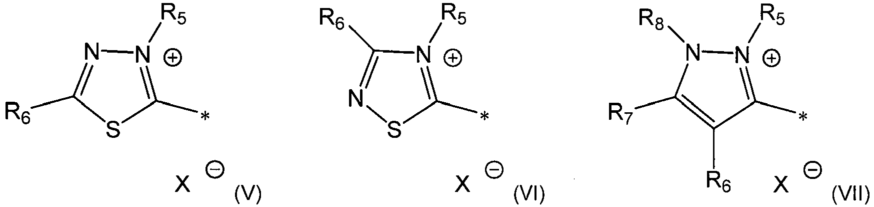

Formel (II) stellt eine kationische Thiazoliumendgruppe dar.

Formel (III) stellt eine kationische Imidazoliumendgruppe dar.

Formel (IV) stellt eine kationische, 1,2,4-Triazoliumendgruppe dar.

Formel (V) stellt eine kationische 1,3,4-Thiadiazoliumendgruppe dar.

Formel (VI) stellt eine kationische 1,2,4-Thiadiazoliumendgruppe dar.

Formel (VII) stellt eine kationische Pyrazoliumendgruppe dar.

In den Formeln (II) bis (VII) ist jeweils eine Position mit einem Stern markiert, welche die Verknüpfungsstelle der heterozyklischen Endgruppe Het 1 bzw. Het 2 mit der Azo-Gruppe in der Formel (I) angibt. Die heterozyklischen Endgruppen Het 1 und Het 2 können in den Farbstoffen der Formel (I) gleich oder verschieden sein. Reinere Färbungen werden erhalten, wenn Het 1 und Het 2 in einem Farbstoff der Formel (I) gleich sind.

Durch die Auswahl verschiedener Endgruppen Het 1 und Het 2 kann das Absorptionsspektrum des Farbstoffes und demnach auch die Färbung des Farbstoffes beeinflusst werden.

Wenn intensive Färbungen im Blauviolettbereich und insbesondere im Blaubereich gewünscht werden, ist es von Vorteil, wenn Het 1 und Het 2 für heterozyklische Endgruppen der Formel (II) stehen.

Sollen brillante Färbungen im Rotbereich erzielt werden, ist es hingegen bevorzugt, wenn Het 1 und Het 2 aus den heterozyklischen Endgruppen der Formeln (III) und/oder (IV) ausgewählt werden.

Darüber hinaus lassen sich mit Farbstoffen der Formel (I), bei welchen Het 1 und Het 2 für eine Gruppe ausgewählt aus den Formeln (II), (III) oder (IV) stehen, besonders reine und leuchtende Farbtöne erzeugen. Aus diesem Grund ist es besonders bevorzugt, wenn das erfindungsgemäße Mittel (a) mindestens einen direktziehenden Farbstoff der allgemeinen Formel (I) enthält, in der

Het1 und Het2 unabhängig voneinander für eine der Strukturen (II), (III) oder (IV) stehen

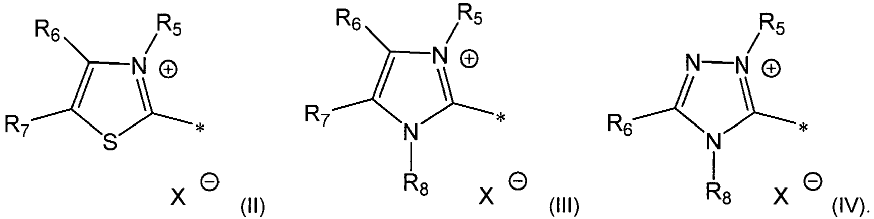

In einer besonders bevorzugten Ausführungsform ist ein erfindungsgemäßes Mittel dadurch gekennzeichnet, dass es (a) mindestens einen direktziehenden Farbstoff der allgemeinen Formel (I) enthält, in der
Het1, Het2 unabhängig voneinander für eine der Strukturen (II), (III) oder (IV) stehen

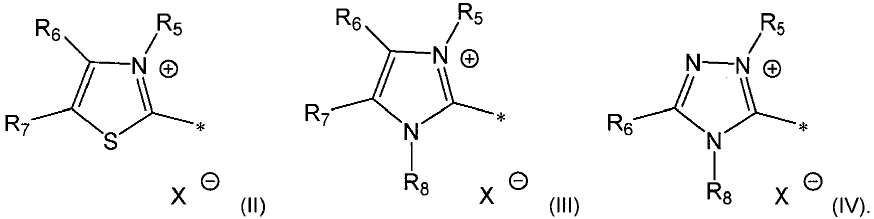

In der Formel (I) geben die Reste R1 bis R4 die Substituenten an den beiden Phenylringen des direktziehenden Farbstoffes an.

Hierbei können R1 und R3 unabhängig voneinander für ein Wasserstoffatom, eine C₁-C₆-Alkylgruppe, ein Halogenatom aus der Gruppe Chlor, Brom, Fluor oder Jod, für eine C₁-C₆-Alkoxygruppe oder für eine Nitrogruppe stehen. Prinzipiell können R1 und R3 in dem Farbstoff der Formel (I) gleich oder verschieden sein. Es ist jedoch bevorzugt wenn R1 und R3 in jedem Farbstoff der Formel (I) gleich sind.

In einer besonders bevorzugten Ausführungsform ist ein erfindungsgemäßes Mittel dadurch gekennzeichnet, dass es (a) mindestens einen direktziehenden Farbstoff der allgemeinen Formel (I) enthält, in der
- R1, R3: unabhängig voneinander für ein Wasserstoffatom, eine C₁-C₆-Alkylgruppe oder für eine C₁-C₆-Alkoxygruppe stehen.

Die Reste R2 und R4 können unabhängig voneinander für ein Wasserstoffatom, eine C₁-C₆-Alkylgruppe, ein Halogenatom aus der Gruppe Chlor, Brom, Fluor oder Jod, für eine C₁-C₆-Alkoxygruppe oder für eine Nitrogruppe stehen. Prinzipiell können R2 und R4 in dem Farbstoff der Formel (I) gleich oder verschieden sein. Es ist jedoch bevorzugt wenn R2 und R4 in jedem Farbstoff der Formel (I) gleich sind.

In einer besonders bevorzugten Ausführungsform ist ein erfindungsgemäßes Mittel dadurch gekennzeichnet, dass es (a) mindestens einen direktziehenden Farbstoff der allgemeinen Formel (I) enthält, in der
- R2, R4: unabhängig voneinander für ein Wasserstoffatom, eine C₁-C₆-Alkylgruppe oder für eine C₁-C₆-Alkoxygruppe, besonders bevorzugt für ein Wasserstoffatom, stehen.

In einer ganz besonders bevorzugten Ausführungsform ist ein erfindungsgemäßes Mittel deshalb dadurch gekennzeichnet, dass es (a) mindestens einen direktziehenden Farbstoff der allgemeinen Formel (I) enthält, in der
R1 für ein Wasserstoffatom, für eine Methylgruppe oder für eine Methoxygruppe steht,
R2 für ein Wasserstoffatom steht,
R3 für ein Wasserstoffatom, für eine Methylgruppe oder für eine Methoxygruppe steht und
R4 für ein Wasserstoffatom steht.

Die heterozyklischen Endgruppen Het 1 und Het 2 der Formeln (II) bis (VII) tragen die Reste R5, R6, R7 und/oder R8.

Die Reste R5 und R8 sind jeweils an ein N-Atom der heterozyklischen Endgruppe gebunden. Hierbei können die Reste R5 und R8 jeweils unabhängig voneinander für eine C₁-C₆-Alkylgruppe oder für eine C₂-C₆-Alkenylgruppe stehen. Es ist bevorzugt, wenn R5 und R8 unabhängig voneinander für eine C1-C6-Alkylgruppe, besonders bevorzugt für eine Methylgruppe oder eine Ethylgruppe, stehen.

Die Reste R6 und R7 können jeweils unabhängig voneinander für ein Wasserstoffatom, eine C₁-C₆-Alkylgruppe, ein Halogenatom aus der Gruppe Chlor, Brom, Fluor oder Jod, für eine C₁-C₆-Alkoxygruppe oder für eine Nitrogruppe stehen. Es ist bevorzugt, wenn R8 und R9 jeweils unabhängig voneinander für ein Wasserstoffatom, eine C₁-C₆-Alkylgruppe, oder für eine C₁-C₆-Alkoxygruppe stehen.

In einer weiteren besonders bevorzugten Ausführungsform ist ein erfindungsgemäßes Mittel deshalb dadurch gekennzeichnet, dass es (a) mindestens einen direktziehenden Farbstoff der allgemeinen Formel (I) enthält, in der
- R5, R8: jeweils unabhängig voneinander für eine C₁-C₆-Alkylgruppe stehen, und
- R6, R7: jeweils unabhängig voneinander für ein Wasserstoffatom, eine C₁-C₆-Alkyl-gruppe, oder für eine C₁-C₆-Alkoxygruppe stehen.

In einer ganz besonders bevorzugten Ausführungsform ist ein erfindungsgemäßes Mittel deshalb dadurch gekennzeichnet, dass es (a) mindestens einen direktziehenden Farbstoff der allgemeinen Formel (I) enthält, in der
- R5, R8: jeweils unabhängig voneinander für eine C₁-C₆-Alkylgruppe stehen, und
- R6, R7: jeweils unabhängig voneinander für ein Wasserstoffatom oder für eine C₁-C₆-Alkylgruppe stehen.

Die erfindungsgemäßen dimeren direktziehenden Farbstoffe der allgemeinen Formel (I) umfassen weiterhin die Gruppierungen A1 und A2. Beide Gruppierungen A1 und A2 sind jeweils mit den beiden Stickstoffatomen verbunden, die an die beiden Phenylruppen der Formel (I) gebunden sind. Zusammen mit den beiden Stickstoffatomen bilden die beiden Gruppierungen A1 und A2 somit einen Heterozyklus. Die beiden Gruppierungen A1 und A2 stehen unabhängig voneinander für eine der Gruppierungen der Formeln (VIII) oder (IX).

∗-(CH₂)n-∗ (VIII)

∗-(CH₂)m-O-(CH₂)p-∗ (IX)

In der Formel (VIII) steht n für eine ganze Zahl von 2 bis 3.

In der Formel (IX) stehen m und p unabhängig voneinander für die Zahlen 2 oder 3.

Besonders intensive Färbungen konnten mit einem direktziehenden Farbstoff der allgemeinen Formel (I) erhalten werden, bei dem A1 und A2 beide für eine Gruppierung der Formel (VIII) stehen, wobei n jeweils für die Zahlen 2 oder 3, besonders bevorzugt für die Zahl 2, steht.

In einer besonders bevorzugten Ausführungsform ist ein erfindungsgemäßes Mittel deshalb dadurch gekennzeichnet, dass es (a) mindestens einen direktziehenden Farbstoff der allgemeinen Formel (I) enthält, in der
- A1, A2: beide für eine Gruppierung der Formel (VIII) stehen,

∗-(CH₂)n-∗ (VIII),

wobei
- n: für die Zahlen 2 oder 3, bevorzugt für die Zahl 2, steht.

Prinzipiell können A1 und A2 in jedem direktziehenden Farbstoff der Formel (I) gleich oder verschieden sein, bevorzugt sind A1 und A2 in jedem direktziehenden Farbstoff der Formel (I) jedoch gleich. Stehen A1 und A2 beide für eine Gruppierung der Formel (VIII) mit n gleich 2, so handelt es sich bei der verbrückenden Einheit aus A1, A2 und den beiden N-Atomen um eine Piperazin-Gruppe. Farbstoffe der Formel (I), welche eine Piperazin-Gruppe als heterozyklische verbindende Einheit
besitzen, zeigen ein besonders gutes Farbaufzugsvermögen auf die keratinischen Fasern.

Bei den erfindungsgemäßen Farbstoffen der Formel (I) handelt es sich um dimere Azofarbstoffe, die zweifach positiv geladen sind. Die beiden positiven Ladungen werden jeweils durch die anionschen Gegenionen X (der Struktureinheiten (II) bis (VII)) neutralisiert. Hierbei ist der dikationische organische Teil für die Färbung der Keratinfasern verantwortlich. Die Gegenionen X dienen lediglich der Wahrung der Elektroneutralität, so dass die genaue Natur der Gegenionen X für die Erzielung des gewünschten Farbergebnisses keine wesentliche Rolle spielt. Da der Farbstoff in einem kosmetischen Mittel eingesetzt wird, müssen die Gegenionen X physiologisch verträglich sein. Physiologisch verträglich bedeutet in diesem Zusammenhang zum Einsatz im kosmetischen Mittel (d.h. zur Anwendung auf dem menschlichen Haar und der menschlichen Haut) geeignet. Bei den Gegenionen X handelt es sich jeweils um physiologisch verträgliche Anionen, bevorzugt aus der Gruppe Chlorid, Bromid, lodid, Methylsulfat, Methylsulfonat, p-Toluolsulfonat, Acetat, Hydrogensulfat, ½ Sulfat oder ½ Tetrachlorozinkat.

Unter Chlorid wird ein Anion Cl⁻ verstanden. Unter Bromid wird ein Anion Br verstanden. Unter lodid wird ein Anion J⁻ verstanden. Unter Methylsulfat wird ein Anion H₃COSO₄⁻ verstanden.

Unter Methylsulfonat wird ein Anion H₃CSO₃⁻ verstanden. Unter p-Toluolsulfonat wird ein Anion H₃C(C₆H₄)SO₃⁻ verstanden. Unter Acetat wird ein Anion H₃CCOO⁻ verstanden. Unter Hydrogensulfat wird ein Anion HSO₄⁻ verstanden.

Unter ½ Sulfat wird ein halbes Äquivalent des doppelt negativ geladenen Anions SO₄²⁻ verstanden. Unter ½ Tetrachlorozinkat wird ein halbes Äquivalent des doppelt negativ geladenen Anions ZnCl₄²⁻ verstanden. Bei Sulfat und Tetrachlorozinkat ist es demzufolge ebenfalls möglich und auch erfindungsgemäß, wenn der dikationische Farbstoff der Formel (I) durch ein SO₄²⁻ Ion bzw. durch ein ZnCl₄²⁻ Ion neutralisiert wird.

Aus Gründen der synthetischen Zugänglichkeit ist es bevorzugt, wenn die beiden Gegenionen X- der Struktureinheiten Het 1 und Het 2 gleich sind.

In einer weiteren bevorzugten Ausführungsform ist ein Mittel zur Farbveränderung von keratinischen Fasern dadurch gekennzeichnet, dass es mindestens eine Verbindung der allgemeinen Formel (I) enthält, die ausgewählt ist aus
- Salzen des 3-Methyl-2-{2-[4-(4-{4-[2-(3-methyl-1,3-thiazol-3-ium-2-yl)diazen-1-yl]phenyl}piperazin-1-yl)phenyl]diazen-1-yl}-1,3-thiazol-3-iums 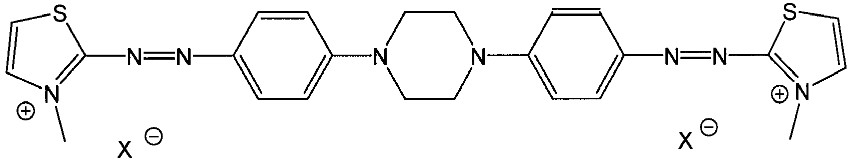
- Salzen des 3-Ethyl-2-{2-[4-(4-{4-[2-(3-ethyl-1,3-thiazol-3-ium-2-yl)diazen-1-yl]phenyl}piperazin-1-yl)phenyl]diazen-1-yl}-1,3-thiazol-3-iums 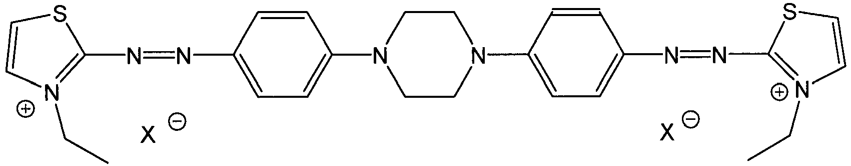
- Salzen des 3-Methyl-2-{2-[3-methyl-4-(4-{2-methyl-4-[2-(3-methyl-1,3-thiazol-3-ium-2-yl)diazen-1-yl]phenyl}piperazin-1-yl)phenyl]diazen-1-yl}-1,3-thiazol-3-iums 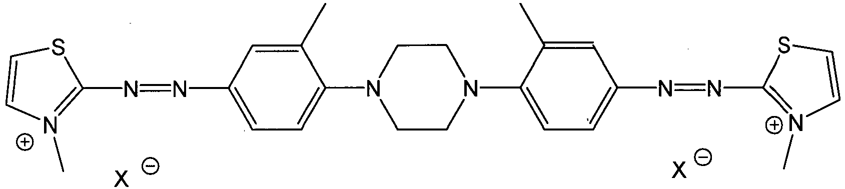
- Salzen des 3-Ethyl-2-{2-[4-(4-{4-[2-(3-ethyl-1,3-thiazol-3-ium-2-yl)diazen-1-yl]-2-methylphenyl}piperazin-1-yl)-3-methylphenyl]diazen-1-yl}-1,3-thiazol-3-iums 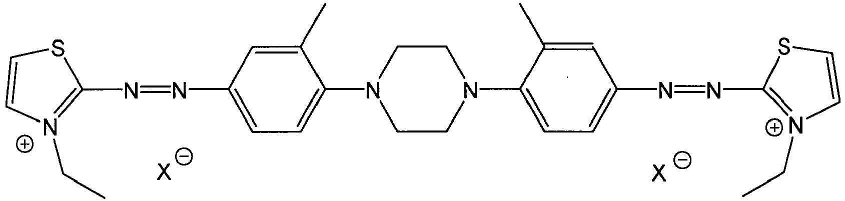
- Salzen des 3-Methyl-2-{2-[2-methyl-4-(4-{3-methyl-4-[2-(3-methyl-1,3-thiazol-3-ium-2-yl)diazen-1-yl]phenyl}piperazin-1-yl)phenyl]diazen-1-yl}-1,3-thiazol-3-iums 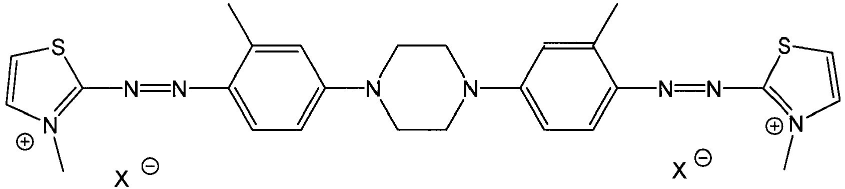
- Salzen des 3-Ethyl-2-{2-[4-(4-{4-[2-(3-ethyl-1,3-thiazol-3-ium-2-yl)diazen-1-yl]-3-methylphenyl}piperazin-1-yl)-2-methylphenyl]diazen-1-yl}-1,3-thiazol-3-iums 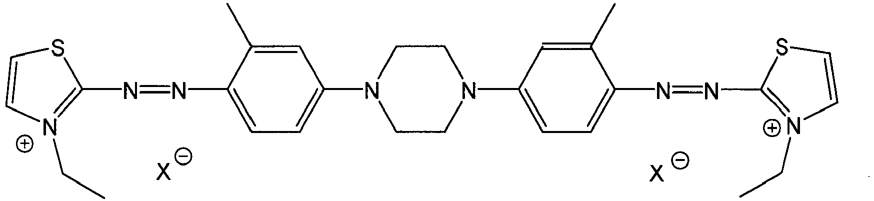
- Salzen des 2-{2-[3-Methoxy-4-(4-{2-methoxy-4-[2-(3-methyl-1,3-thiazol-3-ium-2-yl)diazen-1-yl]phenyl}piperazin-1-yl)phenyl]diazen-1-yl}-3-methyl-1,3-thiazol-3-iums 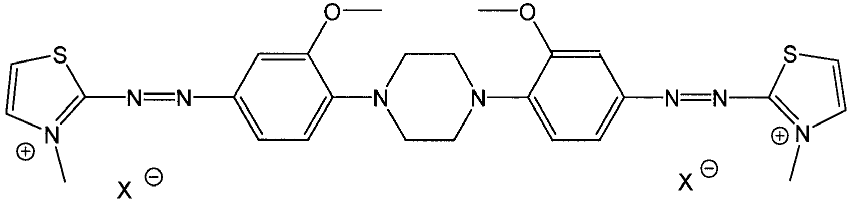
- Salzen des 3-Ethyl-2-{2-[4-(4-{4-[2-(3-ethyl-1,3-thiazol-3-ium-2-yl)diazen-1-yl]-2-methoxyphenyl}piperazin-1-yl)-3-methoxyphenyl]diazen-1-yl}-1,3-thiazol-3-iums 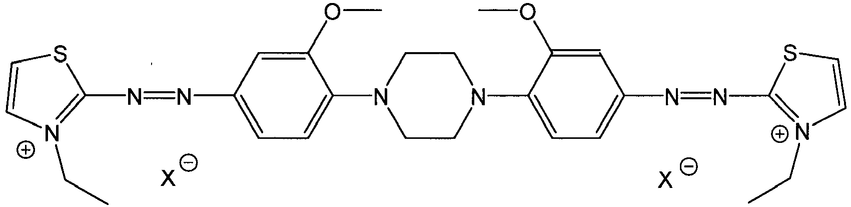
- Salzen des 2-{2-[2-Methoxy-4-(4-{3-methoxy-4-[2-(3-methyl-1,3-thiazol-3-ium-2-yl)diazen-1-yl]phenyl}piperazin-1-yl)phenyl]diazen-1-yl}-3-methyl-1,3-thiazol-3-iums 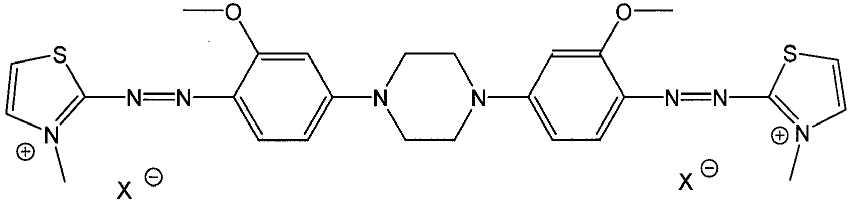
- Salzen des 3-Ethyl-2-{2-[4-(4-{4-[2-(3-ethyl-1,3-thiazol-3-ium-2-yl)diazen-1-yl]-3-methoxyphenyl}piperazin-1-yl)-2-methoxyphenyl]diazen-1-yl}-1,3-thiazol-3-iums 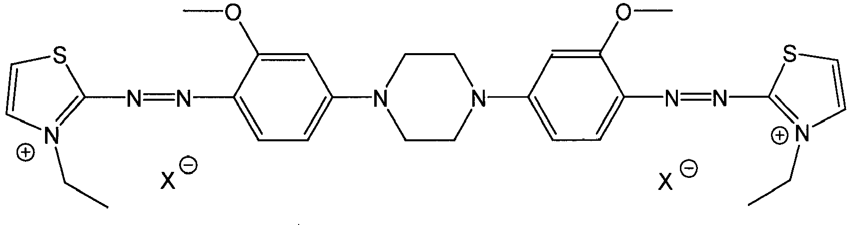
- Salzen des 2-{2-[4-(4-{2,5-Dimethyl-4-[2-(3-methyl-1,3-thiazol-3-ium-2-yl)diazen-1-yl]phenyl}piperazin-1-yl)-2,5-dimethylphenyl]diazen-1-yl}-3-methyl-1,3-thiazol-3-iums 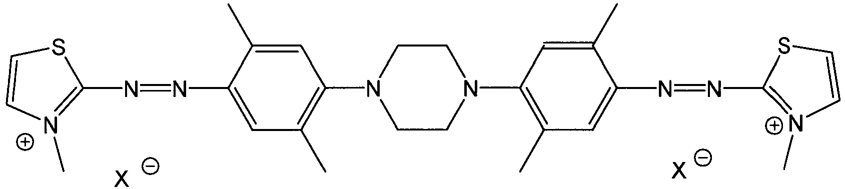
- Salzen des 3-Ethyl-2-{2-[4-(4-{4-[2-(3-ethyl-1,3-thiazol-3-ium-2-yl)diazen-1-yl]-2,5-dimethylphenyl}piperazin-1-yl)-2,5-dimethylphenyl]diazen-1-yl}-1,3-thiazol-3-iums 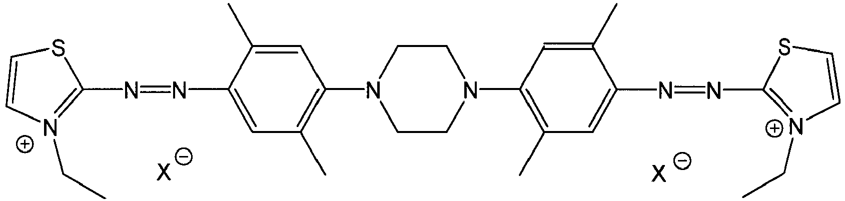
- Salzen des 2-{2-[4-(4-{4-[2-(1,3-Dimethyl-1H-imidazol-3-ium-2-yl)diazen-1-yl]phenyl}piperazin-1-yl)phenyl]diazen-1-yl}-1,3-dimethyl-1H-imidazol-3-iums 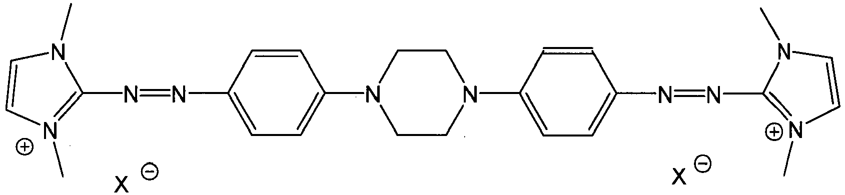
- Salzen des 2-{2-[4-(4-{4-[2-(1,3-Diethyl-1H-imidazol-3-ium-2-yl)diazen-1-yl]phenyl}piperazin-1-yl)phenyl]diazen-1-yl}-1,3-diethyl-1H-imidazol-3-iums 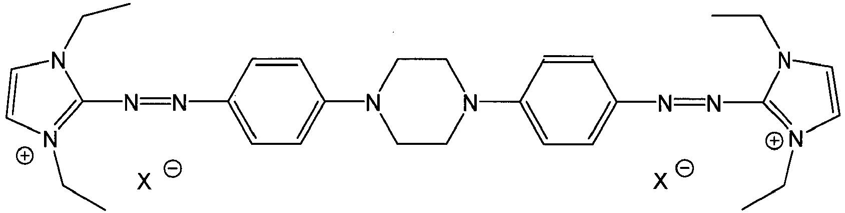
- Salzen des 2-{2-[4-(4-{4-[2-(1,3-Dimethyl-1H-imidazol-3-ium-2-yl)diazen-1-yl]-2-methylphenyl}piperazin-1-yl)-3-methylphenyl]diazen-1-yl}-1,3-dimethyl-1H-imidazol-3-iums 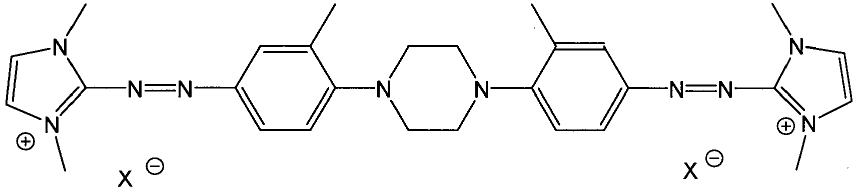
- Salzen des 2-{2-[4-(4-{4-[2-(1,3-Diethyl-1H-imidazol-3-ium-2-yl)diazen-1-yl]-2-methylphenyl}piperazin-1-yl)-3-methylphenyl]diazen-1-yl}-1,3-diethyl-1H-imidazol-3-iums 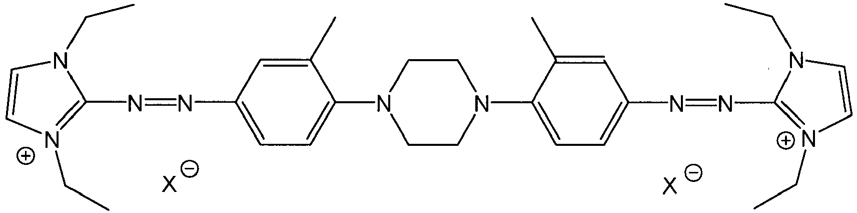
- Salzen des 2-{2-[4-(4-{4-[2-(1,3-Dimethyl-1H-imidazol-3-ium-2-yl)diazen-1-yl]-3-methylphenyl}piperazin-1-yl)-2-methylphenyl]diazen-1-yl}-1,3-dimethyl-1H-imidazol-3-iums 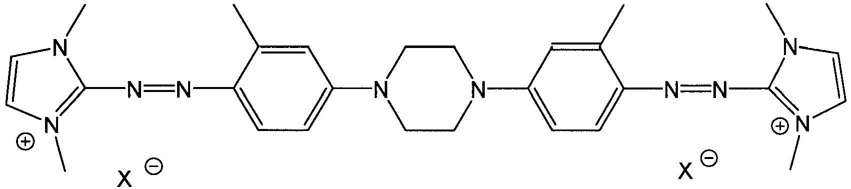
- Salzen des 2-{2-[4-(4-{4-[2-(1,3-Diethyl-1H-imidazol-3-ium-2-yl)diazen-1-yl]-3-methylphenyl}piperazin-1-yl)-2-methylphenyl]diazen-1-yl}-1,3-diethyl-1H-imidazol-3-iums 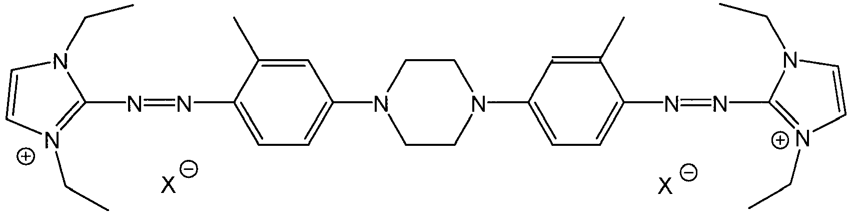
- Salzen des 2-{2-[4-(4-{4-[2-(1,3-Dimethyl-1H-imidazol-3-ium-2-yl)diazen-1-yl]-2-methoxyphenyl}piperazin-1-yl)-3-methoxyphenyl]diazen-1-yl}-1,3-dimethyl-1H-imidazol-3-iums 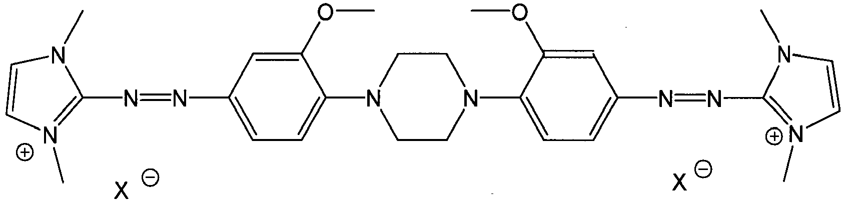
- Salzen des 2-{2-[4-(4-{4-[2-(1,3-Diethyl-1H-imidazol-3-ium-2-yl)diazen-1-yl]-2-methoxyphenyl}piperazin-1-yl)-3-methoxyphenyl]diazen-1-yl}-1,3-diethyl-1H-imidazol-3-iums 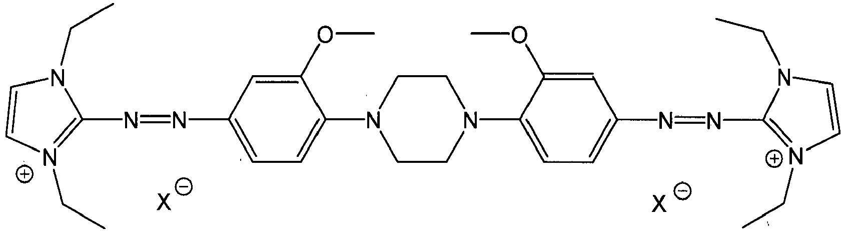
- Salzen des 2-{2-[4-(4-{4-[2-(1,3-Dimethyl-1H-imidazol-3-ium-2-yl)diazen-1-yl]-3-methoxyphenyl}piperazin-1-yl)-2-methoxyphenyl]diazen-1-yl}-1,3-dimethyl-1H-imidazol-3-iums 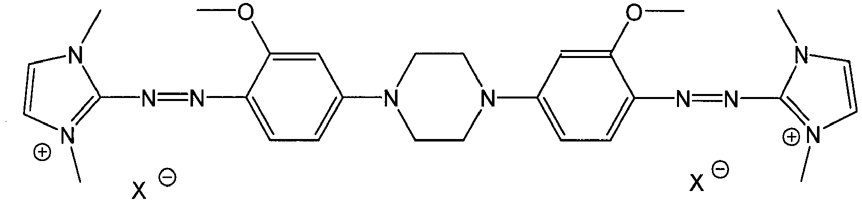
- Salzen des 2-{2-[4-(4-{4-[2-(1,3-Diethyl-1H-imidazol-3-ium-2-yl)diazen-1-yl]-3-methoxyphenyl}piperazin-1-yl)-2-methoxyphenyl]diazen-1-yl}-1,3-diethyl-1H-imidazol-3-iums 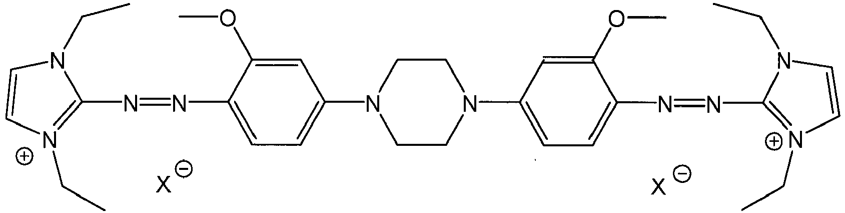
- Salzen des 2-{2-[4-(4-{4-[2-(1,3-Dimethyl-1H-imidazol-3-ium-2-yl)diazen-1-yl]-2,5-dimethylphenyl}piperazin-1-yl)-2,5-dimethylphenyl]diazen-1-yl}-1,3-dimethyl-1H-imidazol-3-iums 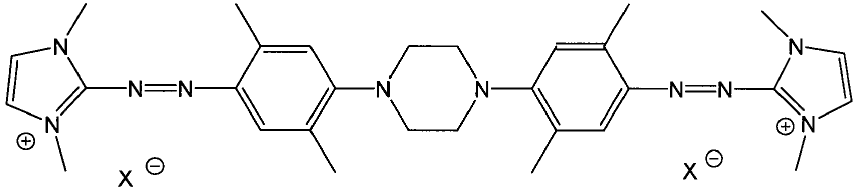
- Salzen des 2-{2-[4-(4-{4-[2-(1,3-Diethyl-1H-imidazol-3-ium-2-yl)diazen-1-yl]-2,5-dimethylphenyl}piperazin-1-yl)-2,5-dimethylphenyl]diazen-1-yl}-1,3-diethyl-1H-imidazol-3-iums 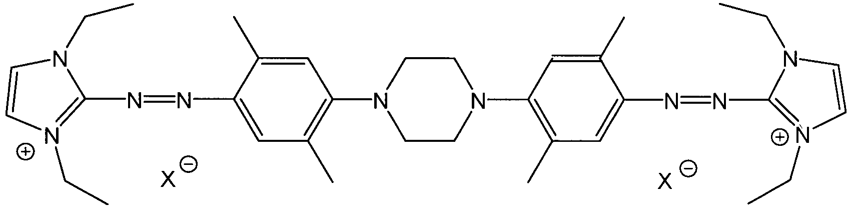
- Salzen des 5-{2-[4-(4-{4-[2-(1,4-Dimethyl-1H-1,2,4-triazol-4-ium-5-yl)diazen-1-yl]phenyl}piperazin-1-yl)phenyl]diazen-1-yl}-1,4-dimethyl-1H-1,2,4-triazol-4-iums 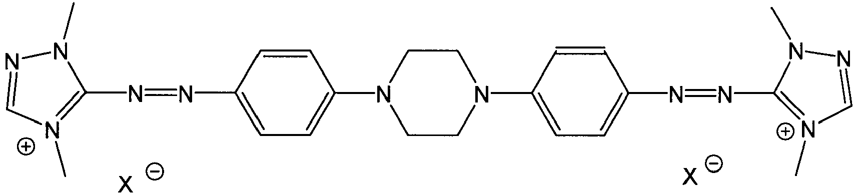
- Salzen des 5-{2-[4-(4-{4-[2-(1,4-Diethyl-1H-1,2,4-triazol-4-ium-5-yl)diazen-1-yl]phenyl}piperazin-1-yl)phenyl]diazen-1-yl}-1,4-diethyl-1H-1,2,4-triazol-4-iums 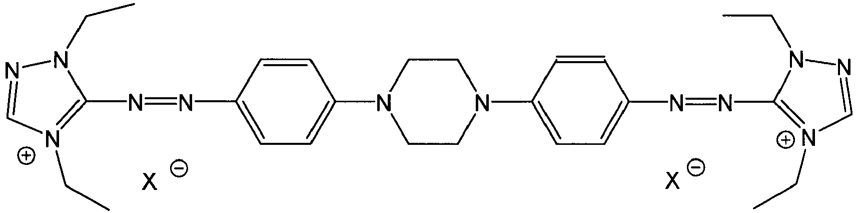
- Salzen des 5-{2-[4-(4-{4-[2-(1,4-Dimethyl-1H-1,2,4-triazol-4-ium-5-yl)diazen-1-yl]-2-methylphenyl}piperazin-1-yl)-3-methylphenyl]diazen-1-yl}-1,4-dimethyl-1H-1,2,4-triazol-4-iums 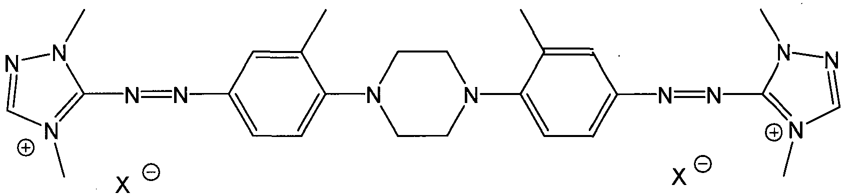
- Salzen des 5-{2-[4-(4-{4-[2-(1,4-Diethyl-1H-1,2,4-triazol-4-ium-5-yl)diazen-1-yl]-2-methylphenyl}piperazin-1-yl)-3-methylphenyl]diazen-1-yl}-1,4-diethyl-1H-1,2,4-triazol-4-iums 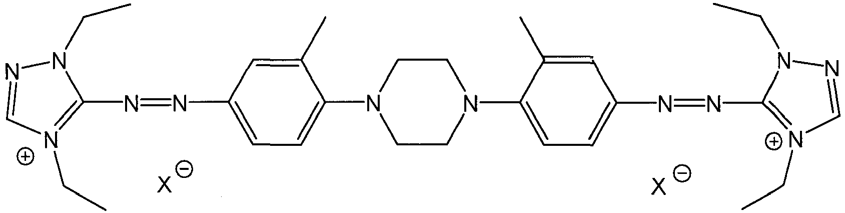
- Salzen des 5-{2-[4-(4-{4-[2-(1,4-Dimethyl-1H-1,2,4-triazol-4-ium-5-yl)diazen-1-yl]-3-methylphenyl}piperazin-1-yl)-2-methylphenyl]diazen-1-yl}-1,4-dimethyl-1H-1,2,4-triazol-4-iums 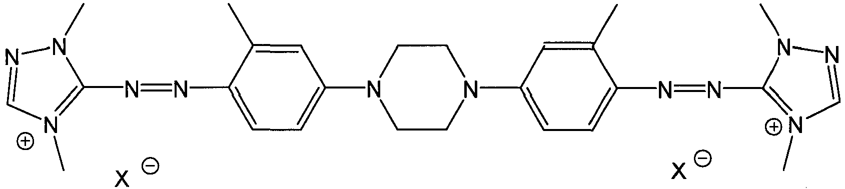
- Salzen des 5-{2-[4-(4-{4-[2-(1,4-Diethyl-1H-1,2,4-triazol-4-ium-5-yl)diazen-1-yl]-3-methylphenyl}piperazin-1-yl)-2-methylphenyl]diazen-1-yl}-1,4-diethyl-1H-1,2,4-triazol-4-iums 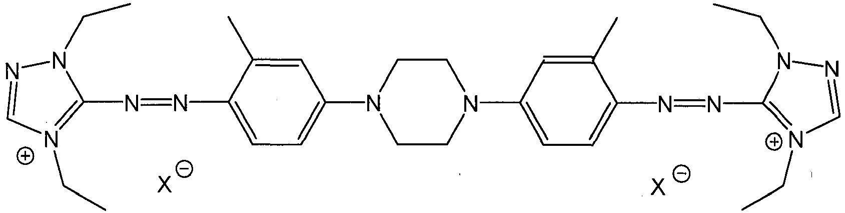
- Salzen des 5-{2-[4-(4-{4-[2-(1,4-Dimethyl-1H-1,2,4-triazol-4-ium-5-yl)diazen-1-yl]-2-methoxyphenyl}piperazin-1-yl)-3-methoxyphenyl]diazen-1-yl}-1,4-dimethyl-1H-1,2,4-triazol-4-iums 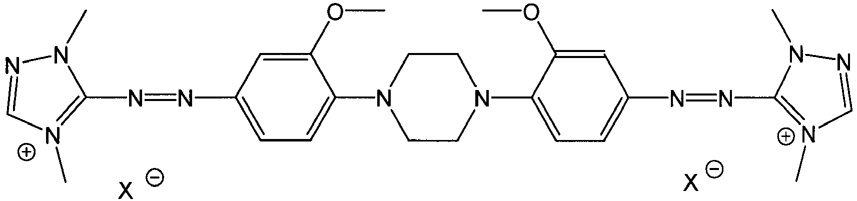
- Salzen des 5-{2-[4-(4-{4-[2-(1,4-diethyl-1H-1,2,4-triazol-4-ium-5-yl)diazen-1-yl]-2-methoxyphenyl}piperazin-1-yl)-3-methoxyphenyl]diazen-1-yl}-1,4-diethyl-1H-1,2,4-triazol-4-iums 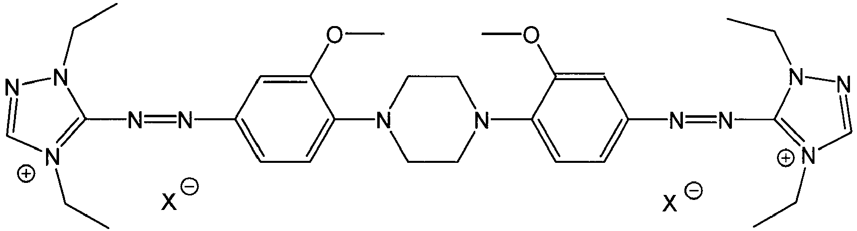
- Salzen des 5-{2-[4-(4-{4-[2-(1,4-Dimethyl-1H-1,2,4-triazol-4-ium-5-yl)diazen-1-yl]-3-methoxyphenyl}piperazin-1-yl)-2-methoxyphenyl]diazen-1-yl}-1,4-dimethyl-1H-1,2,4-triazol-4-iums 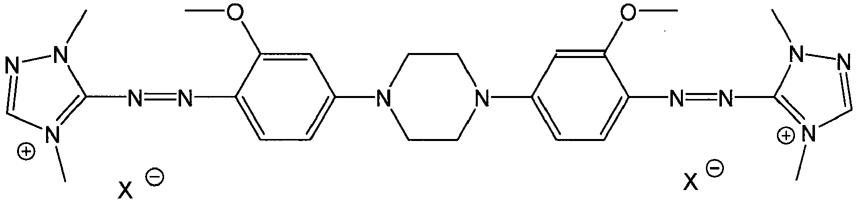
- Salzen des 5-{2-[4-(4-{4-[2-(1,4-Diethyl-1H-1,2,4-triazol-4-ium-5-yl)diazen-1-yl]-3-methoxyphenyl}piperazin-1-yl)-2-methoxyphenyl]diazen-1-yl}-1,4-diethyl-1H-1,2,4-triazol-4-iums 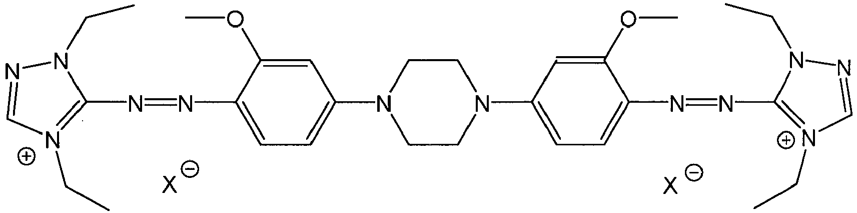
- Salzen des 5-{2-[4-(4-{4-[2-(1,4-Dimethyl-1H-1,2,4-triazol-4-ium-5-yl)diazen-1-yl]-2,5-dimethylphenyl}piperazin-1-yl)-2,5-dimethylphenyl]diazen-1-yl}-1,4-dimethyl-1H-1,2,4-triazol-4-iums 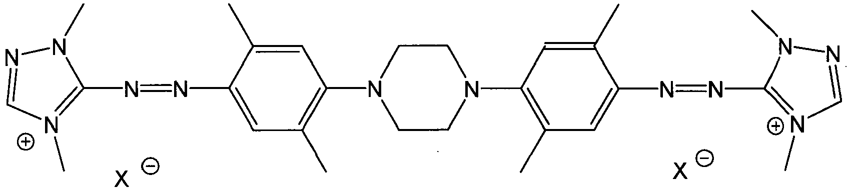
- Salzen des 5-{2-[4-(4-{4-[2-(1,4-Diethyl-1H-1,2,4-triazol-4-ium-5-yl)diazen-1-yl]-2,5-dimethylphenyl}piperazin-1-yl)-2,5-dimethylphenyl]diazen-1-yl}-1,4-diethyl-1H-1,2,4-triazol-4-iums 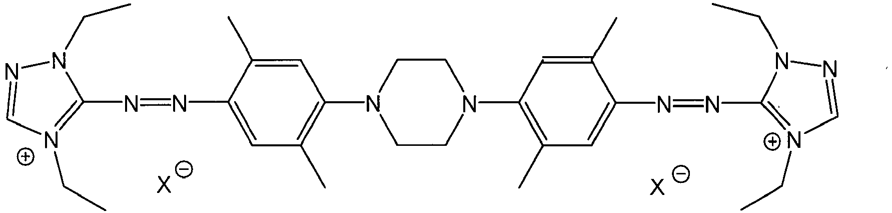
- Salzen des 3-Methyl-2-{2-[4-(5-{4-[2-(3-methyl-1,3-thiazol-3-ium-2-yl)diazen-1-yl]phenyl}-1,5-diazocan-1-yl)phenyl]diazen-1-yl}-1,3-thiazol-3-iums 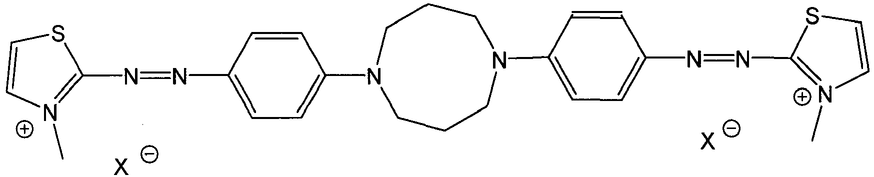
- Salzen des 3-Ethyl-2-{2-[4-(5-{4-[2-(3-ethyl-1,3-thiazol-3-ium-2-yl)diazen-1-yl]phenyl}-1,5-diazocan-1-yl)phenyl]diazen-1-yl}-1,3-thiazol-3-iums 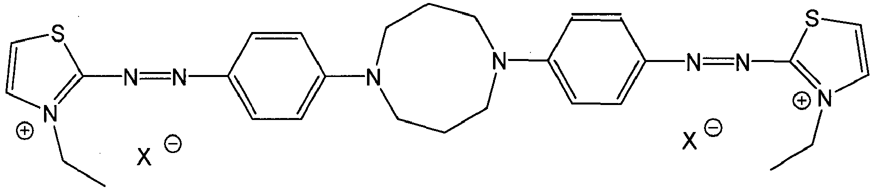
- Salzen des 3-Methyl-2-{2-[3-methyl-4-(5-{2-methyl-4-[2-(3-methyl-1,3-thiazol-3-ium-2-yl)diazen-1-yl]phenyl}-1,5-diazocan-1-yl)phenyl]diazen-1-yl}-1,3-thiazol-3-iums 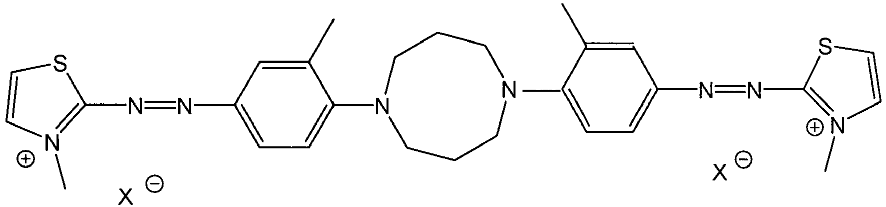
- Salzen des 3-Ethyl-2-{2-[4-(5-{4-[2-(3-ethyl-1,3-thiazol-3-ium-2-yl)diazen-1-yl]-2-methylphenyl}-1,5-diazocan-1-yl)-3-methylphenyl]diazen-1-yl}-1,3-thiazol-3-iums 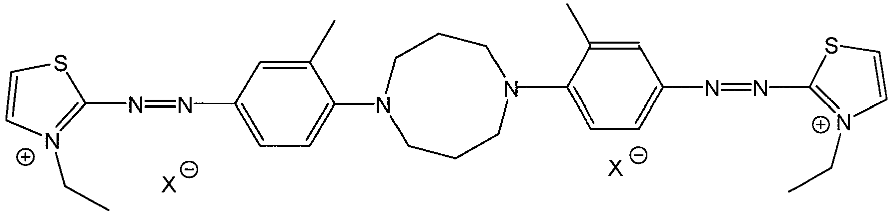
- Salzen des 3-Methyl-2-{2-[2-methyl-4-(5-{3-methyl-4-[2-(3-methyl-1,3-thiazol-3-ium-2-yl)diazen-1-yl]phenyl}-1,5-diazocan-1-yl)phenyl]diazen-1-yl}-1,3-thiazol-3-iums 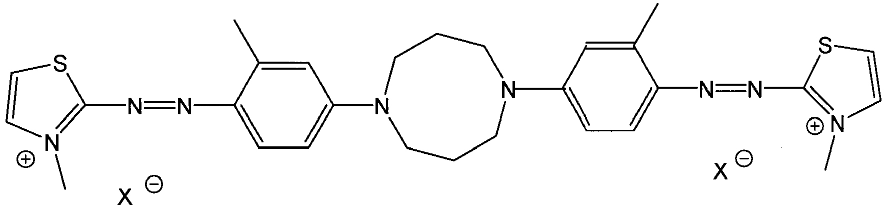
- Salzen des 3-Ethyl-2-{2-[4-(5-{4-[2-(3-ethyl-1,3-thiazol-3-ium-2-yl)diazen-1-yl]-3-methylphenyl}-1,5-diazocan-1-yl)-2-methylphenyl]diazen-1-yl}-1,3-thiazol-3-iums 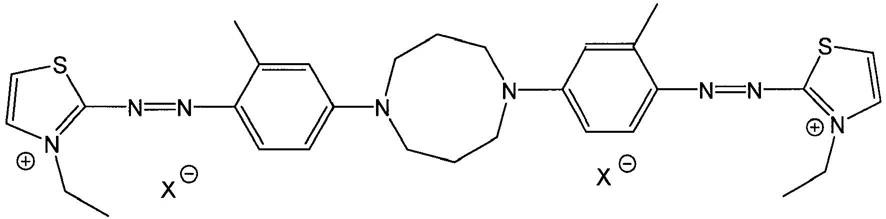
- Salzen des 2-{2-[3-Methoxy-4-(5-{2-methoxy-4-[2-(3-methyl-1,3-thiazol-3-ium-2-yl)diazen-1-yl]phenyl}-1,5-diazocan-1-yl)phenyl]diazen-1-yl}-3-methyl-1,3-thiazol-3-iums 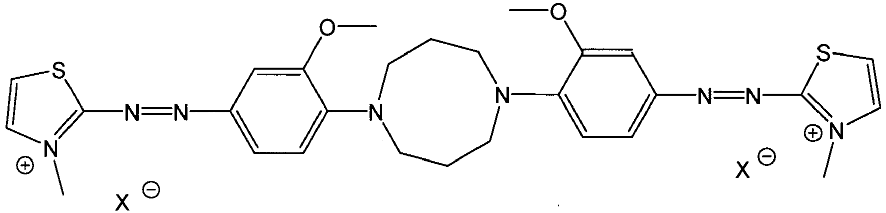
- Salzen des 3-Ethyl-2-{2-[4-(5-{4-[2-(3-ethyl-1,3-thiazol-3-ium-2-yl)diazen-1-yl]-2-methoxyphenyl}-1,5-diazocan-1-yl)-3-methoxyphenyl]diazen-1-yl}-1,3-thiazol-3-iums 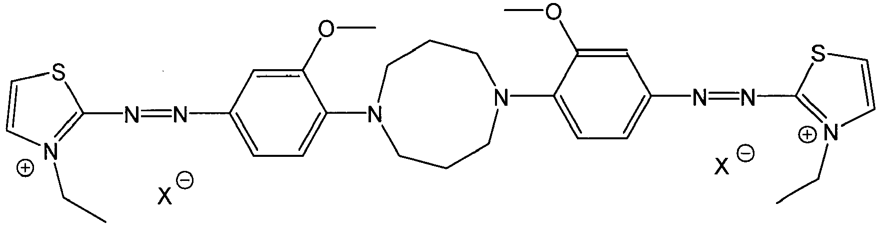
- Salzen des 2-{2-[2-Methoxy-4-(5-{3-methoxy-4-[2-(3-methyl-1,3-thiazol-3-ium-2-yl)diazen-1-yl]phenyl}-1,5-diazocan-1-yl)phenyl]diazen-1-yl}-3-methyl-1,3-thiazol-3-iums 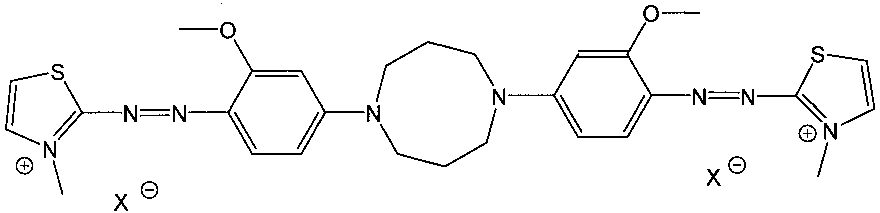
- Salzen des 3-Ethyl-2-{2-[4-(5-{4-[2-(3-ethyl-1,3-thiazol-3-ium-2-yl)diazen-1-yl]-3-methoxyphenyl}-1,5-diazocan-1-yl)-2-methoxyphenyl]diazen-1-yl}-1,3-thiazol-3-iums 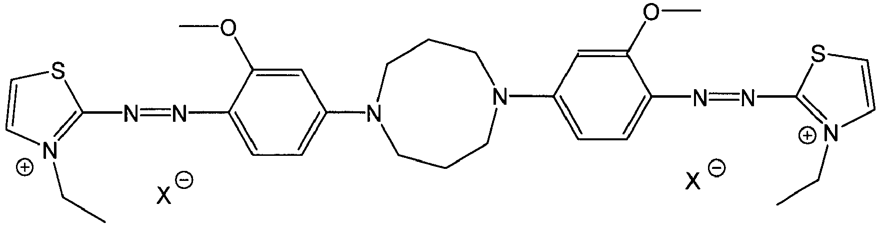
- Salzen des 2-{2-[4-(5-{2,5-Dimethyl-4-[2-(3-methyl-1,3-thiazol-3-ium-2-yl)diazen-1-yl]phenyl}-1,5-diazocan-1-yl)-2,5-dimethylphenyl]diazen-1-yl}-3-methyl-1,3-thiazol-3-iums 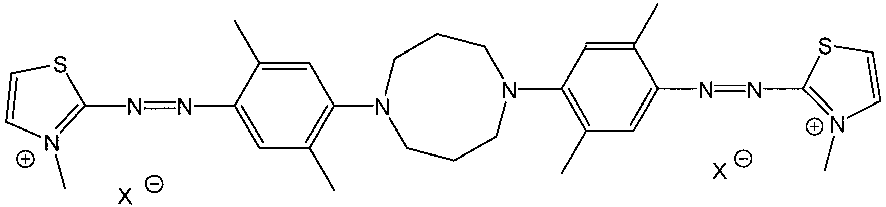
- Salzen des 3-Ethyl-2-{2-[4-(5-{4-[2-(3-ethyl-1,3-thiazol-3-ium-2-yl)diazen-1-yl]-2,5-dimethylphenyl}-1,5-diazocan-1-yl)-2,5-dimethylphenyl]diazen-1-yl}-1,3-thiazol-3-iums 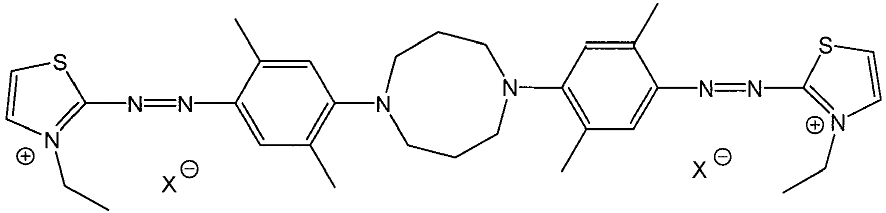
- Salzen des 2-{2-[4-(5-{4-[2-(1,3-Dimethyl-1H-imidazol-3-ium-2-yl)diazen-1-yl]phenyl}-1,5-diazocan-1-yl)phenyl]diazen-1-yl}-1,3-dimethyl-1H-imidazol-3-iums 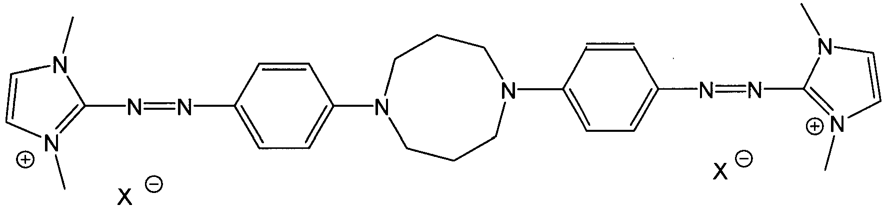
- Salzen des 2-{2-[4-(5-{4-[2-(1,3-Diethyl-1H-imidazol-3-ium-2-yl)diazen-1-yl]phenyl}-1,5-diazocan-1-yl)phenyl]diazen-1-yl}-1,3-diethyl-1H-imidazol-3-iums 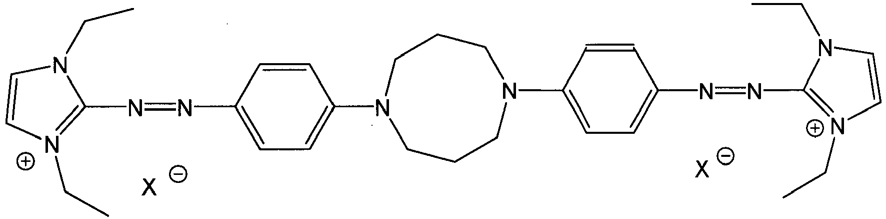
- Salzen des 2-{2-[4-(5-{4-[2-(1,3-Dimethyl-1H-imidazol-3-ium-2-yl)diazen-1-yl]-2-methylphenyl}-1,5-diazocan-1-yl)-3-methylphenyl]diazen-1-yl}-1,3-dimethyl-1H-imidazol-3-iums 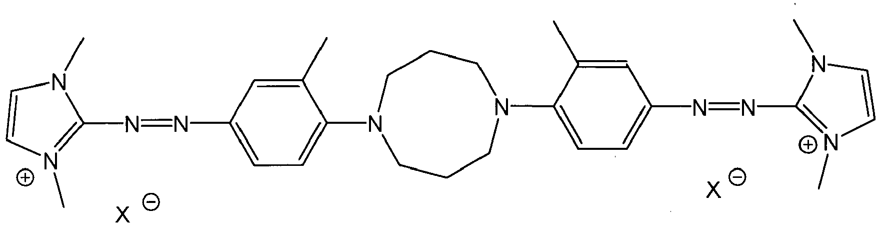
- Salzen des 2-{2-[4-(5-{4-[2-(1,3-Diethyl-1H-imidazol-3-ium-2-yl)diazen-1-yl]-2-methylphenyl}-1,5-diazocan-1-yl)-3-methylphenyl]diazen-1-yl}-1,3-diethyl-1H-imidazol-3-iums 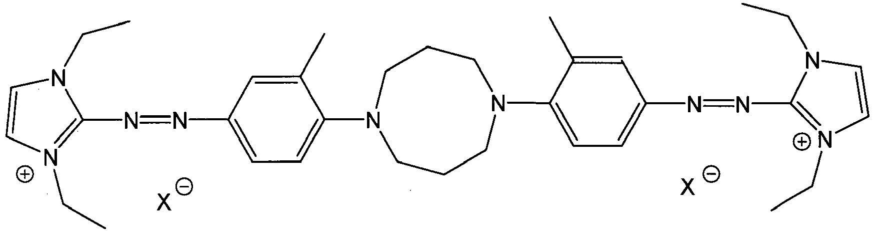
- Salzen des 2-{2-[4-(5-{4-[2-(1,3-Dimethyl-1H-imidazol-3-ium-2-yl)diazen-1-yl]-3-methylphenyl}-1,5-diazocan-1-yl)-2-methylphenyl]diazen-1-yl}-1,3-dimethyl-1H-imidazol-3-iums 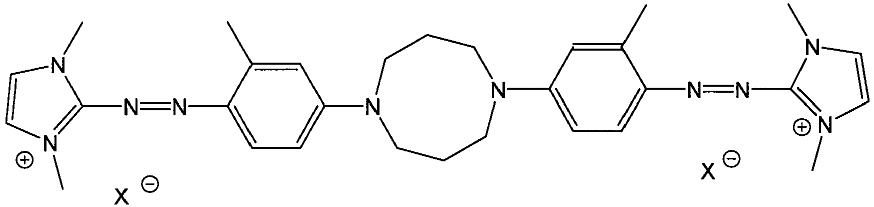
- Salzen des 2-{2-[4-(5-{4-[2-(1,3-Diethyl-1H-imidazol-3-ium-2-yl)diazen-1-yl]-3-methylphenyl}-1,5-diazocan-1-yl)-2-methylphenyl]diazen-1-yl}-1,3-diethyl-1H-imidazol-3-iums 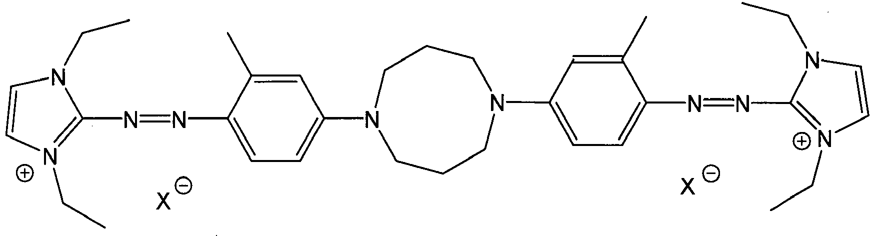
- Salzen des 2-{2-[4-(5-{4-[2-(1,3-Dimethyl-1H-imidazol-3-ium-2-yl)diazen-1-yl]-2-methoxyphenyl}-1,5-diazocan-1-yl)-3-methoxyphenyl]diazen-1-yl}-1,3-dimethyl-1H-imidazol-3-iums 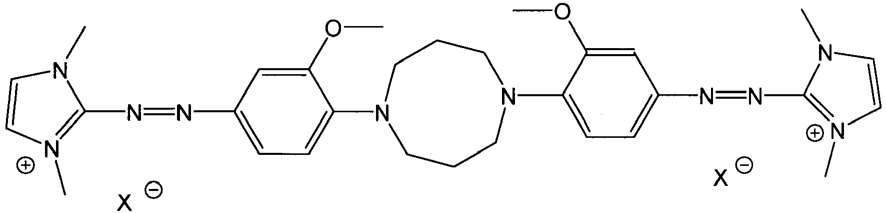
- Salzen des 2-{2-[4-(5-{4-[2-(1,3-Diethyl-1H-imidazol-3-ium-2-yl)diazen-1-yl]-2-methoxyphenyl}-1,5-diazocan-1-yl)-3-methoxyphenyl]diazen-1-yl}-1,3-diethyl-1H-imidazol-3-iums 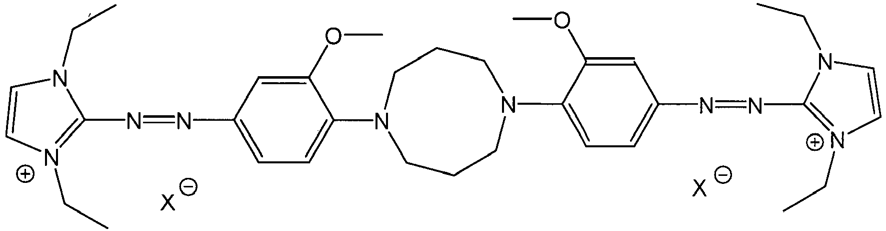
- Salzen des 2-{2-[4-(5-{4-[2-(1,3-Dimethyl-1H-imidazol-3-ium-2-yl)diazen-1-yl]-3-methoxyphenyl}-1,5-diazocan-1-yl)-2-methoxyphenyl]diazen-1-yl}-1,3-dimethyl-1H-imidazol-3-iums 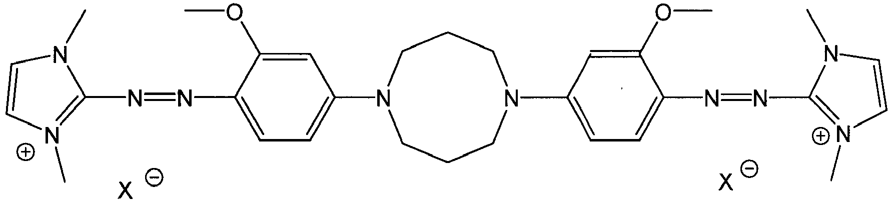
- Salzen des 2-{2-[4-(5-{4-[2-(1,3-Diethyl-1H-imidazol-3-ium-2-yl)diazen-1-yl]-3-methoxyphenyl}-1,5-diazocan-1-yl)-2-methoxyphenyl]diazen-1-yl}-1,3-diethyl-1H-imidazol-3-iums 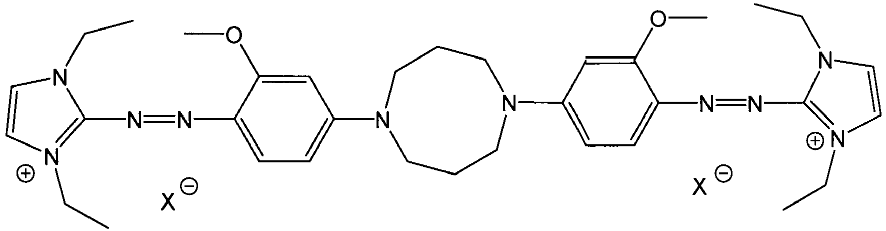
- Salzen des 2-{2-[4-(5-{4-[2-(1,3-Dimethyl-1H-imidazol-3-ium-2-yl)diazen-1-yl]-2,5-dimethylphenyl}-1,5-diazocan-1-yl)-2,5-dimethylphenyl]diazen-1-yl}-1,3-dimethyl-1H-imidazol-3-iums 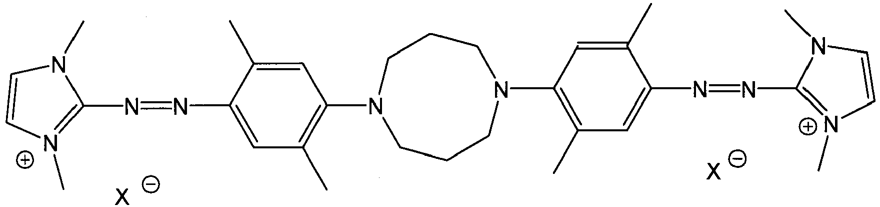
- Salzen des 2-{2-[4-(5-{4-[2-(1,3-Diethyl-1H-imidazol-3-ium-2-yl)diazen-1-yl]-2,5-dimethylphenyl}-1,5-diazocan-1-yl)-2,5-dimethylphenyl]diazen-1-yl}-1,3-diethyl-1H-imidazol-3-iums 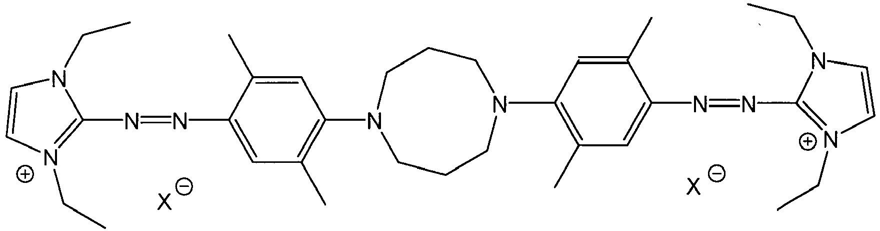
- Salzen des 5-{2-[4-(5-{4-[2-(1,4-Dimethyl-1H-1,2,4-triazol-4-ium-5-yl)diazen-1-yl]phenyl}-1,5-diazocan-1-yl)phenyl]diazen-1-yl}-1,4-dimethyl-1H-1,2,4-triazol-4-iums 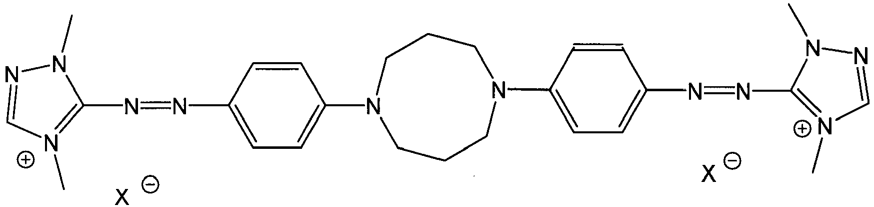
- Salzen des 5-{2-[4-(5-{4-[2-(1,4-Diethyl-1H-1,2,4-triazol-4-ium-5-yl)diazen-1-yl]phenyl}-1,5-diazocan-1-yl)phenyl]diazen-1-yl}-1,4-diethyl-1H-1,2,4-triazol-4-iums 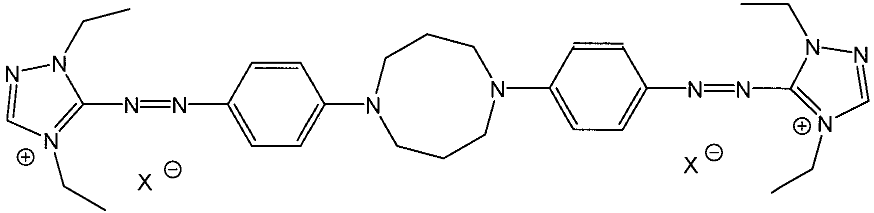
- Salzen des 5-{2-[4-(5-{4-[2-(1,4-Dimethyl-1H-1,2,4-triazol-4-ium-5-yl)diazen-1-yl]-2-methylphenyl}-1,5-diazocan-1-yl)-3-methylphenyl]diazen-1-yl}-1,4-dimethyl-1H-1,2,4-triazol-4-iums 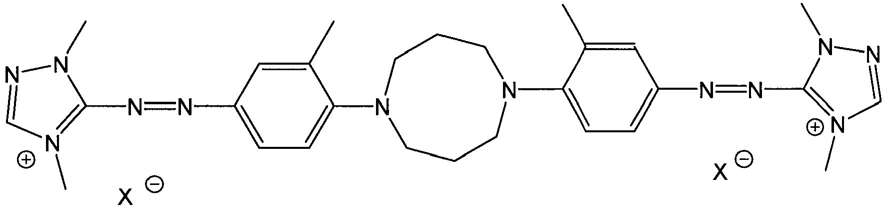
- Salzen des 5-{2-[4-(5-{4-[2-(1,4-diethyl-1H-1,2,4-triazol-4-ium-5-yl)diazen-1-yl]-2-methylphenyl}-1,5-diazocan-1-yl)-3-methylphenyl]diazen-1-yl}-1,4-diethyl-1H-1,2,4-triazol-4-iums 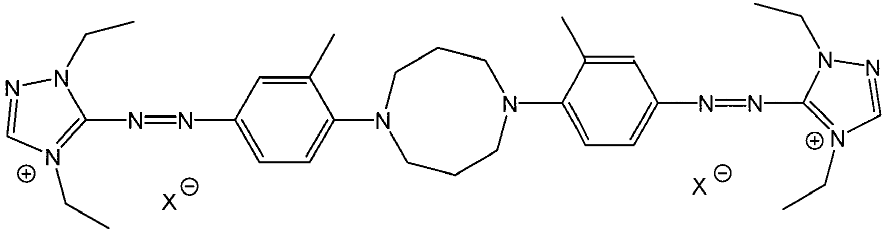
- Salzen des 5-{2-[4-(5-{4-[2-(1,4-dimethyl-1H-1,2,4-triazol-4-ium-5-yl)diazen-1-yl]-3-methylphenyl}-1,5-diazocan-1-yl)-2-methylphenyl]diazen-1-yl}-1,4-dimethyl-1H-1,2,4-triazol-4-iums 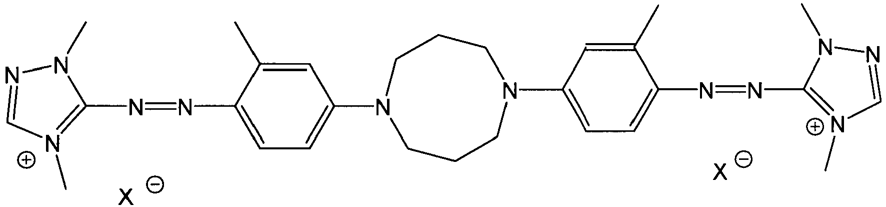
- Salzen des 5-{2-[4-(5-{4-[2-(1,4-diethyl-1H-1,2,4-triazol-4-ium-5-yl)diazen-1-yl]-3-methylphenyl}-1,5-diazocan-1-yl)-2-methylphenyl]diazen-1-yl}-1,4-diethyl-1H-1,2,4-triazol-4-iums 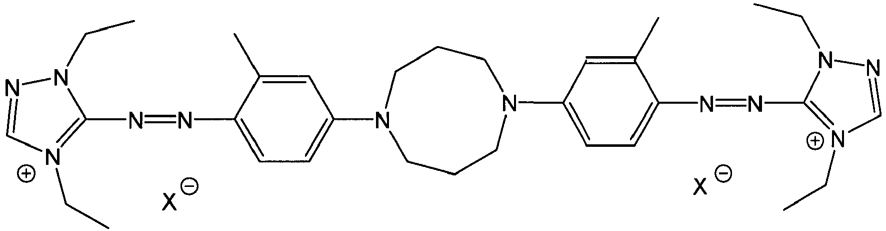
- Salzen des 5-{2-[4-(5-{4-[2-(1,4-Dimethyl-1H-1,2,4-triazol-4-ium-5-yl)diazen-1-yl]-2-methoxyphenyl}-1,5-diazocan-1-yl)-3-methoxyphenyl]diazen-1-yl}-1,4-dimethyl-1H-1,2,4-triazol-4-iums 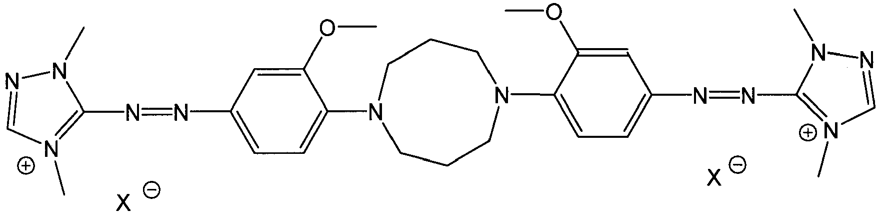
- Salzen des 5-{2-[4-(5-{4-[2-(1,4-Diethyl-1H-1,2,4-triazol-4-ium-5-yl)diazen-1-yl]-2-methoxyphenyl}-1,5-diazocan-1-yl)-3-methoxyphenyl]diazen-1-yl}-1,4-diethyl-1H-1,2,4-triazol-4-iums 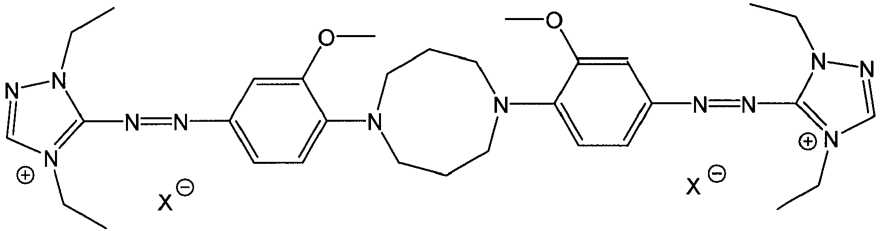
- Salzen des 5-{2-[4-(5-{4-[2-(1,4-dimethyl-1H-1,2,4-thazol-4-ium-5-yl)diazen-1-yl]-3-methoxyphenyl}-1,5-diazocan-1-yl)-2-methoxyphenyl]diazen-1-yl}-1,4-dimethyl-1H-1,2,4-triazol-4-iums 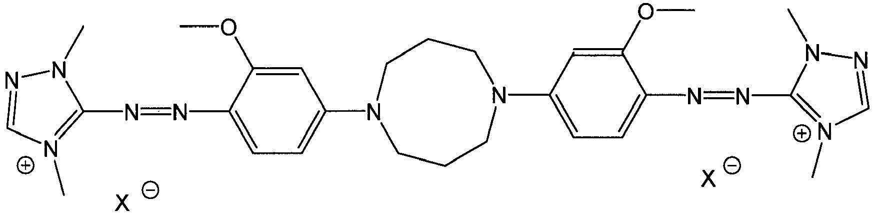
- Salzen des 5-{2-[4-(5-{4-[2-(1,4-diethyl-1H-1,2,4-triazol-4-ium-5-yl)diazen-1-yl]-3-methoxyphenyl}-1,5-diazocan-1-yl)-2-methoxyphenyl]diazen-1-yl}-1,4-diethyl-1H-1,2,4-triazol-4-iums 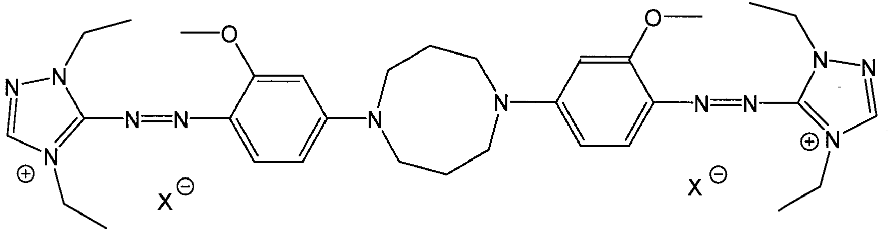
- Salzen des 5-{2-[4-(5-{4-[2-(1,4-Dimethyl-1H-1,2,4-triazol-4-ium-5-yl)diazen-1-yl]-2,5-dimethylphenyl}-1,5-diazocan-1-yl)-2,5-dimethylphenyl]diazen-1-yl}-1,4-dimethyl-1H-1,2,4-triazol-4-iums 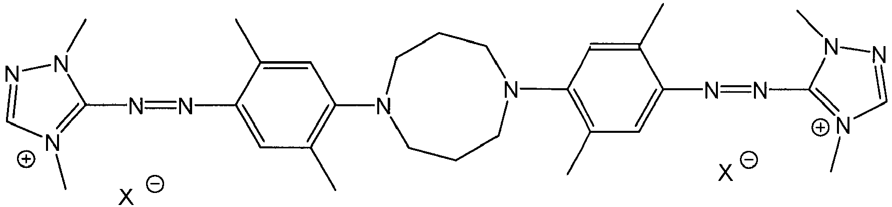 und/oder
- Salzen des 5-{2-[4-(5-{4-[2-(1,4-diethyl-1H-1,2,4-triazol-4-ium-5-yl)diazen-1-yl]-2,5-dimethylphenyl}-1,5-diazocan-1-yl)-2,5-dimethylphenyl]diazen-1-yl}-1,4-diethyl-1H-1,2,4-triazol-4-iums

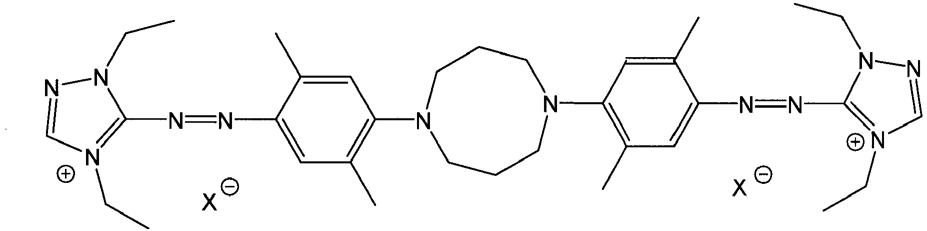

Bei den vorgenannten Verbindungen handelt es sich um dikationische dimere Farbstoffe, wobei das organische Dikation durch die beiden Anionen X- neutralisiert wird. Bei den Anionen X- kann es sich jeweils um ein Anion aus der Gruppe aus Chlorid, Bromid, lodid, Methylsulfat, Methylsulfonat, p-Toluolsulfonat, Acetat, Hydrogensulfat, ½ Sulfat oder ½ Tetrachlorozinkat handeln. Im Rahmen der zu dieser Erfindung führenden Arbeiten hat sich herausgestellt, besonders dann ganz besonders intensive und oxidationsstabile Färbungen erzielt werden konnten, wenn direktziehende Farbstoffe der Formel (la) eingesetzt werden, 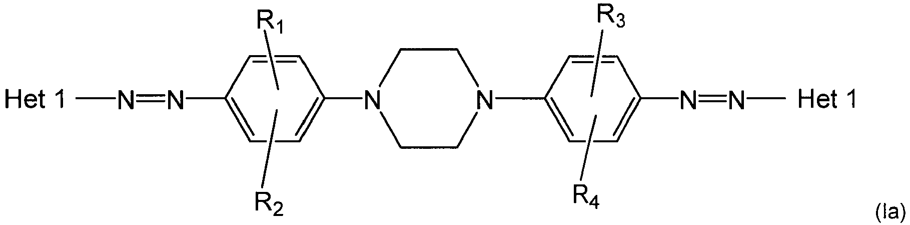 wobei
Het 1 für eine der Strukturen (II), (III) oder (IV) steht, 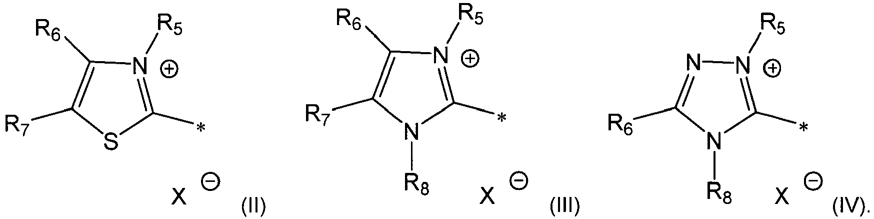
- R1: für ein Wasserstoffatom oder für eine Methylgruppe steht,
- R2: für ein Wasserstoffatom steht,
- R3: für ein Wasserstoffatom oder für eine Methylgruppe steht,
- R4: für ein Wasserstoffatom steht,
- R5, R8: unabhängig voneinander für eine C₁-C₆-Alkylgruppe stehen,
- R6, R7: unabhängig voneinander für ein Wasserstoffatom oder für eine C₁-C₆-Alkylgruppe stehen,
- X-: jeweils für ein physiologisch verträgliches Anion, bevorzugt aus der Gruppe Chlorid, Bromid, Iodid, Methylsulfat, Methylsulfonat, p-Toluolsulfonat, Acetat, Hydrogensulfat, ½ Sulfat oder ½ Tetrachlorozinkat, steht.

Explizit ganz besonders bevorzugt sind daher erfindungsgemäße Mittel zur Farbveränderung von keratinischen Fasern, welche mindestens einen Farbstoff (a) der Formel (I) enthalten, der ausgewählt ist aus der Gruppe aus 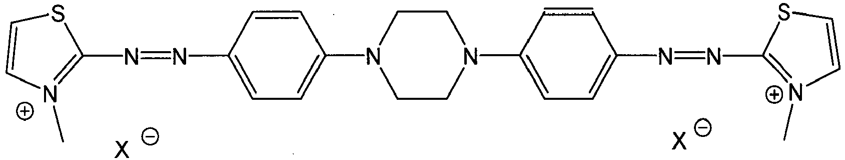
- 3-Methyl-2-{2-[4-(4-{4-[2-(3-methyl-1,3-thiazol-3-ium-2-yl)diazen-1-yl]phenyl}piperazin-1-yl)phenyl]diazen-1-yl}-1,3-thiazol-3-ium Dichlorid
- 3-Methyl-2-{2-[4-(4-{4-[2-(3-methyl-1,3-thiazol-3-ium-2-yl)diazen-1-yl]phenyl}piperazin-1-yl)phenyl]diazen-1-yl}-1,3-thiazol-3-ium Dibromid
- 3-Methyl-2-{2-[4-(4-{4-[2-(3-methyl-1,3-thiazol-3-ium-2-yl)diazen-1-yl]phenyl}piperazin-1-yl)phenyl]diazen-1-yl}-1,3-thiazol-3-ium Sulfat
- 3-Methyl-2-{2-[4-(4-{4-[2-(3-methyl-1,3-thiazol-3-ium-2-yl)diazen-1-yl]phenyl}piperazin-1-yl)phenyl]diazen-1-yl}-1,3-thiazol-3-ium Di(p-toluolsulfonat)
- 3-Methyl-2-{2-[4-(4-{4-[2-(3-methyl-1,3-thiazol-3-ium-2-yl)diazen-1-yl]phenyl}piperazin-1-yl)phenyl]diazen-1-yl}-1,3-thiazol-3-ium Di(methylsulfat)
- 3-Methyl-2-{2-[4-(4-{4-[2-(3-methyl-1,3-thiazol-3-ium-2-yl)diazen-1-yl]phenyl}piperazin-1-yl)phenyl]diazen-1-yl}-1,3-thiazol-3-ium Tetrachlorozinkat 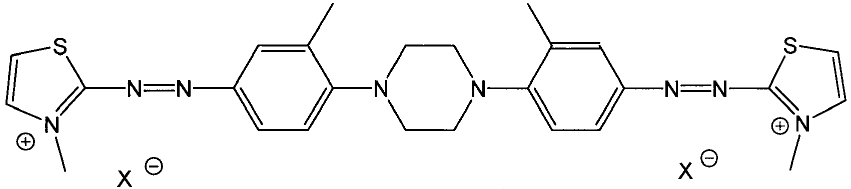
- 3-Methyl-2-{2-[3-methyl-4-(4-{2-methyl-4-[2-(3-methyl-1,3-thiazol-3-ium-2-yl)diazen-1-yl]phenyl}piperazin-1-yl)phenyl]diazen-1-yl}-1,3-thiazol-3-ium Dichlorid
- 3-Methyl-2-{2-[3-methyl-4-(4-{2-methyl-4-[2-(3-methyl-1,3-thiazol-3-ium-2-yl)diazen-1-yl]phenyl}piperazin-1-yl)phenyl]diazen-1-yl}-1,3-thiazol-3-ium Dibromid
- 3-Methyl-2-{2-[3-methyl-4-(4-{2-methyl-4-[2-(3-methyl-1,3-thiazol-3-ium-2-yl)diazen-1-yl]phenyl}piperazin-1-yl)phenyl]diazen-1-yl}-1,3-thiazol-3-ium Sulfat
- 3-Methyl-2-{2-[3-methyl-4-(4-{2-methyl-4-[2-(3-methyl-1,3-thiazol-3-ium-2-yl)diazen-1-yl]phenyl}piperazin-1-yl)phenyl]diazen-1-yl}-1,3-thiazol-3-ium Di(p-toluolsulfonat)
- 3-Methyl-2-{2-[3-methyl-4-(4-{2-methyl-4-[2-(3-methyl-1,3-thiazol-3-ium-2-yl)diazen-1-yl]phenyl}piperazin-1-yl)phenyl]diazen-1-yl}-1,3-thiazol-3-ium Di(methylsulfat)
- 3-Methyl-2-{2-[3-methyl-4-(4-{2-methyl-4-[2-(3-methyl-1,3-thiazol-3-ium-2-yl)diazen-1-yl]phenyl}piperazin-1-yl)phenyl]diazen-1-yl}-1,3-thiazol-3-ium Tetrachlorozinkat 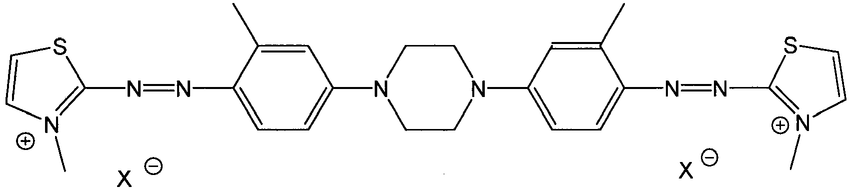
- 3-Methyl-2-{2-[2-methyl-4-(4-{3-methyl-4-[2-(3-methyl-1,3-thiazol-3-ium-2-yl)diazen-1-yl]phenyl}piperazin-1-yl)phenyl]diazen-1-yl}-1,3-thiazol-3-ium Dichlorid
- 3-Methyl-2-{2-[2-methyl-4-(4-{3-methyl-4-[2-(3-methyl-1,3-thiazol-3-ium-2-yl)diazen-1-yl]phenyl}piperazin-1-yl)phenyl]diazen-1-yl}-1,3-thiazol-3-ium Dibromid
- 3-Methyl-2-{2-[2-methyl-4-(4-{3-methyl-4-[2-(3-methyl-1,3-thiazol-3-ium-2-yl)diazen-1-yl]phenyl}piperazin-1-yl)phenyl]diazen-1-yl}-1,3-thiazol-3-ium Sulfat
- 3-Methyl-2-{2-[2-methyl-4-(4-{3-methyl-4-[2-(3-methyl-1,3-thiazol-3-ium-2-yl)diazen-1-yl]phenyl}piperazin-1-yl)phenyl]diazen-1-yl}-1,3-thiazol-3-ium Di(p-toluolsulfonat)
- 3-Methyl-2-{2-[2-methyl-4-(4-{3-methyl-4-[2-(3-methyl-1,3-thiazol-3-ium-2-yl)diazen-1-yl]phenyl}piperazin-1-yl)phenyl]diazen-1-yl}-1,3-thiazol-3-ium Di(methylsulfat)
- 3-Methyl-2-{2-[2-methyl-4-(4-{3-methyl-4-[2-(3-methyl-1,3-thiazol-3-ium-2-yl)diazen-1-yl]phenyl}piperazin-1-yl)phenyl]diazen-1-yl}-1,3-thiazol-3-ium Tetrachlorozinkat 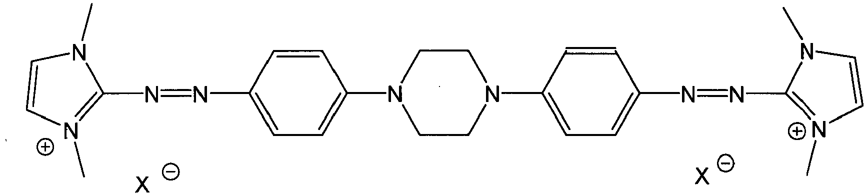
- 2-{2-[4-(4-{4-[2-(1,3-Dimethyl-1H-imidazol-3-ium-2-yl)diazen-1-yl]phenyl}piperazin-1-yl)phenyl]diazen-1-yl}-1,3-dimethyl-1H-imidazol-3-ium Dichlorid
- 2-{2-[4-(4-{4-[2-(1,3-Dimethyl-1H-imidazol-3-ium-2-yl)diazen-1-yl]phenyl}piperazin-1-yl)phenyl]diazen-1-yl}-1,3-dimethyl-1H-imidazol-3-ium Dibromid
- 2-{2-[4-(4-{4-[2-(1,3-Dimethyl-1H-imidazol-3-ium-2-yl)diazen-1-yl]phenyl}piperazin-1-yl)phenyl]diazen-1-yl}-1,3-dimethyl-1H-imidazol-3-ium Sulfat
- 2-{2-[4-(4-{4-[2-(1,3-Dimethyl-1H-imidazol-3-ium-2-yl)diazen-1-yl]phenyl}piperazin-1-yl)phenyl]diazen-1-yl}-1,3-dimethyl-1H-imidazol-3-ium Di(p-toluolsulfonat)
- 2-{2-[4-(4-{4-[2-(1,3-Dimethyl-1H-imidazol-3-ium-2-yl)diazen-1-yl]phenyl}piperazin-1-yl)phenyl]diazen-1-yl}-1,3-dimethyl-1H-imidazol-3-ium Di(methylsulfat)
- 2-{2-[4-(4-{4-[2-(1,3-Dimethyl-1H-imidazol-3-ium-2-yl)diazen-1-yl]phenyl}piperazin-1-yl)phenyl]diazen-1-yl}-1,3-dimethyl-1H-imidazol-3-ium Tetrachlorozinkat 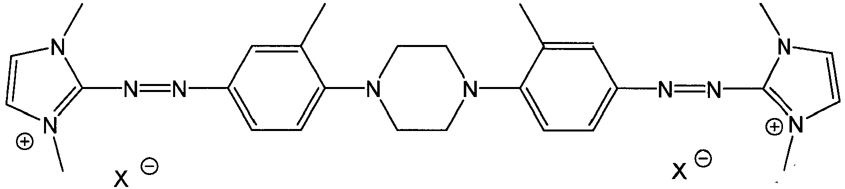
- 2-{2-[4-(4-{4-[2-(1,3-Dimethyl-1H-imidazol-3-ium-2-yl)diazen-1-yl]-2-methylphenyl}piperazin-1-yl)-3-methylphenyl]diazen-1-yl}-1,3-dimethyl-1H-imidazol-3-ium Dichlorid
- 2-{2-[4-(4-{4-[2-(1,3-Dimethyl-1H-imidazol-3-ium-2-yl)diazen-1-yl]-2-methylphenyl}piperazin-1-yl)-3-methylphenyl]diazen-1-yl}-1,3-dimethyl-1H-imidazol-3-ium Dibromid
- 2-{2-[4-(4-{4-[2-(1,3-Dimethyl-1H-imidazol-3-ium-2-yl)diazen-1-yl]-2-methylphenyl}piperazin-1-yl)-3-methylphenyl]diazen-1-yl}-1,3-dimethyl-1H-imidazol-3-ium Sulfat
- 2-{2-[4-(4-{4-[2-(1,3-Dimethyl-1H-imidazol-3-ium-2-yl)diazen-1-yl]-2-methylphenyl}piperazin-1-yl)-3-methylphenyl]diazen-1-yl}-1,3-dimethyl-1H-imidazol-3-ium Di(p-tolulsulfonat)
- 2-{2-[4-(4-{4-[2-(1,3-Dimethyl-1H-imidazol-3-ium-2-yl)diazen-1-yl]-2-methylphenyl}piperazin-1-yl)-3-methylphenyl]diazen-1-yl}-1,3-dimethyl-1H-imidazol-3-ium Di(methylsulfat)
- 2-{2-[4-(4-{4-[2-(1,3-Dimethyl-1H-imidazol-3-ium-2-yl)diazen-1-yl]-2-methylphenyl}piperazin-1-yl)-3-methylphenyl]diazen-1-yl}-1,3-dimethyl-1H-imidazol-3-ium Tetrachlorozinkat 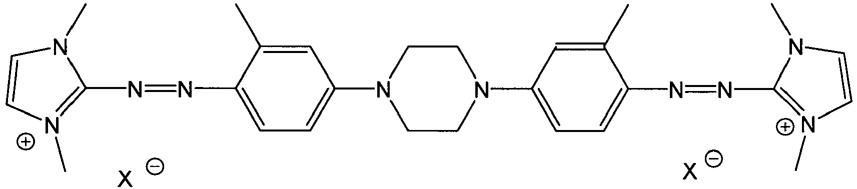
- 2-{2-[4-(4-{4-[2-(1,3-Dimethyl-1H-imidazol-3-ium-2-yl)diazen-1-yl]-3-methylphenyl}piperazin-1-yl)-2-methylphenyl]diazen-1-yl}-1,3-dimethyl-1H-imidazol-3-ium Dichlorid
- 2-{2-[4-(4-{4-[2-(1,3-Dimethyl-1H-imidazol-3-ium-2-yl)diazen-1-yl]-3-methylphenyl}piperazin-1-yl)-2-methylphenyl]diazen-1-yl}-1,3-dimethyl-1H-imidazol-3-ium Dibromid
- 2-{2-[4-(4-{4-[2-(1,3-Dimethyl-1H-imidazol-3-ium-2-yl)diazen-1-yl]-3-methylphenyl}piperazin-1-yl)-2-methylphenyl]diazen-1-yl}-1,3-dimethyl-1H-imidazol-3-ium Sulfat
- 2-{2-[4-(4-{4-[2-(1,3-Dimethyl-1H-imidazol-3-ium-2-yl)diazen-1-yl]-3-methylphenyl}piperazin-1-yl)-2-methylphenyl]diazen-1-yl}-1,3-dimethyl-1H-imidazol-3-ium Di(p-toluolsulfonat)
- 2-{2-[4-(4-{4-[2-(1,3-Dimethyl-1H-imidazol-3-ium-2-yl)diazen-1-yl]-3-methylphenyl}piperazin-1-yl)-2-methylphenyl]diazen-1-yl}-1,3-dimethyl-1H-imidazol-3-ium Di(methylsulfat)
- 2-{2-[4-(4-{4-[2-(1,3-Dimethyl-1H-imidazol-3-ium-2-yl)diazen-1-yl]-3-methylphenyl}piperazin-1-yl)-2-methylphenyl]diazen-1-yl}-1,3-dimethyl-1H-imidazol-3-ium Tetrachlorozinkat 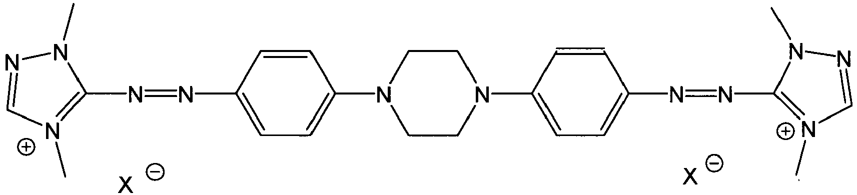
- 5-{2-[4-(4-{4-[2-(1,4-Dimethyl-1H-1,2,4-triazol-4-ium-5-yl)diazen-1-yl]phenyl}piperazin-1-yl)phenyl]diazen-1-yl}-1,4-dimethyl-1H-1,2,4-triazol-4-ium Dichlorid
- 5-{2-[4-(4-{4-[2-(1,4-Dimethyl-1H-1,2,4-triazol-4-ium-5-yl)diazen-1-yl]phenyl}piperazin-1-yl)phenyl]diazen-1-yl}-1,4-dimethyl-1H-1,2,4-triazol-4-ium Dibromid
- 5-{2-[4-(4-{4-[2-(1,4-Dimethyl-1H-1,2,4-triazol-4-ium-5-yl)diazen-1-yl]phenyl}piperazin-1-yl)phenyl]diazen-1-yl}-1,4-dimethyl-1H-1,2,4-triazol-4-ium Sulfat
- 5-{2-[4-(4-{4-[2-(1,4-Dimethyl-1H-1,2,4-triazol-4-ium-5-yl)diazen-1-yl]phenyl}piperazin-1-yl)phenyl]diazen-1-yl}-1,4-dimethyl-1H-1,2,4-triazol-4-ium Di(p-toluolsulfonat)
- 5-{2-[4-(4-{4-[2-(1,4-Dimethyl-1H-1,2,4-triazol-4-ium-5-yl)diazen-1-yl]phenyl}piperazin-1-yl)phenyl]diazen-1-yl}-1,4-dimethyl-1H-1,2,4-triazol-4-ium Di(methylsulfat)
- 5-{2-[4-(4-{4-[2-(1,4-Dimethyl-1H-1,2,4-triazol-4-ium-5-yl)diazen-1-yl]phenyl}piperazin-1-yl)phenyl]diazen-1-yl}-1,4-dimethyl-1H-1,2,4-triazol-4-ium Tetrachlorozinkat 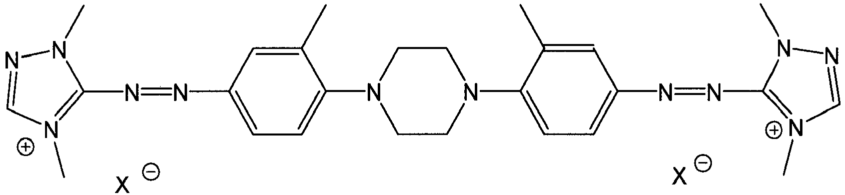
- 5-{2-[4-(4-{4-[2-(1,4-Dimethyl-1H-1,2,4-triazol-4-ium-5-yl)diazen-1-yl]-2-methylphenyl}piperazin-1-yl)-3-methylphenyl]diazen-1-yl}-1,4-dimethyl-1H-1,2,4-triazol-4-ium Dichlorid
- 5-{2-[4-(4-{4-[2-(1,4-Dimethyl-1H-1,2,4-triazol-4-ium-5-yl)diazen-1-yl]-2-methylphenyl}piperazin-1-yl)-3-methylphenyl]diazen-1-yl}-1,4-dimethyl-1H-1,2,4-triazol-4-ium Dibromid
- 5-{2-[4-(4-{4-[2-(1,4-Dimethyl-1H-1,2,4-triazol-4-ium-5-yl)diazen-1-yl]-2-methylphenyl}piperazin-1-yl)-3-methylphenyl]diazen-1-yl}-1,4-dimethyl-1H-1,2,4-triazol-4-ium Sulfat
- 5-{2-[4-(4-{4-[2-(1,4-Dimethyl-1H-1,2,4-triazol-4-ium-5-yl)diazen-1-yl]-2-methylphenyl}piperazin-1-yl)-3-methylphenyl]diazen-1-yl}-1,4-dimethyl-1H-1,2,4-triazol-4-ium Di(p-toluolsulfonat)
- 5-{2-[4-(4-{4-[2-(1,4-Dimethyl-1H-1,2,4-triazol-4-ium-5-yl)diazen-1-yl]-2-methylphenyl}piperazin-1-yl)-3-methylphenyl]diazen-1-yl}-1,4-dimethyl-1H-1,2,4-triazol-4-ium Di(methylsulfat)
- 5-{2-[4-(4-{4-[2-(1,4-Dimethyl-1H-1,2,4-triazol-4-ium-5-yl)diazen-1-yl]-2-methylphenyl}piperazin-1-yl)-3-methylphenyl]diazen-1-yl}-1,4-dimethyl-1H-1,2,4-triazol-4-ium Tetrachlorozinkat 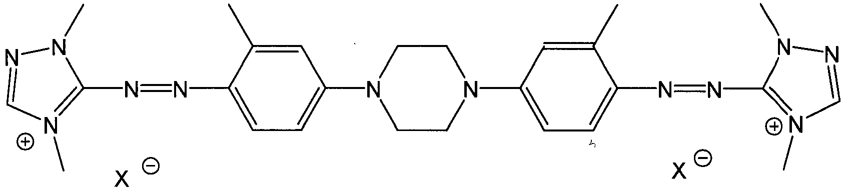
- 5-{2-[4-(4-{4-[2-(1,4-Dimethyl-1H-1,2,4-triazol-4-ium-5-yl)diazen-1-yl]-3-methylphenyl}piperazin-1-yl)-2-methylphenyl]diazen-1-yl}-1,4-dimethyl-1H-1,2,4-triazol-4-ium Dichlorid
- 5-{2-[4-(4-{4-[2-(1,4-Dimethyl-1H-1,2,4-triazol-4-ium-5-yl)diazen-1-yl]-3-methylphenyl}piperazin-1-yl)-2-methylphenyl]diazen-1-yl}-1,4-dimethyl-1H-1,2,4-triazol-4-ium Dibromid
- 5-{2-[4-(4-{4-[2-(1,4-Dimethyl-1H-1,2,4-triazol-4-ium-5-yl)diazen-1-yl]-3-methylphenyl}piperazin-1-yl)-2-methylphenyl]diazen-1-yl}-1,4-dimethyl-1H-1,2,4-triazol-4-ium Sulfat
- 5-{2-[4-(4-{4-[2-(1,4-Dimethyl-1H-1,2,4-triazol-4-ium-5-yl)diazen-1-yl]-3-methylphenyl}piperazin-1-yl)-2-methylphenyl]diazen-1-yl}-1,4-dimethyl-1H-1,2,4-triazol-4-ium Di(p-toluolsulfonat)
- 5-{2-[4-(4-{4-[2-(1,4-Dimethyl-1H-1,2,4-triazol-4-ium-5-yl)diazen-1-yl]-3-methylphenyl}piperazin-1-yl)-2-methylphenyl]diazen-1-yl}-1,4-dimethyl-1H-1,2,4-triazol-4-ium Di(methylsulfat)
- 5-{2-[4-(4-{4-[2-(1,4-Dimethyl-1H-1,2,4-triazol-4-ium-5-yl)diazen-1-yl]-3-methylphenyl}piperazin-1-yl)-2-methylphenyl]diazen-1-yl}-1,4-dimethyl-1H-1,2,4-triazol-4-ium Tetrachlorozinkat

Die erfindungsgemäßen Mittel zur Farbveränderung von keratinischen Fasern enthalten den oder die direktziehenden Farbstoffe der Formel (I) vorzugsweise in einer Gesamtmenge von 0,01 bis 4,5 Gew.-%, bevorzugt von 0,05 bis 2,8 Gew.-%, weiter bevorzugt von 0,1 bis 2,2 Gew.-% und besonders bevorzugt von 0,2 bis 1,2 Gew.-% enthält. Die Mengenangabe in Gewichtsprozent bezieht sich hierbei auf die Gesamtmenge aller im Mittel enthaltenen Verbindungen der Formel (I), die zum Gesamtgewicht des Mittels in Relation gesetzt wird.

In einer weiteren bevorzugten Ausführungsform ist ein erfindungsgemäßes Mittel zur Farbveränderung von keratinischen Fasern daher dadurch gekennzeichnet, dass es - bezogen auf das Gesamtgewicht des Mittels - einen oder mehrere direktziehende Farbstoffe (a) der Formel (I) in einer Gesamtmenge von 0,01 bis 4,5 Gew.-%, bevorzugt von 0,05 bis 2,8 Gew.-%, weiter bevorzugt von 0,1 bis 2,2 Gew.-% und besonders bevorzugt von 0,2 bis 1,2 Gew.-% enthält.

Die Farbstoffe der allgemeinen Formel (I) können beispielsweise in Analogie zu einem Verfahren hergestellt werden, wie es in WO 2002/100369 A2 beschrieben ist.

So kann beispielsweise das Edukt 2-Aminothiazol in konzentrierter Schwefelsäure mit Nitrosylschwefelsäure in das Diazoniumion überführt werden:

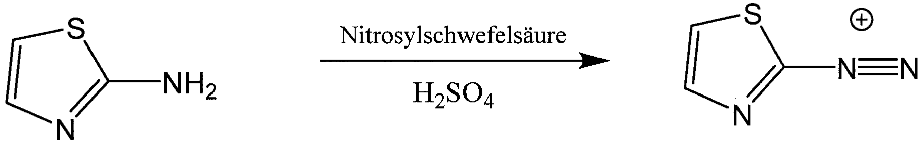

Das reaktive Diazoniumion geht dann mit dimeren Anilin-Derivaten eine doppelte Azokupplungsreaktion ein:

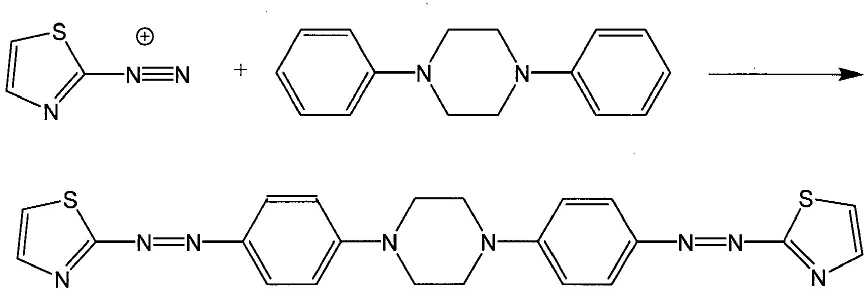

Das in der Azokupplungsreaktion einsetzbare Edukt 1,4-Diphenylpiperazin ist beispielsweise gemäß einem der folgenden Synthesewege herstellbar:
Journal of Heterocyclic Chemistry, 14, p. 535, 1977 The Journal of Organic Chemistry, 52, p. 1673, 1987 Tetrahedron Letters, 27, p. 377, 1986

Der in der Azokupplungsreaktion entstehende neutrale dimere Farbstoff kann dann schließlich mit Quaternierungsmitteln doppelt quaterniert werden. Die Quaternierungsreaktion wird bevorzugt in einem polaren aprotischen Lösungsmittel (wie beispielsweise DMSO, DMF etc.) durchgeführt. Als Quaternierungsmittel kommen zum Beispiel Dimethylsulfat, Methylbromid oder p-Toluolsulfonat in Frage.

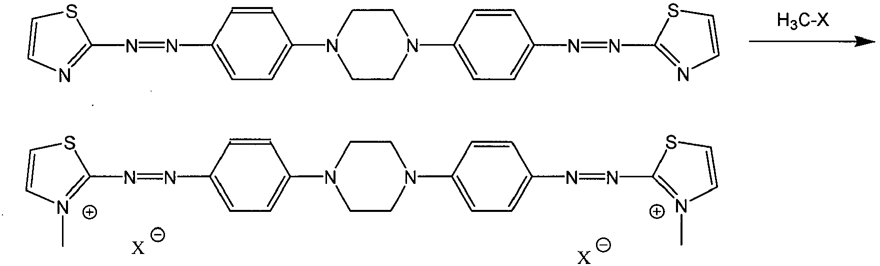

Ein weiteres Herstellverfahren dieser Farbstoffe wird beispielsweise in GB 1186753 offenbart.

Die erfindungsgemäßen Mittel enthalten zusätzlich mindestens ein Tensid. Hierbei hat sich herausgestellt, dass insbesondere dann sehr intensive Farbergebnisse erhalten werden konnten, wenn die erfindungsgemäßen Mittel zusätzlich mindestens ein anionisches Tensid (b) enthielten.

Tenside sind amphiphile (bifunktionelle) Verbindungen, die aus mindestens einem hydrophoben und mindestens einem hydrophilen Molekülteil bestehen. Bei dem hydrophoben Rest handelt es sich bevorzugt eine Kohlenwasserstoffkette mit 8-24 Kohlenstoff-Atomen, die gesättigt oder ungesättigt, linear oder verzweigt sein kann. Besonders bevorzugt ist diese C₈-C₂₄-Alkylkette linear.

Bei anionischen Tensiden umfasst der hydrophile Molekülteil eine negativ geladene hydrophile Kopfgruppe. Bei der negativ geladenen hydrophilen Kopfgruppe kann es sich beispielsweise um eine Carbonsäuregruppe bwz. das Salz einer Carbonsäuregruppe, eine Sulfonsäuregruppe bzw. das Salz der Sulfonsäuregruppe, eine Schwefelsäureestergruppierung bzw. das Salz hiervon, eine Phosphonsäuregruppe bzw. das Salz der Phosphonsäuregruppe, oder eine Phosphorsäureestergruppierung bzw. das Salz hiervon handeln.

Üblicherweise umfasst das erfindungsgemäße kosmetische Mittel einen wässrigen Träger. In wässriger Lösung liegen die vorgenannten hydrophilen Kopfgruppen des anionischen Tensids - wie beispielsweise die Carbonsäure und die Salze der Carbonsäuren - in einem Gleichgewicht vor, dessen Lage vom pH-Wert des Mittels mitbestimmt wird. Wird als anionisches Tensid somit beispielsweise eine Fettsäure eingesetzt, so liegt ein geringer Teil der Fettsäure in wässriger Lösung in Form der protonierten Fettsäure vor, wohingegen der Großteil der Fettsäure in wässriger Lösung deprotoniert und auf diesem Wege in das Salz der Fettsäure überführt wird. Aus diesem Grund umfasst die Definition eines anionischen Tensid auch ein Tenside mit einer - noch protonierten - Säuregruppe.

Ein anionisches Tensid (b) im Sinne der vorliegenden Erfindung enthält keine kationischen Gruppierungen, d.h. zwitterionische Tenside sind von der Definition eines anionischen Tensids nicht mit umfasst.

Erfindungsgemäße anionische Tenside sind demnach gekennzeichnet durch die Anwesenheit einer wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder
Polyglykolether-Gruppen, Ester-, Ether- und Amidgruppen sowie Hydroxylgruppen enthalten sein. Typische Beispiele für anionische Tenside sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Acyllactylate, Acyltartrate, Acylglutamate, Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Beispiele für erfindungsgemäße anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe, die durch Veresterung von Fettsäuren mit dem Natriumsalz der 2-Hydroxyethan-sulfonsäure (Isethionsäure) zugänglich sind. Wenn man für diese Veresterung Fettsäuren mit 8 bis 24 C-Atomen, also z. B. Laurin-, Myristin-, Palimitin- oder Stearinsäure oder auch technische Fettsäurefraktionen, z. B. die aus Kokosfettsäure erhältliche C₁₂ - C₁₈-Fettsäurefraktion einsetzt, erhält man die erfindungsgemäß bevorzugt geeigneten C₁₂ - C₁₈-Acylisethionate,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen. Sulfobernsteinsäuremono- und -dialkylester können durch Umsetzung von Maleinsäureanhydrid mit einem Fettalkohol mit 8 - 24 C-Atomen zum Maleinsäuremonoester des Fettalkohols und Weiterreaktion mit Natriumsulfit zum Sulfobernsteinsäureester hergestellt werden. Besonders geeignete Sulfobernsteinsäureester leiten sich von Fettalkoholfraktionen mit 12 - 18 C-Atomen ab, wie sie z. B. aus Kokosfettsäure oder Kokosfettsäuremethylester durch Hydrierung zugänglich sind,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Hydroxysulfonate im wesentlichen entsprechend mindestens einer der beiden folgenden Formeln oder deren Mischungen sowie deren salzen, CH₃-(CH₂)_{y}-CHOH-(CH₂)ₚ-(CH-SO₃M)-(CH₂)_{z}-CH₂-O-(CₙH₂ₙO)ₓ-H, und/oder CH₃-(CH₂)_{y}-(CH-SO₃M)-(CH₂)ₚ-CHOH-(CH₂)_{z}- CH₂-O-(CₙH₂ₙO)ₓ-H wobei in beiden Formeln y und z = 0 oder ganze Zahlen von 1 bis 18, p = 0, 1 oder 2 und die Summe (y+z+p) eine Zahl von 12 bis 18, x = 0 oder eine Zahl von 1 bis 30 und n eine ganze Zahl von 2 bis 4 sowie M = H oder Alkali-, insbesondere Natrium, Kalium, Lithium, Erdalkali- , insbesondere Magnesium, Calcium, Zink und/oder einem Ammoniumion, welches gegebenenfalls substituiert sein kann, insbesondere Mono-, Di-, Tri- oder Tetraammoniumionen mit C1 bis C4 Alkyl-, Alkenyl- oder Arylresten,
- sulfatierte Hydroxyalkylpolyethylen- und/oder Hydroxyalkylenpropylenglykolether der Formel R¹-(CHOSO₃M)-CHR³-(OCHR⁴-CH₂)n-OR² mit R¹, einem linearen Alkylrest mit 1 bis 24 C-Atomen, R² für einen linearen oder verzweigten, gesättigten Alkylrest mit 1 bis 24 C-Atomen, R³ für Wasserstoff oder einen linearen Alkylrest mit 1 bis 24 C-Atomen, R⁴ für Wasserstoff oder einen Methylrest und M für Wasserstoff, Ammonium, Alkylammonium, Alkanolammonium, worin die Alkyl- und Alkanolreste je 1 bis 4 C-Atome aufweisen, oder ein Metallatom ausgewählt aus Lithium, Natrium, Kalium, Calcium oder Magnesium und n für eine Zahl im Bereich von 0 bis 12 stehen und weiterhin die Gesamtzahl der in R¹ und R³ enthaltenen C-Atome 2 bis 44 beträgt,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel,

   R¹(OCH₂CH₂)ₙ-O-(PO-OX)-OR²,

   in der R¹ bevorzugt für einen aliphatischen Kohlenwasserstoffrest mit 8 bis 30 Kohlenstoffatomen, R² für Wasserstoff, einen Rest (CH₂CH₂O)ₙR² oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C₁ bis C₄ - Kohlenwasserstoffrest, steht,
- sulfatierte Fettsäurealkylenglykolester der Formel RCO(AlkO)ₙSO₂M
   in der RCO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Metall steht, wie Alkalimetall, insbesondere Natrium, Kalium, Lithium, Erdalkalimetall, insbesondere Magnesium, Calcium, Zink, oder Ammoniumion, wie ⁺NR³R⁴R⁵R⁶, mit R³ bis R⁶ unabhängig voneinander stehend für Wasserstoff oder einen C1 bis C4 - Kohlenwasserstoffrest,
- Monoglyceridsulfate und Monoglyceridethersulfate der Formel

   R⁸OC-(OCH₂CH₂)ₓ-OCH₂-[CHO(CH₂CH₂O)_{y}H]-CH₂O(CH₂CH₂O)_{z}-SO₃X,

   in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate eingesetzt, in der R⁸CO für einen linearen Acylrest mit 8 bis 18 Kohlenstoffatomen steht,
- Amidethercarbonsäuren, R¹-CO-NR²-CH₂CH₂-O-(CH₂CH₂O)ₙCH₂COOM, mit R¹ als geradkettigen oder verzweigten Alkyl- oder Alkenylrest mit einer Zahl an Kohlenstoffatomen in der Kette von 2 bis 30, n steht für eine ganze Zahl von 1 bis 20 und R² steht für Wasserstoff, einen Methyl-, Ethyl-, Propyl-, Isopropyl-, n-Butyl-, t-Butyl- oder iso-Butylrest und M steht für Wasserstoff oder ein Metall wie Alkalimetall, insbesondere Natrium, Kalium, Lithium, Erdalkalimetall, insbesondere Magnesium, Calcium, Zink, oder ein Ammoniumion, wie ⁺NR³R⁴R⁵R⁶, mit R³ bis R⁶ unabhängig voneinander stehend für Wasserstoff oder einen C1 bis C4 - Kohlenwasserstoffrest. Derartige Produkte sind beispielsweise von der Firma Chem-Y unter der Produktbezeichnung Akypo® erhältlich, und
- Acylglutamate der Formel XOOC-CH2CH2CH(C(NH)OR)-COOX, in der RCO für einen linearen oder verzweigten Acylrest mit 6 bis 22 Kohlenstoffatomen und 0 und/oder 1, 2 oder 3 Doppelbindungen und X für Wasserstoff, ein Alkali- und/oder Erdalkalimetall, Ammonium, Alkylammonium, Alkanolammonium oder Glucammonium steht.

Die Behandlung von keratinischen Fasern mit Mitteln, die (a) mindestens einen direktiehenden Farbstoff der Formel (I) und (b) mindestens ein anionisches Tensid enthielten, führte zu besonders intensiven Färbungen in attraktiven Nuanncen. Hierbei hat sich überraschenderweise gezeigt, dass das Farbaufzugsvermögen durch Einsatz eines oder mehrere spezieller anionischer Tenside noch weiter optimiert werden konnte. Besonders intensive Blaufärbungen wurden erhalten, wenn die Farbstoffe (a) der Formel (I) mit mindestens einem anionischen Tensid (b) aus der Gruppe der
- linearen und verzweigten Fettsäuren mit 8 bis 30 C-Atomen,
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe, die durch Veresterung von Fettsäuren mit dem Natriumsalz der 2-Hydroxyethan-sulfonsäure (Isethionsäure) zugänglich sind. Wenn man für diese Veresterung Fettsäuren mit 8 bis 24 C-Atomen, also z. B. Laurin-, Myristin-, Palimitin- oder Stearinsäure oder auch technische Fettsäurefraktionen, z. B. die aus Kokosfettsäure erhältliche C₁₂ - C₁₈-Fettsäurefraktion einsetzt, erhält man die erfindungsgemäß bevorzugt geeigneten C₁₂ - C₁₈-Acylisethionate,
- Amidethercarbonsäuren, R¹-CO-NR²-CH₂CH₂-O-(CH₂CH₂O)ₙCH₂COOM, mit R¹ als geradkettigen oder verzweigten Alkyl- oder Alkenylrest mit einer Zahl an Kohlenstoffatomen in der Kette von 2 bis 30, n steht für eine ganze Zahl von 1 bis 20 und R² steht für Wasserstoff, einen Methyl-, Ethyl-, Propyl-, Isopropyl-, n-Butyl-, t-Butyl- oder iso-Butylrest und M steht für Wasserstoff oder ein Metall wie Alkalimetall, insbesondere Natrium, Kalium, Lithium, Erdalkalimetall, insbesondere Magnesium, Calcium, Zink, oder ein Ammoniumion, wie ⁺NR³R⁴R⁵R⁶, mit R³ bis R⁶ unabhängig voneinander stehend für Wasserstoff oder einen C1 bis C4 - Kohlenwasserstoffrest. Derartige Produkte sind beispielsweise von der Firma Chem-Y unter der Produktbezeichnung Akypo® erhältlich, und
- Acylglutamate der Formel XOOC-CH2CH2CH(C(NH)OR)-COOX, in der RCO für einen linearen oder verzweigten Acylrest mit 6 bis 22 Kohlenstoffatomen und 0 und/oder 1, 2 oder 3 Doppelbindungen und X für Wasserstoff, ein Alkali- und/oder Erdalkalimetall, Ammonium, Alkylammonium, Alkanolammonium oder Glucammonium steht.

In einer weiteren besonders bevorzugten Ausführungsform ist ein erfindungsgemäßes Mittel daher dadurch gekennzeichnet, dass es (b) mindestens ein anionisches Tensid enthält, das ausgewählt ist aus den
- linearen und verzweigten Fettsäuren mit 8 bis 30 C-Atomen,
- Ethercarbonsäuren der Formel R-O-(CH₂-CH₂O)ₓ-CH₂-COOM, in der
   - R: für eine lineare Alkylgruppe mit 8 bis 30 C-Atomen steht,
   - x: für eine ganze Zahl von 0 bis 16 steht,
   - M: für Wasserstoff oder ein Metall wie ein Alkalimetall, insbesondere Natrium, Kalium, Lithium, ein Erdalkalimetall, insbesondere ½ Magnesium, ½ Calcium, ½ Zink, oder ein Ammoniumion (NH4⁺) steht,
- Acylsarcosiden mit 8 bis 24 C-Atomen in der Acylgruppe,
- Acyltauriden mit 8 bis 24 C-Atomen in der Acylgruppe,
- Acylisethionatne mit 8 bis 24 C-Atomen in der Acylgruppe, die durch Veresterung von C₈-C₂₄-Fettsäuren mit dem Natriumsalz der 2-Hydroxyethan-sulfonsäure (Isethionsäure) zugänglich sind,
- Amidethercarbonsäuren, R¹-CO-NR²-CH₂CH₂-O-(CH₂CH₂O)_{y}CH₂COOM, wobei
   - R¹: für eine C2-C30-Alkylgruppe steht,
   - y: für eine ganze Zahl von 1 bis 20 steht,
   - R²: für Wasserstoff, einen Methyl-, Ethyl-, Propyl-, Isopropyl-, n-Butyl-, t-Butyl- oder iso-Butylrest steht, und
   - M: für Wasserstoff oder ein Metall wie ein Alkalimetall, insbesondere Natrium, Kalium, Lithium, ein Erdalkalimetall, insbesondere ½ Magnesium, ½ Calcium, ½ Zink, oder ein Ammoniumion (NH4⁺) steht.

Ein besonders gutes Farbaufzugsvermögen konnte beobachtet werden, wenn als Aniontensid (b) mindestens eine Ethercarbonsäuren der Formel R-O-(CH2-CH2O)x-CH2-COOH oder ihr Salz eingesetzt wurde. Der Einsatz von - gegebenenfalls ethoxylierten - Ethercarbonsäuren ist daher explizit ganz besonders bevorzgut.

In einer explizit ganz besonders bevorzugten Ausführungsform ist ein erfindungsgemäßes Mittel daher dadurch gekennzeichnet, dass es mindestens ein anionisches Tensid (b) aus der Gruppe der Ethercarbonsäuren der Formel (B1) enthält

R-O-(CH2-CH2O)x-CH2-COOM (B1),

wobei
- R: für eine lineare Alkylgruppe mit 8 bis 30 C-Atomen steht,
- x: für eine ganze Zahl von 0 bis 16 steht,
- M: für Wasserstoff oder ein Metall wie ein Alkalimetall, insbesondere Natrium, Kalium, Lithium, ein Erdalkalimetall, insbesondere ½ Magnesium, ½ Calcium, 1/2 Zink, oder ein Ammoniumion (NH4⁺) steht.

Weiterhin besonders bevorzugt steht x für die Zahlen 5, 6 oder 7.

In einer weiterhin ganz besonders bevorzugten Ausführungsform ist ein erfindungsgemäßes Mittel daher dadurch gekennzeichnet, dass es als anionisches Tensid (b) mindestens eine Ethercarbonsäure der Formel (B1) enthält

R-O-(CH2-CH2O)x-CH2-COOM (B1),

wobei
- R: für eine lineare Alkylgruppe mit 8 bis 30 C-Atomen steht,
- x: für die ganze Zahl von 5 bis 11, insbesondere für die Zahlen 5, 6 oder 7, steht,
- M: für Wasserstoff oder ein Metall wie ein Alkalimetall, insbesondere Natrium, Kalium, Lithium, ein Erdalkalimetall, insbesondere ½ Magnesium, ½ Calcium, ½ Zink, oder ein Ammoniumion (NH4⁺) steht.

Die ganz besonders bevorzugten anionischen Tenside der Formel (B1) sind beispielsweise unter dem Handelsnamen erhältlich
- Akypo Soft 45 HP der Firma Kao (Natrium Laureth-6 Carboxylat)
- Akypo Soft 45 NV der Firma Kao (Natrium Laureth-5 Carboxylat)
- Akypo RLM 100 NV der Firma Kao (Natrium Laureth-11 Carboxylat)

Bevorzugt enthalten die erfindungsgemäßen Mittel zum Färben von keratinischen Fasern ein oder mehrere anionische Tenside (b) in einer Gesamtmenge von 0,05 bis 4,5 Gew.-%, bevorzugt von 0,1 bis 2,1 Gew.-%, weiter bevorzugt von 0,15 bis 1,8 Gew.-% und ganz besonders bevorzugt von 0,2 bis 0,9 Gew.-%. Hierbei sind die Angaben in Gew.-% auf die Gesamtmenge aller anionischen Tenside (b) bezogen, die zur Gesamtmenge des Färbemittels in Relation gesetzt werden.

In einer weiteren besonders bevorzugten Ausführungsform ist ein erfindungsgemäßes Mittel zur Farbveränderung von keratinischen Fasern daher dadurch gekennzeichnet, dass es - bezogen auf das Gesamtgewicht des Mittels - ein oder mehrere anionische Tenside (b) in einer Gesamtmenge von 0,05 bis 9,5 Gew.-%, bevorzugt von 0,1 bis 3,1 Gew.-%, weiter bevorzugt von 0,15 bis 2,5 Gew.-% und ganz besonders bevorzugt von 0,2 bis 0,9 Gew.-% enthält.

Wie zuvor beschrieben hat sich gezeigt, dass durch Variation des bzw. der anionischen Tenside (b) das Farbaufzugsvermögen der direktziehenden Farbstoffe der Formel (I) beeinflusst werden kann.

Generell kann der Farbaufzug durch Einsatz mindestens eines anionischen Tensids verbessert werden. Der Einsatz mindestens einer - gegebenenfalls ethoxylierten - Ethercarbonsäure und/oder ihres Salzes, wie sie in Formel (B1) beschrieben ist, hat sich in diesem Zusammenhang als besonders vorteilhaft erwiesen. Es wurde beobachtet, dass die Keratinfasern sich insbesondere dann in besonders intensiven Nuancen färben ließen, wenn das bzw. die Ethercarbonsäuren (und/oder ihre Salze) als das hauptsächliche anionische Tensid im erfindungsgemäßen Mittel enthalten waren.

Mit anderen Worten waren die Färbeergebnisse dann besonders intensiv, wenn die erfindungsgemäßen Mitteln ein oder mehrere Ethercarbonsäuren der Formel (B1) enthielten, und wenn darüber hinaus alle weiteren eingesetzten anionischen Tenside nur in geringeren Mengen vorhanden waren. Der Einsatz der Ethercarbonsäure(n) der Formel (B1) als Haupt-Tensid lässt sich durch Angabe eines Gewichtsverhältnisses quantifizieren, welches die Gesamtmenge der im Mittel enthaltenen anionischen Tenside der Formel (B1) zur Gesamtmenge aller im Mittel enthaltenen anionischen Tenside (b) in Relation setzt.

In einer weiteren besonders bevorzugten Ausführungsform ist ein erfindungsgemäßes Mittel zur Farveränderung von keratinischen Fasern daher dadurch gekennzeichnet, dass das Gewichtsverhältnis aus allen im Mittel enthaltenen anionischen Tensiden der Formel (B1) zur Gesamtmenge der im Mittel enthaltenen anionischen Tenside bei einem Wert von 0,5, bevorzugt von 0,6, weiter bevorzugt von 0,75 und besonders bevorzugt von 0,9 liegt,
wobei es sich bei den anionischen Tensiden der Formel (B1) um folgende Tenside handelt

R-O-(CH2-CH2O)x-CH2-COOM (B1),

mit
- R: gleich einer linearen Alkylgruppe mit 8 bis 30 C-Atomen,
- x: gleich einer ganzen Zahl von 0 bis 16,
- M: gleich Wasserstoff oder einem Metall wie ein Alkalimetall, insbesondere Natrium, Kalium, Lithium, ein Erdalkalimetall, insbesondere ½ Magnesium, ½ Calcium, ½ Zink, oder ein Ammoniumion (NH4⁺).

Beispiel: Ein Mittel zum Färben von keratinischen Fasern enthält:
(a) 1,0 g 3-Methyl-2-{2-[4-(4-{4-[2-(3-methyl-1,3-thiazol-3-ium-2-yl)diazen-1-yl]phenyl}piperazin-1-yl)phenyl]diazen-1-yl}-1,3-thiazol-3-ium Di(methylsulfat)
(b) 0,23 g Natrium laureth-6 carboxylat (B1) und
(b) 0,10 g Natriumlaureth-Sulfat (2 EO)

Das Gewichtsverhältnis aus allen im Mittel enthaltenen anionischen Tensiden der Formel (B1) zur Gesamtmenge der im Mittel enthaltenen anionischen Tenside liegt bei einem Wert von [0,23 / (0,23 + 0,1)] = 0,70

Beispiel: Ein Mittel zum Färben von keratinischen Fasern enthält:
(a) 0,5 g 3-Methyl-2-{2-[4-(4-{4-[2-(3-methyl-1,3-thiazol-3-ium-2-yl)diazen-1-yl]phenyl}piperazin-1-yl)phenyl]diazen-1-yl}-1,3-thiazol-3-ium Di(methylsulfat)
(b) 0,23 g Natrium laureth-6 carboxylat (B1) und
keine weiteren anionischen Tenside

Das Gewichtsverhältnis aus allen im Mittel enthaltenen anionischen Tensiden der Formel (B1) zur Gesamtmenge der im Mittel enthaltenen anionischen Tenside liegt bei einem Wert von [0,23 / 0,23] = 1,0

Weiterhin können die erfindungsgemäßen Mittel auch ein oder mehrere kationische Tenside enthalten. Unter kationischen Tensiden werden Tenside, also grenzflächenaktive Verbindungen, mit jeweils einer oder mehreren positiven Ladungen verstanden. Kationische Tenside enthalten ausschließlich positive Ladungen. Üblicherweise sind diese Tenside aus einem hydrophoben Teil und einer hydrophilen Kopfgruppe aufgebaut, wobei der hydrophobe Teil in der Regel aus einem Kohlenwasserstoff-Gerüst (z.B. bestehend aus einer oder zwei linearen oder verzweigten Alkylketten) besteht, und die positive(n) Ladung(en) in der hydrophilen Kopfgruppe lokalisiert sind. Kationische Tenside adsorbieren an Grenzflächen und aggregieren in wässriger Lösung oberhalb der kritischen Micellbildungskonzentration zu positiv geladenen Micellen.

Beispiele für Kationtenside sind
- quartäre Ammoniumverbindungen, die als hydrophobe Reste ein oder zwei Alkylketten mit einer Kettenlänge von 8 bis 28 C-Atomen tragen können
- quartäre Phosphoniumsalze, substituert mit einer oder mehrern Alkylketten mit einer einer Kettenlänge von 8 bis 28 C-Atomen oder
- tertiäre Sulfonium-Salze.

Weiterhin kann die kationische Ladung auch in Form einer Onium-Struktur Bestandteil eines heterozyklischen Ringes (z.B. eines Imidazoliumringe oder einer Pyridiniumringes) sein.

Neben der funktionellen Einheit, welche die kationische Ladung trägt, kann das Kationtensid auch weitere ungeladene funktionelle Gruppen beinhalten, diese ist beispielsweise bei Esterquats der Fall. Geeignete kationische Tenside dieses Typs sind beispielsweise physiologisch verträgliche Salze des N,N,N-Trimethyl-1-hexadecanaminiums, insbesondere N,N,N-Trimethyl-1-hexadecanaminiumchlorid, welches auch unter dem Handelsnamen Dehyquart A-CA vertrieben wird.

Bei einem weiteren geeigneten kationischen Tensid handelt es sich um ein physiologisch verträgliches Salze des Dimethyl-distearyldimethylammoniums, besonders bevorzugt um Dimethyldistearylammoniumchlorid.

Weitere kationische Tenside können aus der Gruppe der kationischen Imidazoliumverbindungen ausgewählt werden.

Das erfindungsgemäße Mittel kann das bzw. die kationischen Tenside in einer Gesamtmenge von 0,1 bis 4,8 Gew.-%, bevorzugt von 0,2 bis 2,4 Gew.-%, weiter bevorzugt von 0,3 bis 1,8 Gew.-%- bezogen auf das Gesamtgewicht des Mittels - enthalten.

Weiterhin hat es sich als vorteilhaft erwiesen, wenn die Mittel weitere, nichtionogene grenzflächenaktive Stoffe enthalten. Bevorzugte nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge der anwendungsbereiten Mittel, eingesetzt.

In einer weiteren bevorzugten Ausführungsform enthalten die erfindungsgemäßen Mittel neben der Verbindung der Formel (I) zusätzlich mindestens einen weiteren direktziehenden Farbstoff. Durch die Kombination mit weiteren kationischen direktziehenden Farbstoffen lässt sich das erzielbare Nuancenspektrum erweitern, und die Färbeeigenschaften lassen sich noch weiter verbessern. Direktziehende Farbstoffe können in anionische, kationische und nichtionische direktziehende Farbstoffe unterteilt werden.

Die zusätzlich im Mittel enthaltenen direktziehenden Farbstoffe sind vorzugsweise ausgewählt aus den kationischen direktziehenden Farbstoffen, da diese mit den Farbstoffen der Formel (I) gut kompatibel sind.

In einer weiteren besonders bevorzugten Ausführungsform ist ein erfindungsgemäßes Mittel daher dadurch gekennzeichnet, dass es zusätzlich mindestens einen weiteren kationischen direktziehenden Farbstoff enthält, der von den Farbstoffen der Formel (I) verschieden ist.

Als besonders gut kompatibel haben sich ein oder mehrere Farbstoffe aus der Gruppe Basic Yellow 87, Basic Orange 31, Basic Red 51, Basic Violet 2 und Cationic Blue 347 erwiesen.

Ganz besonders gut kompatibel sind die Farbstoffe der Formel (I) mit den kationischen Azofarbstoffen Basic Orange 31 und Basic Red 51. Durch Kombination eines Farbstoffes der Formel (I) mit Basic Orange 31 und/oder Basic Red 51 können - außer rein gelben Nuancen - Nuancen im gesamten Farbspektrum erzeugt werden.

In einer weiteren besonders bevorzugten Ausführungsform ist ein erfindungsgemäßes Mittel dadurch gekennzeichnet, dass es zusätzlich Basic Orange 31 und/oder Basic Red 51 enthält.

Das erfindungsgemäße Mittel kann aber auch zusätzlich mindestens einen nichtionischen direktziehenden Farbstoff enthalten. Dieser kann ausgewählt sein aus der Gruppe HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Zusätzlich können auch anionische direktziehende Farbstoffe enthalten sein, die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten sind. Die erfindungsgemäßen Mittel können weiterhin auch zusammen mit Oxidationsfärbemitteln eingesetzt werden. Solche Oxidationsfärbemittel enthalten zusätzlich mindestens ein Oxidationsfarbstoffvorprodukt, bevorzugt zumindest ein Oxidationsfarbstoffvorprodukt vom Entwickler-Typ und mindestens ein Oxidationsfarbstoffvorprodukt vom Kuppler-Typ. Besonders geeignete Oxidationsfarbstoffvorprodukte vom Entwickler-Typ sind dabei ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)-phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind dabei ausgewählt aus der Gruppe, gebildet aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

Die zusätzlichen direktziehenden Farbstoffe, Entwicklerkomponenten und Kupplerkomponenten werden bevorzugt jeweils in einem Anteil von 0,0001 bis 5,0 Gew.-%, vorzugsweise 0,001 bis 3,5 Gew.-%, jeweils bezogen auf das anwendungsbereite Mittel, verwendet. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Soll die Färbung mit den erfindungsgemäßen direktziehenden Farbstoffen der Formel (I) und eine oxidative Aufhellung der Keratinfasern in einem Schritt erfolgen, so enthalten die erfindungsgemäßen Mittel zusätzlich ein Oxidationsmittel, bevorzugt Wasserstoffperoxid und/oder eines seiner festen Anlagerungsprodukte an organische oder anorganische Verbindungen.

In einer bevorzugten Ausführungsform wird Wasserstoffperoxid selbst als wässrige Lösung verwendet. Die Konzentration einer Wasserstoffperoxid-Lösung im erfindungsgemäßen Mittel wird einerseits von den gesetzlichen Vorgaben und andererseits von dem gewünschten Effekt bestimmt; vorzugsweise werden 6 bis 12 Gew.-%ige Lösungen in Wasser verwendet. Erfindungsgemäß bevorzugte anwendungsbereite Mittel des ersten Erfindungsgegenstands sind dadurch gekennzeichnet, dass sie, bezogen auf das Gesamtgewicht des anwendungsbereiten Mittels, 0,5 bis 20 Gew.-%, vorzugsweise 1 bis 12,5 Gew.-%, besonders bevorzugt 2,5 bis 10 Gew.-% und insbesondere 3 bis 6 Gew.-% Wasserstoffperoxid, jeweils bezogen auf das Gesamtgewicht des Mittels, enthalten.

In einer weiteren besonders bevorzugten Ausführungsform ist ein erfindungsgemäßes Mittel dadurch gekennzeichnet, dass es - bezogen auf das Gesamtgewicht des Mittels - 0,5 bis 12,5 Gew.-%, bevorzugt 2,5 bis 10 Gew.-% und insbesondere 3 bis 6 Gew.-% Wasserstoffperoxid enthält.

Zur Erzielung einer verstärkten Aufhell- und Bleichwirkung kann das Mittel weiterhin mindestens ein Peroxo-Salz enthalten. Geeignete Peroxo-Salze sind anorganische Peroxoverbindungen, bevorzugt ausgewählt aus der Gruppe, gebildet aus Ammoniumperoxodisulfat, Alkalimetallperoxodisulfaten, Ammoniumperoxomonosulfat, Alkalimetallperoxomonosulfaten, Alkalimetallperoxodiphosphaten und Erdalkalimetallperoxiden. Besonders bevorzuge sind Peroxodisulfate, insbesondere Ammoniumperoxodisulfat, Kaliumperoxodisulfat und Natriumperoxodisulfat.

In einer weiteren besonders bevorzugten Ausführungsform ist ein erfindungsgemäßes Mittel dadurch gekennzeichnet, dass es zusätzlich mindestens ein Persulfat aus der Gruppe Ammoniumperoxodisulfat, Kaliumperoxodisulfat und Natriumperoxodisulfat enthält.

Die Persulfate sind jeweils in einer Menge von 0,5 bis 20 Gew.-%, vorzugsweise 1 bis 12,5 Gew.-%, besonders bevorzugt 2,5 bis 10 Gew.-% und insbesondere 3 bis 6 Gew.-%, bezogen auf das Gesamtgewicht des anwendungsbereiten Mittels, in dem erfindungsgemäßen Mittel enthalten.

Bei Ammoniumperoxodisulfat handelt es sich um eine Verbindung der Formel (NH₄)₂S₂O₈. Bei Kaliumperoxodisulfat handelt es sich um eine Verbindung der Formel K₂S₂O₈. Bei Natrjiumperoxodisulfat handelt es sich um eine Verbindung der Formel Na₂S₂O₈.

In einer weiteren besonders bevorzugten Ausführungsform ist ein erfindungsgemäßes Mittel dadurch gekennzeichnet, dass es - bezogen auf das Gesamtgewicht des Mittels - ein oder mehrere Persulfate aus der Gruppe Ammoniumperoxodisulfat, Kaliumperoxodisulfat und Natriumperoxodisulfat in einer Gesamtmenge von 0,5 bis 20 Gew.-%, vorzugsweise von 1,0 bis 12,5 Gew.-%, weiter bevorzugt von 2,5 bis 10 Gew.-% und besonders bevorzugt von 3,0 bis 6,0 Gew.-% enthält.

In einer weiteren besonders bevorzugten Ausführungsform ist ein erfindungsgemäßes Mittel dadurch gekennzeichnet, dass es - bezogen auf das Gesamtgewicht des Mittels - (c) ein oder mehrere Persulfate aus der Gruppe Ammoniumperoxodisulfat, Kaliumperoxodisulfat und Natriumperoxodisulfat in einer Gesamtmenge von 0,5 bis 20 Gew.-%, vorzugsweise von 1,0 bis 12,5 Gew.-%, weiter bevorzugt von 2,5 bis 10 Gew.-% und besonders bevorzugt von 3,0 bis 6,0 Gew.-% enthält.

Die erfindungsgemäßen direktziehenden Farbstoffe der allgemeinen Formel (I) haben sich gegenüber bestimmten Gemischen aus Ammoniumperoxodisulfat, Kaliumperoxodisulfat und Natriumperoxodisulfat als besonders stabil erwiesen.

In einer weiteren besonders bevorzugten Ausführungsform ist ein erfindungsgemäßes Mittel dadurch gekennzeichnet, dass es - bezogen auf das Gesamtgewicht des Mittels -
- 0,2 bis 13,5 Gew.-%, bevorzugt 0,5 bis 1,5 Gew.-%, Ammoniumperoxodisulfat
- 0,5 bis 4,5 Gew.-%, bevorzugt 1,5 bis 2,5 Gew.-%, Kaliumperoxodisulfat und
- 0,2 bis 1,8 Gew.-%, bevorzugt 0,4 bis 0,8 Gew.-%, Natriumperoxodisulfat enthält.

Das Färbe- und/oder Mattier-Mittel kann zur Verstärkung der Blondierwirkung weitere Bleichkraftverstärker enthalten, wie beispielsweise Tetraacetylethylendiamin (TAED), 1,5-Diacetyl-2,4-dioxohexahydro-1,3,5-triazin (DADHT), Tetraacetylglykoluril (TAGU), N-Nonanoylsuccinimid (NOSI), n-Nonanoyl- oder Isononanoyloxybenzolsulfonat (n- bzw. i-NOBS), Phthalsäureanhydrid, Triacetin, Ethylenglykoldiacetat und 2,5-Diacetoxy-2,5-dihydrofuran sowie Carbonatsalze bzw. Hydrogencarbonatsalze, insbesondere Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat, und stickstoffhaltige, heterocyclische Bleichkraftverstärker, wie 4-Acetyl-1-methylpyridinium-p-toluolsulfonat, 2-Acetyl-1-methylpyridinium-p-toluolsulfonat, sowie N-Methyl-3,4-dihydroisochinolinium-p-toluolsulfonat.

Zur weiteren Steigerung der Aufhellung kann der erfindungsgemäßen Zusammensetzung zusätzlich mindestens eine SiO₂-Verbindung, wie Kieselsäure oder Silicate, insbesondere Wassergläser, zugesetzt sein. Es kann erfindungsgemäß bevorzugt sein, die SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder.

Die Färbemittel können weiterhin zusätzliche Wirk-, Hilfs- und Zusatzstoffe enthalten, um die Färbeleistung zu verbessern und weitere gewünschte Eigenschaften der Mittel einzustellen.

Vorzugsweise werden die Färbemittel als flüssige Zubereitung bereitgestellt und den Mitteln daher gegebenenfalls zusätzlich eine weitere oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, zwitterionischen, amphoteren und nichtionischen Tensiden und Emulgatoren ausgewählt.

Die erfindungsgemäßen Färbemittel können weitere Hilfs- und Zusatzstoffe enthalten. So hat es sich als vorteilhaft erwiesen, wenn die Mittel mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind
- anionische, synthetische Polymere;
- kationische, synthetische Polymere;
- natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose,

Amylopektin und Dextrine, sowie Cellulosederivate, wie beispielsweise Methylcellulose, Carboxyalkylcellulosen und Hydroxyalkylcellulosen;
- nichtionische, vollsynthetische Polymere, wie Polyvinylalkohol oder Polyvinylpyrrolidinon; sowie
- anorganische Verdickungsmittel, insbesondere Schichtsilikate wie beispielsweise Bentonit, besonders Smektite, wie Montmorillonit oder Hectorit.

Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des anwendungsbereiten Mittels zwischen 7 und 11, insbesondere zwischen 8 und 10,5, liegt. Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)-Alkalimetallmetasilikaten, (Erd-)Alkalimetallphosphaten und (Erd-)Alkalimetallhydrogenphosphaten, ausgewählt werden. Bevorzugte anorganische Alkalisierungsmittel sind Magnesiumcarbonat, Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein organisches Alkalisierungsmittel enthalten. Eine Ausführungsform des ersten Erfindungsgegenstands ist dadurch gekennzeichnet, dass das Mittel zusätzlich mindestens ein Alkalisierungsmittel enthält, welches ausgewählt ist aus der Gruppe, die gebildet wird aus Ammoniak, Alkanolaminen und basischen Aminosäuren, insbesondere aus Ammoniak, Monoethanolamin und Arginin oder seinen verträglichen Salzen.

Weiterhin hat es sich als vorteilhaft erweisen, wenn die Färbemittel, insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und-distearat sowie PEG-3-distearat; Pigmente sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

Die Mittel des ersten Erfindungsgegenstands können in Verfahren zum Färben und auch in Verfahren zum gleichzeitigen Blondieren bzw. Aufhellen und Färben menschlicher Haare genutzt werden.

Die erfindungsgemäßen Mittel können als Einkomponentenmittel oder als Mehrkomponentenmittel wie Zweikomponentenmittel oder Dreikomponentenmittel formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt.

Unter dem erfindungsgemäßen Mittel zur Farbveränderung von keratinischen Fasern wird immer das anwendungsbereite Mittel verstanden.

Wird das erfindungsgemäße Mittel dem Anwender in Form eines Einkomponentenmittels zur Verfügung gestellt, dann muss das anwendungsbereite Mittel nicht erst hergestellt werden, sondern kann direkt aus dem Behältnis, in dem es konfektioniert wurde, entnommen und auf die keratinischen Fasern appliziert werden.

Bei Blondiermitteln handelt es sich jedoch üblicherweise um Zweikomponentenprodukte, bei welchen eine oxidationsmittelhaltige Komponente (A1) kurz vor der Anwendung mit einem (Alkalisierungs-)Mittel (A2) vermischt und diese anwendungsbereite Mischung auf die Haare aufgetragen wird.

In diesem Fall handelt es sich bei dem erfindungsgemäßen Mittel um das anwendungsbereite Mittel, welches kurz vor der Anwendung durch Vermischen von (A1) und (A2) hergestellt wurde.

Hierbei können die direktziehenden Farbstoffe der allgemeinen Formel (I) in der Komponente (A1) (d.h. zusammen mit dem Oxidationsmittel) oder aber in der Komponente (A2) (zusammen mit dem Alkalisierungsmittel) konfektioniert werden.

Es ist ebenfalls möglich und erfindungsgemäß, wenn das anwendungsbereite Mittel kurz vor der Anwendung auf den menschlichen Haaren durch Vermischen von 3 Komponenten hergestellt wird, wobei
- die Komponente (A1) mindestens einen direktziehenden Farbstoff der allgemeinen Formel (I) und mindestens ein Alkalisierungsmittel
- die Komponente (A2) mindestens ein erstes Oxidationsmittel (z.B. Wasserstoffperoxid) und
- die Komponente (A3) mindestens ein zweites Oxidationsmittel (z.B. ein oder mehrere Poroxodisulfatsalze) enthält.

Während der Einwirkzeit der Mittel auf der Faser kann es vorteilhaft sein, den Aufhellvorgang oder Mattiervorgang durch Wärmezufuhr zu unterstützen. Die Wärmezufuhr kann durch eine externe Wärmequelle, wie z.B. warme Luft eines Warmluftgebläses, als auch, insbesondere bei einer Haaraufhellung am lebenden Probanden, durch die Körpertemperatur des Probanden erfolgen. Bei letzterer Möglichkeit wird üblicherweise die behandelte Partie mit einer Haube abgedeckt. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Nach Ende der Einwirkzeit wird die verbleibende Färbezubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Wasser erfolgen kann, wenn das Aufhellmittel einen stark tensidhaltigen Träger besitzt.

Bezüglich weiterer bevorzugter Ausführungsformen der erfindungsgemäßen Verwendung und Verfahren gilt mutatis mutandis das zu den erfindungsgemäßen Mitteln Gesagte.

### Beispiele

### Direktzieher 1: 3-Methyl-2-{2-[4-(4-{4-[2-(3-methyl-1,3-thiazol-3-ium-2-yl)diazen-1-yl]phenyl}piperazin-1-yl)phenyl]diazen-1-yl}-1,3-thiazol-3-ium Dimethylsulfat

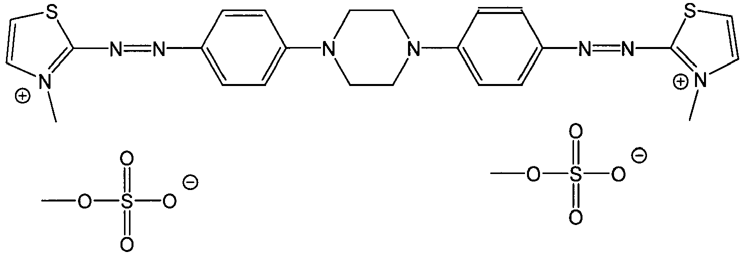

Der Farbstoff DZ 1 wurde nach einem Verfahren synthetisiert, wie es in dem GB 1186753 beschrieben ist. Als Quaternierungsmittel wurde Dimethylsulfat verwendet.

### Färbebeispiele

### Formulierungen

Es wurden die folgenden Färbecremes hergestellt (alle Angaben in Gew.-%, Aktivsubstanz)

| | **Bsp 1** | **Bsp 2** |
|---|---|---|
| Cetearyl Alcohol (C16/C18-Fettalkohol) | 1,0 | 1,0 |
| Coconut Alcohol (C12/C18-Fettalkohol) | 1,0 | 1,0 |
| Methylparaben | 0,1 | 0,1 |
| Propylparaben | 0,1 | 0,1 |
| Ceteareth-12 | 3,0 | --- |
| Ceteareth-20 | 3,0 | --- |
| Natrium Laureth-5 Carboxylat | --- | 1,0 |
| DZ 1 (erfindungsgemäß) | 1,0 | 1,0 |
| Ammoniumsulfat | 1,0 | 1,0 |
| Wasser | ad 100 | ad 100 |

Die Fettalkohole und Konservierungsmittel wurden zusammen mit den Tensiden aufgeschmolzen. Diese Schmelze wurde mit heißem Wasser emulgiert, anschließend wurde der in wenig heißem Wasser vorgelöste Farbstoff hinzu gegeben. Dann wurde die Ammoniumsulfatlösung hinzugefügt. Der angegebene pH-Wert wurde mit Ammoniak (sowie ggf. Zitronensäure) eingestellt, danach wurde mit Wasser auf 100 g aufgefüllt.

### Ausfärbungen

Jeweils 1,8 g der Färbecreme wurden auf eine ca. 6 cm lange Strähne Menschenhaares (Kerling Euronaturhaar, 80% ergraut) aufgetragen und dort 30 Minuten bei 30 °C belassen. Nach Beendigung der Einwirkzeit wurde das Haar ausgespült, mit einem üblichen Haarwaschmittel gewaschen und anschließend getrocknet. Nach dem Trocknen wurden die Färbung und die Farbintensität der Strähnen visuell unter der Tageslichtlampe beurteilt.

| | Formulierung | pH-Wert | Farbnuance | Farbintensität |
|---|---|---|---|---|
| Bsp 1 | DZ 1 mit Ceteareth-12 und Ceteareth-20 | 9,5 | graublau | +++ |
| Bsp 2 | DZ 1 mit Natrium Laureth-5 Carboxylat | 9,5 | dunkelblau | ++++ |

| | | | | |
|---|---|---|---|---|
| Farbintensität: + = schlecht +++ = mittel +++++ = sehr gut | | | | |

## Patentansprüche

1. Mittel zur Farbveränderung von menschlichen Haaren, enthaltend in einem kosmetischen Träger
(a) mindestens einen direktziehenden Farbstoff der Formel (I), 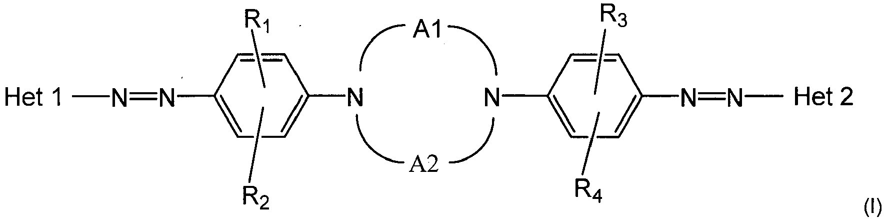 wobei
Het1, Het2 unabhängig voneinander für eine der Strukturen (II), (III), (IV), (V), (VI) oder (VII) stehen, 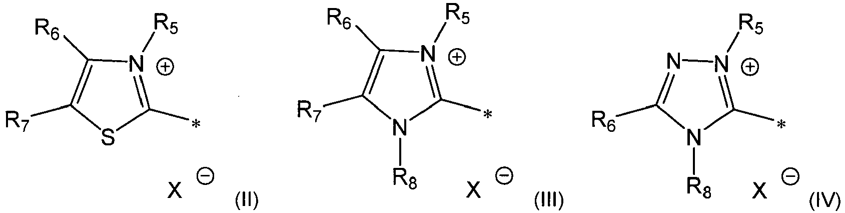 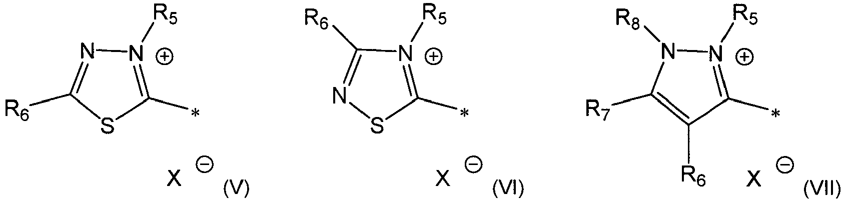
R1, R3 unabhängig voneinander für ein Wasserstoffatom, eine C₁-C₆-Alkylgruppe, ein Halogenatom aus der Gruppe Chlor, Brom, Fluor oder Jod, für eine C₁-C₆-Alkoxygruppe oder für eine Nitrogruppe stehen,
R2, R4, unabhängig voneinander für ein Wasserstoffatom, eine C₁-C₆-Alkylgruppe, ein Halogenatom aus der Gruppe Chlor, Brom, Fluor oder Jod, für eine C₁-C₆-Alkoxygruppe oder für eine Nitrogruppe stehen,
R5, R8 jeweils unabhängig voneinander für eine C₁-C₆-Alkylgruppe oder für eine C₂-C₆-Alkenylgruppe stehen,
R6, R7 jeweils unabhängig voneinander für ein Wasserstoffatom, eine C₁-C₆-Alkylgruppe, ein Halogenatom aus der Gruppe Chlor, Brom, Fluor oder Jod, für eine C₁-C₆-Alkoxygruppe oder für eine Nitrogruppe stehen,
A1, A2 unabhängig voneinander für eine Gruppierung der Formeln (VIII) oder (IX) stehen,
*-(CH₂)n-* _{(VIII)}
*-(CH₂)m-O-(CH₂)p* _{(IX)}
n für eine ganze Zahl von 2 bis 6 steht,
m, p jeweils unabhängig voneinander für die Zahlen 2 oder 3 stehen,
X- für ein physiologisch verträgliches Anion, bevorzugt aus der Gruppe Chlorid, Bromid, lodid, Methylsulfat, Methylsulfonat, p-Toluolsulfonat, Acetat, Hydrogen-sulfat, ½ Sulfat oder ½ Tetrachlorozinkat, steht, und
(b) mindestens ein Tensid.

2. Mittel nach Anspruch 1, **dadurch gekennzeichnet, dass** es (a) mindestens einen direktziehenden Farbstoff der allgemeinen Formel (I) enthält, in der
Het1, Het2 unabhängig voneinander für eine der Strukturen (II), (III) oder (IV) stehen

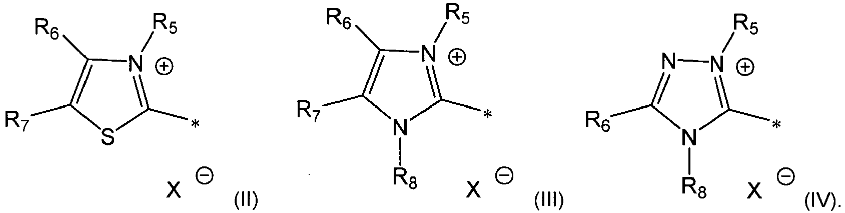

3. Mittel nach einem der Ansprüche 1 bis 2, **dadurch gekennzeichnet, dass** es (a) mindestens einen direktziehenden Farbstoff der allgemeinen Formel (I) enthält, in der
R1, R3 unabhängig voneinander für ein Wasserstoffatom, eine C₁-C₆-Alkylgruppe oder für eine C₁-C₆-Alkoxygruppe stehen.

4. Mittel nach einem der Ansprüche 1 bis 3, **dadurch gekennzeichnet, dass** es (a) mindestens einen direktziehenden Farbstoff der allgemeinen Formel (I) enthält, in der
R2, R4 unabhängig voneinander für ein Wasserstoffatom, eine C₁-C₆-Alkylgruppe oder für eine C₁-C₆-Alkoxygruppe, besonders bevorzugt für ein Wasserstoffatom, stehen.

5. Mittel nach einem der Ansprüche 1 bis 4, **dadurch gekennzeichnet, dass** es (a) mindestens einen direktziehenden Farbstoff der allgemeinen Formel (I) enthält, in der
R5, R8 jeweils unabhängig voneinander für eine C₁-C₆-Alkylgruppe stehen, und
R6, R7 jeweils unabhängig voneinander für ein Wasserstoffatom, eine C₁-C₆-Alkylgruppe, oder für eine C₁-C₆-Alkoxygruppe stehen.

6. Mittel nach einem der Ansprüche 1 bis 5, **dadurch gekennzeichnet, dass** es (a) mindestens einen direktziehenden Farbstoff der allgemeinen Formel (I) enthält, in der
A1, A2 beide für eine Gruppierung der Formel (VIII) stehen,
*-(CH₂)n-* (VIII),
wobei
n für die Zahlen 2 oder 3, bevorzugt für die Zahl 2, steht.

7. Mittel nach einem der Ansprüche 1 bis 6, **dadurch gekennzeichnet, dass** es (a) mindestens einen direktziehenden Farbstoff der allgemeinen Formel (I) enthält, der ausgewählt ist aus
- Salzen des 3-Methyl-2-{2-[4-(4-{4-[2-(3-methyl-1,3-thiazol-3-ium-2-yl)diazen-1-yl]phenyl}piperazin-1-yl)phenyl]diazen-1-yl}-1,3-thiazol-3-iums,
- Salzen des 3-Ethyl-2-{2-[4-(4-{4-[2-(3-ethyl-1,3-thiazol-3-ium-2-yl)diazen-1-yl]phenyl}piperazin-1-yl)phenyl]diazen-1-yl}-1,3-thiazol-3-iums,
- Salzen des 3-Methyl-2-{2-[3-methyl-4-(4-{2-methyl-4-[2-(3-methyl-1,3-thiazol-3-ium-2-yl)diazen-1-yl]phenyl}piperazin-1-yl)phenyl]diazen-1-yl}-1,3-thiazol-3-iums,
- Salzen des 3-Ethyl-2-{2-[4-(4-{4-[2-(3-ethyl-1,3-thiazol-3-ium-2-yl)diazen-1-yl]-2-methylphenyl}piperazin-1-yl)-3-methylphenyl]diazen-1-yl}-1,3-thiazol-3-iums,
- Salzen des 3-Methyl-2-{2-[2-methyl-4-(4-{3-methyl-4-[2-(3-methyl-1,3-thiazol-3-ium-2-yl)diazen-1-yl]phenyl}piperazin-1-yl)phenyl]diazen-1-yl}-1,3-thiazol-3-iums,
- Salzen des 3-Ethyl-2-{2-[4-(4-{4-[2-(3-ethyl-1,3-thiazol-3-ium-2-yl)diazen-1-yl]-3-methylphenyl}piperazin-1-yl)-2-methylphenyl]diazen-1-yl}-1,3-thiazol-3-iums,
- Salzen des 2-{2-[3-Methoxy-4-(4-{2-methoxy-4-[2-(3-methyl-1,3-thiazol-3-ium-2-yl)diazen-1-yl]phenyl}piperazin-1-yl)phenyl]diazen-1-yl}-3-methyl-1,3-thiazol-3-iums,
- Salzen des 3-Ethyl-2-{2-[4-(4-{4-[2-(3-ethyl-1,3-thiazol-3-ium-2-yl)diazen-1-yl]-2-methoxyphenyl}piperazin-1-yl)-3-methoxyphenyl]diazen-1-yl}-1,3-thiazol-3-iums,
- Salzen des 2-{2-[2-Methoxy-4-(4-{3-methoxy-4-[2-(3-methyl-1,3-thiazol-3-ium-2-yl)diazen-1-yl]phenyl}piperazin-1-yl)phenyl]diazen-1-yl}-3-methyl-1,3-thiazol-3-iums,
- Salzen des 3-Ethyl-2-{2-[4-(4-{4-[2-(3-ethyl-1,3-thiazol-3-ium-2-yl)diazen-1-yl]-3-methoxyphenyl}piperazin-1-yl)-2-methoxyphenyl]diazen-1-yl}-1,3-thiazol-3-iums,
- Salzen des 2-{2-[4-(4-{2,5-Dimethyl-4-[2-(3-methyl-1,3-thiazol-3-ium-2-yl)diazen-1-yl]phenyl}piperazin-1-yl)-2,5-dimethylphenyl]diazen-1-yl}-3-methyl-1,3-thiazol-3-iums,
- Salzen des 3-Ethyl-2-{2-[4-(4-{4-[2-(3-ethyl-1,3-thiazol-3-ium-2-yl)diazen-1-yl]-2,5-dimethylphenyl}piperazin-1-yl)-2,5-dimethylphenyl]diazen-1-yl}-1,3-thiazol-3-iums,
- Salzen des 2-{2-[4-(4-{4-[2-(1,3-Dimethyl-1H-imidazol-3-ium-2-yl)diazen-1-yl]phenyl}piperazin-1-yl)phenyl]diazen-1-yl}-1,3-dimethyl-1H-imidazol-3-iums,
- Salzen des 2-{2-[4-(4-{4-[2-(1,3-Diethyl-1H-imidazol-3-ium-2-yl)diazen-1-yl]phenyl}piperazin-1-yl)phenyl]diazen-1-yl}-1,3-diethyl-1H-imidazol-3-iums,
- Salzen des 2-{2-[4-(4-{4-[2-(1,3-Dimethyl-1H-imidazol-3-ium-2-yl)diazen-1-yl]-2-methylphenyl}piperazin-1-yl)-3-methylphenyl]diazen-1-yl}-1,3-dimethyl-1H-imidazol-3-iums,
- Salzen des 2-{2-[4-(4-{4-[2-(1,3-Diethyl-1H-imidazol-3-ium-2-yl)diazen-1-yl]-2-methylphenyl}piperazin-1-yl)-3-methylphenyl]diazen-1-yl}-1,3-diethyl-1H-imidazol-3-iums,
- Salzen des 2-{2-[4-(4-{4-[2-(1,3-Dimethyl-1H-imidazol-3-ium-2-yl)diazen-1-yl]-3-methylphenyl}piperazin-1-yl)-2-methylphenyl]diazen-1-yl}-1,3-dimethyl-1H-imidazol-3-iums,
- Salzen des 2-{2-[4-(4-{4-[2-(1,3-Diethyl-1H-imidazol-3-ium-2-yl)diazen-1-yl]-3-methylphenyl}piperazin-1-yl)-2-methylphenyl]diazen-1-yl}-1,3-diethyl-1H-imidazol-3-iums,
- Salzen des 2-{2-[4-(4-{4-[2-(1,3-Dimethyl-1H-imidazol-3-ium-2-yl)diazen-1-yl]-2-methoxyphenyl}piperazin-1-yl)-3-methoxyphenyl]diazen-1-yl}-1,3-dimethyl-1H-imidazol-3-iums,
- Salzen des 2-{2-[4-(4-{4-[2-(1,3-Diethyl-1H-imidazol-3-ium-2-yl)diazen-1-yl]-2-methoxyphenyl}piperazin-1-yl)-3-methoxyphenyl]diazen-1-yl}-1,3-diethyl-1H-imidazol-3-iums,
- Salzen des 2-{2-[4-(4-{4-[2-(1,3-Dimethyl-1H-imidazol-3-ium-2-yl)diazen-1-yl]-3-methoxyphenyl}piperazin-1-yl)-2-methoxyphenyl]diazen-1-yl}-1,3-dimethyl-1H-imidazol-3-iums,
- Salzen des 2-{2-[4-(4-{4-[2-(1,3-Diethyl-1H-imidazol-3-ium-2-yl)diazen-1-yl]-3-methoxyphenyl}piperazin-1-yl)-2-methoxyphenyl]diazen-1-yl}-1,3-diethyl-1H-imidazol-3-iums,
- Salzen des 2-{2-[4-(4-{4-[2-(1,3-Dimethyl-1H-imidazol-3-ium-2-yl)diazen-1-yl]-2,5-dimethylphenyl}piperazin-1-yl)-2,5-dimethylphenyl]diazen-1-yl}-1,3-dimethyl-1H-imidazol-3-iums,
- Salzen des 2-{2-[4-(4-{4-[2-(1,3-Diethyl-1H-imidazol-3-ium-2-yl)diazen-1-yl]-2,5-dimethylphenyl}piperazin-1-yl)-2,5-dimethylphenyl]diazen-1-yl}-1,3-diethyl-1H-imidazol-3-iums,
- Salzen des 5-{2-[4-(4-{4-[2-(1,4-Dimethyl-1H-1,2,4-triazol-4-ium-5-yl)diazen-1-yl]phenyl)piperazin-1-yl)phenyl]diazen-1-yl}-1,4-dimethyl-1H-1,2,4-triazol-4-iums,
- Salzen des 5-{2-[4-(4-{4-[2-(1,4-Diethyl-1H-1,2,4-triazol-4-ium-5-yl)diazen-1-yl]phenyl}piperazin-1-yl)phenyl]diazen-1-yl}-1,4-diethyl-1H-1,2,4-triazol-4-iums,
- Salzen des 5-{2-[4-(4-{4-[2-(1,4-Dimethyl-1H-1,2,4-triazol-4-ium-5-yl)diazen-1-yl]-2-methylphenyl}piperazin-1-yl)-3-methylphenyl]diazen-1-yl}-1,4-dimethyl-1H-1,2,4-triazol-4-iums,
- Salzen des 5-{2-[4-(4-{4-[2-(1,4-Diethyl-1H-1,2,4-triazol-4-ium-5-yl)diazen-1-yl]-2-methylphenyl}piperazin-1-yl)-3-methylphenyl]diazen-1-yl}-1,4-diethyl-1H-1,2,4-triazol-4-iums,
- Salzen des 5-{2-[4-(4-{4-[2-(1,4-Dimethyl-1H-1,2,4-triazol-4-ium-5-yl)diazen-1-yl]-3-methylphenyl}piperazin-1-yl)-2-methylphenyl]diazen-1-yl}-1,4-dimethyl-1H-1,2,4-triazol-4-iums,
- Salzen des 5-{2-[4-(4-{4-[2-(1,4-Diethyl-1H-1,2,4-triazol-4-ium-5-yl)diazen-1-yl]-3-methylphenyl}piperazin-1-yl)-2-methylphenyl]diazen-1-yl}-1,4-diethyl-1H-1,2,4-triazol-4-iums,
- Salzen des 5-{2-[4-(4-{4-[2-(1,4-Dimethyl-1H-1,2,4-triazol-4-ium-5-yl)diazen-1-yl]-2-methoxyphenyl}piperazin-1-yl)-3-methoxyphenyl]diazen-1-yl}-1,4-dimethyl-1H-1,2,4-triazol-4-iums,
- Salzen des 5-{2-[4-(4-{4-[2-(1,4-diethyl-1H-1,2,4-triazol-4-ium-5-yl)diazen-1-yl]-2-methoxyphenyl}piperazin-1-yl)-3-methoxyphenyl]diazen-1-yl}-1,4-diethyl-1H-1,2,4-triazol-4-iums,
- Salzen des 5-{2-[4-(4-{4-[2-(1,4-Dimethyl-1H-1,2,4-triazol-4-ium-5-yl)diazen-1-yl]-3-methoxyphenyl}piperazin-1-yl)-2-methoxyphenyl]diazen-1-yl}-1,4-dimethyl-1H-1,2,4-triazol-4-iums,
- Salzen des 5-{2-[4-(4-{4-[2-(1,4-Diethyl-1H-1,2,4-triazol-4-ium-5-yl)diazen-1-yl]-3-methoxyphenyl}piperazin-1-yl)-2-methoxyphenyl]diazen-1-yl}-1,4-diethyl-1H-1,2,4-triazol-4-iums,
- Salzen des 5-{2-[4-(4-{4-[2-(1,4-Dimethyl-1H-1,2,4-triazol-4-ium-5-yl)diazen-1-yl]-2,5-dimethylphenyl}piperazin-1-yl)-2,5-dimethylphenyl]diazen-1-yl}-1,4-dimethyl-1H-1,2,4-triazol-4-iums,
- Salzen des 5-{2-[4-(4-{4-[2-(1,4-Diethyl-1H-1,2,4-triazol-4-ium-5-yl)diazen-1-yl]-2,5-dimethylphenyl}piperazin-1-yl)-2,5-dimethylphenyl]diazen-1 -yl}-1,4-diethyl-1H-1,2,4-triazol-4-iums,
- Salzen des 3-Methyl-2-{2-[4-(5-{4-[2-(3-methyl-1,3-thiazol-3-ium-2-yl)diazen-1-yl]phenyl}-1,5-diazocan-1-yl)phenyl]diazen-1-yl}-1,3-thiazol-3-iums,
- Salzen des 3-Ethyl-2-{2-[4-(5-{4-[2-(3-ethyl-1,3-thiazol-3-ium-2-yl)diazen-1-yl]phenyl}-1,5-diazocan-1-yl)phenyl]diazen-1-yl}-1,3-thiazol-3-iums,
- Salzen des 3-Methyl-2-{2-[3-methyl-4-(5-{2-methyl-4-[2-(3-methyl-1,3-thiazol-3-ium-2-yl)diazen-1-yl]phenyl}-1,5-diazocan-1-yl)phenyl]diazen-1-yl}-1,3-thiazol-3-iums,
- Salzen des 3-Ethyl-2-{2-[4-(5-{4-[2-(3-ethyl-1,3-thiazol-3-ium-2-yl)diazen-1-yl]-2-methylphenyl}-1,5-diazocan-1-yl)-3-methylphenyl]diazen-1-yl}-1,3-thiazol-3-iums,
- Salzen des 3-Methyl-2-{2-[2-methyl-4-(5-{3-methyl-4-[2-(3-methyl-1,3-thiazol-3-ium-2-yl)diazen-1-yl]phenyl}-1,5-diazocan-1-yl)phenyl]diazen-1-yl}-1,3-thiazol-3-iums,
- Salzen des 3-Ethyl-2-{2-[4-(5-{4-[2-(3-ethyl-1,3-thiazol-3-ium-2-yl)diazen-1-yl]-3-methylphenyl}-1,5-diazocan-1-yl)-2-methylphenyl]diazen-1-yl}-1,3-thiazol-3-iums,
- Salzen des 2-{2-[3-Methoxy-4-(5-{2-methoxy-4-[2-(3-methyl-1,3-thiazol-3-ium-2-yl)diazen-1-yl]phenyl}-1,5-diazocan-1-yl)phenyl]diazen-1-yl}-3-methyl-1,3-thiazol-3-iums,
- Salzen des 3-Ethyl-2-{2-[4-(5-{4-[2-(3-ethyl-1,3-thiazol-3-ium-2-yl)diazen-1-yl]-2-methoxyphenyl}-1,5-diazocan-1-yl)-3-methoxyphenyl]diazen-1-yl}-1,3-thiazol-3-iums,
- Salzen des 2-{2-[2-Methoxy-4-(5-{3-methoxy-4-[2-(3-methyl-1,3-thiazol-3-ium-2-yl)diazen-1-yl]phenyl}-1,5-diazocan-1-yl)phenyl]diazen-1-yl}-3-methyl-1,3-thiazol-3-iums,
- Salzen des 3-Ethyl-2-{2-[4-(5-{4-[2-(3-ethyl-1,3-thiazol-3-ium-2-yl)diazen-1-yl]-3-methoxyphenyl}-1,5-diazocan-1-yl)-2-methoxyphenyl]diazen-1-yl}-1,3-thiazol-3-iums,
- Salzen des 2-{2-[4-(5-{2,5-Dimethyl-4-[2-(3-methyl-1,3-thiazol-3-ium-2-yl)diazen-1-yl]phenyl}-1,5-diazocan-1-yl)-2,5-dimethylphenyl]diazen-1-yl}-3-methyl-1,3-thiazol-3-iums,
- Salzen des 3-Ethyl-2-{2-[4-(5-{4-[2-(3-ethyl-1,3-thiazol-3-ium-2-yl)diazen-1-yl]-2,5-dimethylphenyl}-1,5-diazocan-1-yl)-2,5-dimethylphenyl]diazen-1-yl}-1,3-thiazol-3-iums,
- Salzen des 2-{2-[4-(5-{4-[2-(1,3-Dimethyl-1H-imidazol-3-ium-2-yl)diazen-1-yl]phenyl}-1,5-diazocan-1-yl)phenyl]diazen-1-yl}-1,3-dimethyl-1H-imidazol-3-iums,
- Salzen des 2-{2-[4-(5-{4-[2-(1,3-Diethyl-1H-imidazol-3-ium-2-yl)diazen-1-yl]phenyl}-1,5-diazocan-1-yl)phenyl]diazen-1-yl}-1,3-diethyl-1H-imidazol-3-iums,
- Salzen des 2-{2-[4-(5-{4-[2-(1,3-Dimethyl-1H-imidazol-3-ium-2-yl)diazen-1-yl]-2-methylphenyl}-1,5-diazocan-1-yl)-3-methylphenyl]diazen-1-yl}-1,3-dimethyl-1H-imidazol-3-iums,
- Salzen des 2-{2-[4-(5-{4-[2-(1,3-Diethyl-1H-imidazol-3-ium-2-yl)diazen-1-yl]-2-methylphenyl}-1,5-diazocan-1-yl)-3-methylphenyl]diazen-1-yl}-1,3-diethyl-1H-imidazol-3-iums,
- Salzen des 2-{2-[4-(5-{4-[2-(1,3-Dimethyl-1-H-imidazol-3-ium-2-yl)diazen-1-yl]-3-methylphenyl}-1,5 diazocan-1-yl)-2-methylphenyl]diazen-1-yl}-1,3-dimethyl-1H-imidazol-3-iums,
- Salzen des 2-{2-[4-(5-{4-[2-(1,3-Diethyl-1H-imidazol-3-ium-2-yl)diazen-1-yl]-3-methylphenyl}-1,5-diazocan-1-yl)-2-methylphenyl]diazen-1-yl}-1,3-diethyl-1H-imidazol-3-iums,
- Salzen des 2-{2-[4-(5-{4-[2-(1,3-Dimethyl-1H-imidazol-3-ium-2-yl)diazen-1-yl]-2-methoxyphenyl}-1,5-diazocan-1-yl)-3-methoxyphenyl]diazen-1-yl}-1,3-dimethyl-1H-imidazol-3-iums,
- Salzen des 2-{2-[4-(5-{4-[2-(1,3-Diethyl-1H-imidazol-3-ium-2-yl)diazen-1-yl]-2-methoxyphenyl}-1,5-diazocan-1-yl)-3-methoxyphenyl]diazen-1-yl}-1,3-diethyl-1H-imidazol-3-iums,
- Salzen des 2-{2-[4-(5-{4-[2-(1,3-Dimethyl-1H-imidazol-3-ium-2-yl)diazen-1-yl]-3-methoxyphenyl}-1,5-diazocan-1-yl)-2-methoxyphenyl]diazen-1-yl}-1,3-dimethyl-1H-imidazol-3-iums,
- Salzen des 2-{2-[4-(5-{4-[2-(1,3-Diethyl-1H-imidazol-3-ium-2-yl)diazen-1-yl]-3-methoxyphenyl}-1,5-diazocan-1-yl)-2-methoxyphenyl]diazen-1-yl}-1,3-diethyl-1H-imidazol-3-iums,
- Salzen des 2-{2-[4-(5-{4-[2-(1,3-Dimethyl-1H-imidazol-3-ium-2-yl)diazen-1-yl]-2,5-dimethylphenyl}-1,5-diazocan-1-yl)-2,5-dimethylphenyl]diazen-1-yl}-1,3-dimethyl-1H-imidazol-3-iums,
- Salzen des 2-{2-[4-(5-{4-[2-(1,3-Diethyl-1H-imidazol-3-ium-2-yl)diazen-1-yl]-2,5-dimethylphenyl}-1,5-diazocan-1-yl)-2,5-dimethylphenyl]diazen-1-yl}-1,3-diethyl-1H-imidazol-3-iums,
- Salzen des 5-{2-[4-(5-{4-[2-(1,4-Dimethyl-1H-1,2,4-triazol-4-ium-5-yl)diazen-1-yl]phenyl}-1,5-diazocan-1-yl)phenyl]diazen-1-yl}-1,4-dimethyl-1H-1,2,4-triazol-4-iums,
- Salzen des 5-{2-[4-(5-{4-[2-(1,4-Diethyl-1H-1,2,4-triazol-4-ium-5-yl)diazen-1-yl]phenyl}-1,5-diazocan-1-yl)phenyl]diazen-1-yl}-1,4-diethyl-1H-1,2,4-triazol-4-iums,
- Salzen des 5-{2-[4-(5-{4-[2-(1,4-Dimethyl-1H-1,2,4-triazol-4-ium-5-yl)diazen-1-yl]-2-methylphenyl}-1,5-diazocan-1-yl)-3-methylphenyl]diazen-1-yl}-1,4-dimethyl-1H-1,2,4-triazol-4-iums,
- Salzen des 5-{2-[4-(5-{4-[2-(1,4-diethyl-1H-1,2,4-triazol-4-ium-5-yl)diazen-1-yl]-2-methylphenyl}-1,5-diazocan-1-yl)-3-methylphenyl]diazen-1-yl}-1,4-diethyl-1H-1,2,4-triazol-4-iums,
- Salzen des 5-{2-[4-(5-{4-[2-(1,4-dimethyl-1H-1,2,4-triazol-4-ium-5-yl)diazen-1-yl]-3-methylphenyl}-1,5-diazocan-1-yl)-2-methylphenyl]diazen-1-yl}-1,4-dimethyl-1H-1,2,4-triazol-4-iums,
- Salzen des 5-{2-[4-(5-{4-[2-(1,4-diethyl-1H-1,2,4-triazol-4-ium-5-yl)diazen-1-yl]-3-methylphenyl}-1,5-diazocan-1-yl)-2-methylphenyl]diazen-1-yl}-1,4-diethyl-1H-1,2,4-triazol-4-iums,
- Salzen des 5-{2-[4-(5-{4-[2-(1,4-Dimethyl-1H-1,2,4-triazol-4-ium-5-yl)diazen-1-yl]-2-methoxyphenyl}-1,5-diazocan-1-yl)-3-methoxyphenyl]diazen-1-yl}-1,4-dimethyl-1H-1,2,4-triazol-4-iums,
- Salzen des 5-{2-[4-(5-{4-[2-(1,4-Diethyl-1H-1,2,4-triazol-4-ium-5-yl)diazen-1-yl]-2-methoxyphenyl}-1,5-diazocan-1-yl)-3-methoxyphenyl]diazen-1-yl}-1,4-diethyl-1H-1,2,4-triazol-4-iums,
- Salzen des 5-{2-[4-(5-{4-[2-(1,4-dimethyl-1H-1,2,4-triazol-4-ium-5-yl)diazen-1-yl]-3-methoxyphenyl}-1,5-diazocan-1-yl)-2-methoxyphenyl]diazen-1-yl}-1,4-dimethyl-1H-1,2,4-triazol-4-iums,
- Salzen des 5-{2-[4-(5-{4-[2-(1,4-diethyl-1H-1,2,4-triazol-4-ium-5-yl)diazen-1-yl]-3-methoxyphenyl}-1,5-diazocan-1-yl)-2-methoxyphenyl]diazen-1-yl}-1,4-diethyl-1H-1,2,4-triazol-4-iums,
- Salzen des 5-{2-[4-{5-{4-[2-(1,4-Dimethyl-1H-1,2,4-triazol-4-ium-5-yl)diazen-1-yl]-2,5-dimethylphenyl}-1,5-diazocan-1-yl)-2,5-dimethylphenyl]diazen-1-yl}-1,4-dimethyl-1H-1,2,4-triazol-4-iums und/oder
- Salzen des 5-{2-[4-(5-{4-[2-(1,4-diethyl-1H-1,2,4-triazol-4-ium-5-yl)diazen-1-yl]-2,5-dimethylphenyl}-1,5-diazocan-1-yl)-2,5-dimethylphenyl]diazen-1-yl}-1,4-diethyl-1H-1,2,4-triazol-4-iums.

8. Mittel nach einem der Ansprüche 1 bis 7, **dadurch gekennzeichnet, dass** es - bezogen auf das Gesamtgewicht des Mittels - einen oder mehrere direktziehende Farbstoffe (a) der Formel (I) in einer Gesamtmenge von 0,01 bis 4,5 Gew.-%, bevorzugt von 0,05 bis 2,8 Gew.-%, weiter bevorzugt von 0,1 bis 2,2 Gew.-% und besonders bevorzugt von 0,2 bis 1,2 Gew.-% enthält.

9. Mittel nach einem der Ansprüche 1 bis 8, **dadurch gekennzeichnet, dass** es (b) mindestens ein anionisches Tensid enthält, das ausgewählt ist aus den
- linearen und verzweigten Fettsäuren mit 8 bis 30 C-Atomen,
- Ethercarbonsäuren der Formel R-O-(CH₂-CH₂O)ₓ-CH₂-COOM, in der
R für eine lineare Alkylgruppe mit 8 bis 30 C-Atomen steht,
x für eine ganze Zahl von 0 bis 16 steht,
M für Wasserstoff oder ein Metall wie ein Alkalimetall, insbesondere Natrium, Kalium, Lithium, ein Erdalkalimetall, insbesondere ½ Magnesium, ½ Calcium, ½ Zink, oder ein Ammoniumion (NH4⁺) steht,
- Acylsarcosiden mit 8 bis 24 C-Atomen in der Acylgruppe,
- Acyltauriden mit 8 bis 24 C-Atomen in der Acylgruppe,
- Acylisethionaten mit 8 bis 24 C-Atomen in der Acylgruppe, die durch Veresterung von C₈-C₂₄-Fettsäuren mit dem Natriumsalz der 2-Hydroxyethan-sulfonsäure (Isethionsäure) zugänglich sind,
- Amidethercarbonsäuren, R¹-CO-NR²-CH₂CH₂-O-(CH₂CH₂O)_{y}CH₂COOM, wobei
R¹ für eine C2-C30-Alkylgruppe steht,
y für eine ganze Zahl von 1 bis 20 steht,
R² für Wasserstoff, einen Methyl-, Ethyl-, Propyl-, Isopropyl-, n-Butyl-, t-Butyl- oder iso-Butylrest steht, und
M für Wasserstoff oder ein Metall wie ein Alkalimetall, insbesondere Natrium, Kalium, Lithium, ein Erdalkalimetall, insbesondere ½ Magnesium, ½ Calcium, ½ Zink, oder ein Ammoniumion (NH4⁺) steht.

10. Mittel nach einem der Ansprüche 1 bis 9, **dadurch gekennzeichnet, dass** mindestens ein anionisches Tensid (b) aus der Gruppe der Ethercarbonsäuren der Formel (B1) enthält
R-O-(CH2-CH2O)x-CH2-COOM (B1),
wobei
R für eine lineare Alkylgruppe mit 8 bis 30 C-Atomen steht,
x für eine ganze Zahl von 0 bis 16 steht,
M für Wasserstoff oder ein Metall wie ein Alkalimetall, insbesondere Natrium, Kalium, Lithium, ein Erdalkalimetall, insbesondere ½ Magnesium, ½ Calcium, ½ Zink, oder ein Ammoniumion (NH4⁺) steht.

11. Mittel nach einem der Ansprüche 1 bis 10, **dadurch gekennzeichnet, dass** es - bezogen auf das Gesamtgewicht des Mittels - ein oder mehrere anionische Tenside (b) in einer Gesamtmenge von 0,05 bis 9,5 Gew.-%, bevorzugt von 0,1 bis 3,1 Gew.-%, weiter bevorzugt von 0,15 bis 2,5 Gew.-% und ganz besonders bevorzugt von 0,2 bis 0,9 Gew.-% enthält.

12. Mittel nach einem der Ansprüche 1 bis 11, **dadurch gekennzeichnet, dass** das Gewichtsverhältnis aus allen im Mittel enthaltenen anionischen Tensiden der Formel (B1) zur Gesamtmenge der im Mittel enthaltenen anionischen Tenside bei einem Wert von 0,5, bevorzugt von 0,6, weiter bevorzugt von 0,75 und besonders bevorzugt von 0,9 liegt,
wobei es sich bei den anionischen Tensiden der Formel (B1) um folgende Tenside handelt
R-O-(CH2-CH2O)x-CH2-COOM (B1),
mit
R gleich einer linearen Alkylgruppe mit 8 bis 30 C-Atomen,
x gleich einer ganzen Zahl von 0 bis 16,
M gleich Wasserstoff oder einem Metall wie ein Alkalimetall, insbesondere Natrium, Kalium, Lithium, ein Erdalkalimetall, insbesondere ½ Magnesium, ½ Calcium, ½ Zink, oder ein Ammoniumion (NH4⁺).

13. Mittel nach einem der Ansprüche 1 bis 12, **dadurch gekennzeichnet, dass** es - bezogen auf das Gesamtgewicht des Mittels - 0,5 bis 12,5 Gew.-%, bevorzugt 2,5 bis 10 Gew.-% und besonders bevorzugt 4,0 bis 9,0 Gew.-% Wasserstoffperoxid enthält.

14. Mittel nach einem der Ansprüche 1 bis 13, **dadurch gekennzeichnet, dass** es mindestens ein Persulfat aus der Gruppe Ammoniumperoxodisulfat, Kaliumperoxodisulfat und Natriumperoxodisulfat enthält.

15. Mittel nach einem der Ansprüche 1 bis 14, **dadurch gekennzeichnet, dass** es - bezogen auf das Gesamtgewicht des Mittels -
- 0,2 bis 13,5 Gew.-%, bevorzugt 0,5 bis 1,5 Gew.-%, Ammoniumperoxodisulfat
- 0,5 bis 4,5 Gew.-%, bevorzugt 1,5 bis 2,5 Gew.-%, Kaliumperoxodisulfat und
- 0,2 bis 1,8 Gew.-%, bevorzugt 0,4 bis 0,8 Gew.-%, Natriumperoxodisulfat enthält.

## Claims

1. An agent for changing the color of human hair, containing, in a cosmetic carrier, (a) at least one direct dye of formula (I), 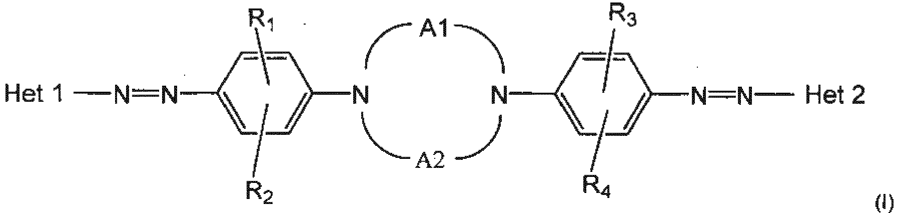 in which
Het1 and Het2 represent, independently of one another, one of structures (II), (III), (IV), (V), (VI) or (VII), 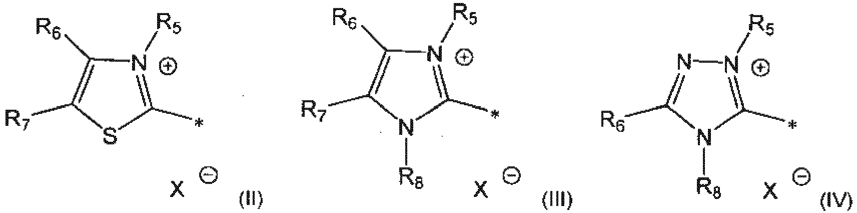 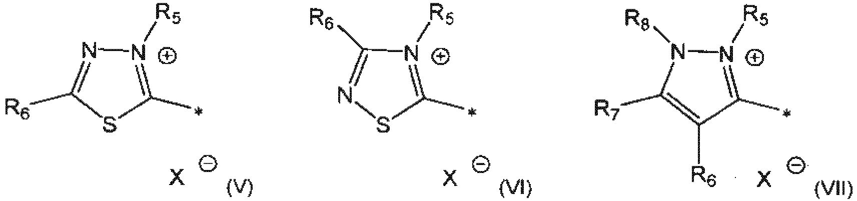
R1 and R3 represent, independently of one another, a hydrogen atom, a C₁-C₆ alkyl group, a halogen atom from the group of chlorine, bromine, fluorine or iodine, a C₁-C₆ alkoxy group, or a nitro group,
R2 and R4 represent, independently of one another, a hydrogen atom, a C₁-C₆ alkyl group, a halogen atom from the group of chlorine, bromine, fluorine or iodine, a C₁-C₆ alkoxy group, or a nitro group,
R5 and R8 each represent, independently of one another, a C₁-C₆ alkyl group or a C2-C6 alkenyl group,
R6 and R7 each represent, independently of one another, a hydrogen atom, a C₁-C₆ alkyl group, a halogen atom from the group of chlorine, bromine, fluorine or iodine, a C₁-C₆ alkoxy group, or a nitro group,
A1 and A2 represent, independently of one another, a group of formulas (VIII) or (IX),
*-(CH₂)n-* (VIII)
*-(CH₂)m-O-(CH₂)p-* (IX)
n represents an integer from 2 to 6,
m and p each represent, independently of one another, the numbers 2 or 3,
X- represents a physiologically acceptable anion, preferably from the group of chloride, bromide, iodide, methyl sulfate, methyl sulfonate, p-toluene sulfonate, acetate, hydrogen sulfate, ½ sulfate or ½ tetrachlorozincate, and
(b) at least one surfactant.

2. The agent according to claim 1, **characterized in that** it contains (a) at least one direct dye of general formula (I), in which
Het1 and Het2represent, independently of one another, one of structures (II), (III) or (IV)

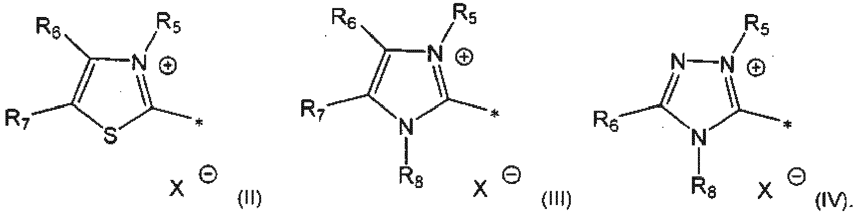

3. The agent according to one of claims 1 to 2, **characterized in that** it contains (a) at least one direct dye of general formula (I), in which
R1 and R3 represent, independently of one another, a hydrogen atom, a C₁-C₆ alkyl group or a C₁-C₆ alkoxy group.

4. The agent according to one of claims 1 to 3, **characterized in that** it contains (a) at least one direct dye of general formula (I), in which
R2 and R4 represent, independently of one another, a hydrogen atom, a C₁-C₆ alkyl group or a C₁-C₆ alkoxy group, particularly preferably a hydrogen atom.

5. The agent according to one of claims 1 to 4, **characterized in that** it contains (a) at least one direct dye of general formula (I), in which
R5 and R8 each represent, independently of one another, a C₁-C₆ alkyl group, and
R6 and R7 each represent, independently of one another, a hydrogen atom, a C₁-C₆ alkyl group or a C₁-C₆ alkoxy group.

6. The agent according to one of claims 1 to 5, **characterized in that** it contains (a) at least one direct dye of general formula (I), in which
A1 and A2 both represent a group of formula (VIII),
*-(CH₂)n-* (VIII).
in which
n represents the numbers 2 or 3, preferably the number 2.

7. The agent according to one of claims 1 to 6, **characterized in that** it contains (a) at least one direct dye of general formula (I), which dye is selected from
- salts of 3-methyl-2-{2-[4-(4-{4-[2-(3-methyl-1,3-thiazol-3-ium-2-yl)diazen-1-yl]phenyl}piperazin-1-yl)phenyl]diazen-1-yl}-1,3-thiazol-3-ium,
- salts of 3-ethyl-2-{2-[4-(4-{4-[2-(3-ethyl-1,3-thiazol-3-ium-2-yl)diazen-1-yl]phenyl}piperazin-1-yl)phenyl]diazen-1-yl}-1,3-thiazol-3-ium,
- salts of 3-methyl-2-{2-[3-methyl-4-(4-{2-methyl-4-[2-(3-methyl-1,3-thiazol-3-ium-2-yl)diazen-1-yl]phenyl}piperazin-1-yl)phenyl]diazen-1-yl}-1,3-thiazol-3-ium,
- salts of 3-ethyl-2-{2-[4-(4-{4-[2-(3-ethyl-1,3-thiazol-3-ium-2-yl)diazen-1-yl]-2-methylphenyl}piperazin-1-yl)-3-methylphenyl]diazen-1-yl}-1,3-thiazol-3-ium,
- salts of 3-methyl-2-{2-[2-methyl-4-(4-{3-methyl-4-[2-(3-methyl-1,3-thiazol-3-ium-2-yl)diazen-f-yl]phenyl}piperazin-1-yl)phenyl]diazen-1-yl}-1,3-thiazol-3-ium,
- salts of 3-ethyl-2-{2-[4-(4-{4-[2-(3-ethyl-1,3-thiazol-3-ium-2-yl)diazen-1-yl]-3-methylphenyl}piperazin-1-yl)-2-methylphenyl]diazen-1-yl}-1,3-thiazol-3-ium,
- salts of 2-{2-[3-methoxy-4-(4-{2-methoxy-4-[2-(3-methyl-1,3-thiazol-3-ium-2-yl)diazen-1-yl]phenyl}piperazin-1-yl)phenyl]diazen-1-yl}-3-methyl-1,3-thiazol-3-ium,
- salts of 3-ethyl-2-{2-[4-(4-{4-[2-(3-ethyl-1,3-thiazol-3-ium-2-yl)diazen-1-yl]-2-methoxyphenyl}piperazin-1-yl)-3-methoxyphenyl]diazen-1-yl}-1,3-thiazol-3-ium,
- salts of 2-{2-[2-methoxy-4-(4-{3-methoxy-4-[2-(3-methyl-1,3-thiazol-3-ium-2-yl)diazen-1-yl]phenyl}piperazin-1-yl)phenyl]diazen-1-yl}-3-methyl-1,3-thiazol-3-ium,
- salts of 3-ethyl-2-{2-[4-(4-{4-[2-(3-ethyl-1,3-thiazol-3-ium-2-yl)diazen-1-yl]-3-methoxyphenyl}piperazin-1-yl)-2-methoxyphenyl]diazen-1-yl}-1,3-thiazol-3-ium,
- salts of 2-{2-[4-(4-{2,5-dimethyl-4-[2-(3-methyl-1,3-thiazol-3-ium-2-yl)diazen-1-yl]phenyl}piperazin-1-yl)-2,5-dimethylphenyl]diazen-1-yl}-3-methyl-1,3-thiazol-3-ium,
- salts of 3-ethyl-2-{2-[4-(4-{4-[2-(3-ethyl-1,3-thiazol-3-ium-2-yl)diazen-1-yl]-2,5-dimethylphenyl}piperazin-1-yl)-2,5-dimethylphenyl]diazen-1-yl}-1,3-thiazol-3-ium,
- salts of 2-{2-[4-(4-{4-[2-(1,3-dimethyl-1H-imidazol-3-ium-2-yl)diazen-1-yl]phenyl}piperazin-1-yl)phenyl]diazen-1-yl}-1,3-dimethyl-1H-imidazol-3-ium,
- salts of 2-{2-[4-(4-{4-[2-(1,3-diethyl-1H-imidazol-3-ium-2-yl)diazen-1-yl]phenyl}piperazin-1-yl)phenyl]diazen-1-yl}-1,3-diethyl-1H-imidazol-3-ium,
- salts of 2-{2-[4-(4-{4-[2-(1,3-dimethyl-1H-imidazol-3-ium-2-yl)diazen-1-yl]-2-methylphenyl}piperazin-1-yl)-3-methylphenyl]diazen-1-yl}-1,3-dimethyl-1H-imidazol-3-ium,
- salts of 2-{2-[4-(4-{4-[2-(1,3-diethyl-1H-imidazol-3-ium-2-yl)diazen-1-yl]-2-methylphenyl}piperazin-1-yl)-3-methylphenyl]diazen-1-yl}-1,3-diethyl-1H-imidazol-3-ium,
- salts of 2-{2-[4-(4-{4-[2-(1,3-dimethyl-1H-imidazol-3-ium-2-yl)diazen-1-yl]-3-methylphenyl}piperazin-1-yl)-2-methylphenyl]diazen-1-yl}-1,3-dimethyl-1H-imidazol-3-ium,
- salts of 2-{2-[4-(4-{4-[2-(1,3-diethyl-1H-imidazol-3-ium-2-yl)diazen-1-yl]-3-methylphenyl}piperazin-1-yl)-2-methylphenyl]diazen-1-yl}-1,3-diethyl-1H-imidazol-3-ium,
- salts of 2-{2-[4-(4-{4-[2-(1,3-dimethyl-1H-imidazol-3-ium-2-yl)diazen-1-yl]-2-methoxyphenyl}piperazin-1-yl)-3-methoxyphenyl]diazen-1-yl}-1,3-dimethyl-1H-imidazol-3-ium,
- salts of 2-{2-[4-(4-{4-[2-(1,3-diethyl-1H-imidazol-3-ium-2-yl)diazen-1-yl]-2-methoxyphenyl}piperazin-1-yl)-3-methoxyphenyl]diazen-1-yl}-1,3-diethyl-1H-imidazol-3-ium,
- salts of 2-{2-[4-(4-{4-[2-(1,3-dimethyl-1H-imidazol-3-ium-2-yl)diazen-1-yl]-3-methoxyphenyl}piperazin-1-yl)-2-methoxyphenyl]diazen-1-yl}-1,3-dimethyl-1H-imidazol-3-ium,
- salts of 2-{2-[4-(4-{4-[2-(1,3-diethyl-1H-imidazol-3-ium-2-yl)diazen-1-yl]-3-methoxyphenyl}piperazin-1-yl)-2-methoxyphenyl]diazen-1-yl}-1,3-diethyl-1H-imidazol-3-ium,
- salts of 2-{2-[4-(4-{4-[2-(1,3-dimethyl-1H-imidazol-3-ium-2-yl)diazen-1-yl]-2,5-dimethylphenyl}piperazin-1-yl)-2,5-dimethylphenyl]diazen-1-yl}-1,3-dimethyl-1H-imidazol-3-ium,
- salts of 2-{2-[4-(4-{4-[2-(1,3-diethyl-1H-imidazol-3-ium-2-yl)diazen-1-yl]-2,5-dimethylphenyl}piperazin-1-yl)-2,5-dimethylphenyl]diazen-1-yl}-1,3-diethyl-1H-imidazol-3-ium,
- salts of 5-{2-[4-(4-{4-[2-(1,4-dimethyl-1H-1,2,4-triazol-4-ium-5-yl)diazen-1-yl]phenyl}piperazin-1-yl)phenyl]diazen-1-yl}-1,4-dimethyl-1H-1,2,4-triazol-4-ium,
- salts of 5-{2-[4-(4-{4-[2-(1,4-diethyl-1H-1,2,4-triazol-4-ium-5-yl)diazen-1-yl]phenyl}piperazin-1-yl)phenyl]diazen-1-yl}-1,4-diethyl-1H-1,2,4-triazol-4-ium,
- salts of 5-{2-[4-(4-{4-[2-(1,4-dimethyl-1H-1,2,4-triazol-4-ium-5-yl)diazen-1-yl]-2-methylphenyl}piperazin-1-yl)-3-methylphenyl]diazen-1-yl}-1,4-dimethyl-1H-1,2,4-triazol-4-ium,
- salts of 5-{2-[4-(4-{4-[2-(1,4-diethyl-1H-1,2,4-triazol-4-ium-5-yl)diazen-1-yl]-2-methylphenyl}piperazin-1-yl)-3-methylphenyl]diazen-1-yl}-1,4-diethyl-1H-1,2,4-triazol-4-ium,
- salts of 5-{2-[4-(4-{4-[2-(1,4-dimethyl-1H-1,2,4-triazol-4-ium-5-yl)diazen-1-yl]-3-methylphenyl}piperazin-1-yl)-2-methylphenyl]diazen-1-yl}-1,4-dimethyl-1H-1,2,4-triazol-4-ium,
- salts of 5-{2-[4-(4-{4-[2-(1,4-diethyl-1H-1,2,4-triazol-4-ium-5-yl)diazen-1-yl]-3-methylphenyl}piperazin-1-yl)-2-methylphenyl]diazen-1-yl}-1,4-diethyl-1H-1,2,4-triazol-4-ium,
- salts of 5-{2-[4-(4-{4-[2-(1,4-dimethyl-1H-1,2,4-triazol-4-ium-5-yl)diazen-1-yl]-2-methoxyphenyl}piperazin-1-yl)-3-methoxyphenyl]diazen-1-yl}-1,4-dimethyl-1H-1,2,4-triazol-4-ium,
- salts of 5-{2-[4-(4-{4-[2-(1,4-diethyl-1H-1,2,4-triazol-4-ium-5-yl)diazen-1-yl]-2-methoxyphenyl}piperazin-1-yl)-3-methoxyphenyl]diazen-1-yl}-1,4-diethyl-1H-1,2,4-triazol-4-ium,
- salts of 5-{2-[4-(4-{4-[2-(1,4-dimethyl-1H-1,2,4-triazol-4-ium-5-yl)diazen-1-yl]-3-methoxyphenyl}piperazin-1-yl)-2-methoxyphenyl]diazen-1-yl}-1,4-dimethyl-1H-1,2,4-triazol-4-ium,
- salts of 5-{2-[4-(4-{4-[2-(1,4-diethyl-1H-1,2,4-triazol-4-ium-5-yl)diazen-1-yl]-3-methoxyphenyl}piperazin-1-yl)-2-methoxyphenyl]diazen-1-yl}-1,4-diethyl-1H-1,2,4-triazol-4-ium,
- salts of 5-{2-[4-(4-{4-[2-(1,4-dimethyl-1 H-1,2,4-triazol-4-ium-5-yl)diazen-1-yl]-2,5-dimethylphenyl}piperazin-1-yl)-2,5-dimethylphenyl]diazen-1-yl}-1,4-dimethyl-1H-1,2,4-triazol-4-ium,
- salts of 5-{2-[4-(4-{4-[2-(1,4-diethyl-1H-1,2,4-triazol-4-ium-5-yl)diazen-1-yl]-2,5-dimethylphenyl}piperazin-1-yl)-2,5-dimethylphenyl]diazen-1-yl}-1,4-diethyl-1H-1,2,4-triazol-4-ium,
- salts of 3-methyl-2-{2-[4-(5-{4-[2-(3-methyl-1,3-thiazol-3-ium-2-yl)diazen-1-yl]phenyl}-1,5-diazocan-1-yl)phenyl]diazen-1-yl}-1,3-thiazol-3-ium,
- salts of 3-ethyl-2-{2-[4-(5-{4-[2-(3-ethyl-1,3-thiazol-3-ium-2-yl)diazen-1-yl]phenyl}-1,5-diazocan-1-yl)phenyl]diazen-1-yl}-1,3-thiazol-3-ium,
- salts of 3-methyl-2-{2-[3-methyl-4-(5-{2-methyl-4-[2-(3-methyl-1,3-thiazol-3-ium-2-yl)diazen-1-yl]phenyl}-1,5-diazocan-1-yl)phenyl]diazen-1-yl}-1,3-thiazol-3-ium,
- salts of 3-ethyl-2-{2-[4-(5-{4-[2-(3-ethyl-1,3-thiazol-3-ium-2-yl)diazen-1-yl]-2-methylphenyl}-1,5-diazocan-1-yl)-3-methylphenyl]diazen-1-yl}-1,3-thiazol-3-ium,
- salts of 3-methyl-2-{2-[2-methyl-4-(5-{3-methyl-4-[2-(3-methyl-1,3-thiazol-3-ium-2-yl)diazen-1-yl]phenyl}-1,5-diazocan-1-yl)phenyl]diazen-1-yl}-1,3-thiazol-3-ium,
- salts of 3-ethyl-2-{2-[4-(5-{4-[2-(3-ethyl-1,3-thiazol-3-ium-2-yl)diazen-1-yl]-3-methylphenyl}-1,5-diazocan-1-yl)-2-methylphenyl]diazen-1-yl}-1,3-thiazol-3-ium,
- salts of 2-{2-[3-methoxy-4-(5-{2-methoxy-4-[2-(3-methyl-1,3-thiazol-3-ium-2-yl)diazen-1-yl]phenyl}-1,5-diazocan-1-yl)phenyl]diazen-1-yl}-3-methyl-1,3-thiazol-3-ium,
- salts of 3-ethyl-2-{2-[4-(5-{4-[2-(3-ethyl-1,3-thiazol-3-ium-2-yl)diazen-1-yl]-2-methoxyphenyl}-1,5-diazocan-1-yl)-3-methoxyphenyl]diazen-1-yl}-1,3-thiazol-3-ium,
- salts of 2-{2-[2-methoxy-4-(5-{3-methoxy-4-[2-(3-methyl-1,3-thiazol-3-ium-2-yl)diazen-1-yl]phenyl}-1,5-diazocan-1-yl)phenyl]diazen-1-yl}-3-methyl-1,3-thiazol-3-ium,
- salts of 3-ethyl-2-{2-[4-(5-{4-[2-(3-ethyl-1,3-thiazol-3-ium-2-yl)diazen-1-yl]-3-methoxyphenyl}-1,5-diazocan-1-yl)-2-methoxyphenyl]diazen-1-yl}-1,3-thiazol-3-ium,
- salts of 2-{2-[4-(5-{2,5-dimethyl-4-[2-(3-methyl-1,3-thiazol-3-ium-2-yl)diazen-1-yl]phenyl}-1,5-diazocan-1-yl)-2,5-dimethylphenyl]diazen-1-yl}-3-methyl-1,3-thiazol-3-ium,
- salts of 3-ethyl-2-{2-[4-(5-{4-[2-(3-ethyl-1,3-thiazol-3-ium-2-yl)diazen-1-yl]-2,5-dimethylphenyl}-1,5-diazocan-1-yl)-2,5-dimethylphenyl]diazen-1-yl}-1,3-thiazol-3-ium,
- salts of 2-{2-[4-(5-{4-[2-(1,3-dimethyl-1H-imidazol-3-ium-2-yl)diazen-1-yl]phenyl}-1,5-diazocan-1-yl)phenylldiazen-1-yl}-1,3-dimethyl-IH-imidazol-3-ium,
- salts of 2-{2-[4-(5-{4-[2-(1,3-diethyl-1H-imidazol-3-ium-2-yl)diazen-1-yl]phenyl}-1,5-diazocan-1-yl)phenyl]diazen-1-yl}-1,3-diethyl-1H-imidazol-3-ium,
- salts of 2-{2-[4-(5-{4-[2-(1,3-dimethyl-1H-imidazol-3-ium-2-yl)diazen-1-yl]-2-methylphenyl}-1,5-diazocan-1-yl)-3-methylphenyl]diazen-1-yl}-1,3-dimethyl-1H-imidazol-3-ium,
- salts of 2-{2-[4-(5-{4-[2-(1,3-diethyl-1H-imidazol-3-ium-2-yl)diazen-1-yl]-2-methylphenyl}-1,5-diazocan-1-yl)-3-methylphenyl]diazen-1-yl}-1,3-diethyl-1H-imidazol-3-ium,
- salts of 2-{2-[4-(5-{4-[2-(1,3-dimethyl-1H-imidazol-3-ium-2-yl)diazen-1-yl]-3-methylphenyl}-1,5-diazocan-1-yl)-2-methylphenyl]diazen-1-yl}-1,3-dimethyl-1H-imidazol-3-ium,
- salts of 2-{2-[4-(5-{4-[2-(1,3-diethyl-1H-imidazol-3-ium-2-yl)diazen-1-yl]-3-methylphenyl}-1,5-diazocan-1-yl)-2-methylphenyl]diazen-1-yl}-1,3-diethyl-1H-imidazol-3-ium,
- salts of 2-{2-[4-(5-{4-[2-(1,3-dimethyl-1H-imidazol-3-ium-2-yl)diazen-1-yl]-2-methoxyphenyl}-1,5-diazocan-1-yl)-3-methoxyphenyl]diazen-1-yl}-1,3-dimethyl-1H-imidazol-3-ium,
- salts of 2-{2-[4-(5-{4-[2-(1,3-diethyl-1H-imidazol-3-ium-2-yl)diazen-1-yl]-2-methoxyphenyl}-1,5-diazocan-1-yl)-3-methoxyphenyl]diazen-1-yl}-1,3-diethyl-1H-imidazol-3-ium,
- salts of 2-{2-[4-(5-{4-[2-(1,3-dimethyl-1H-imidazol-3-ium-2-yl)diazen-1-yl]-3-methoxyphenyl}-1,5-diazocan-1-yl)-2-methoxyphenyl]diazen-1-yl}-1,3-dimethyl-1H-imidazol-3-ium,
- salts of 2-{2-[4-(5-{4-[2-(1,3-diethyl-1H-imidazol-3-ium-2-yl)diazen-1-yl]-3-methoxyphenyl}-1,5-diazocan-1-yl)-2-methoxyphenyl]diazen-1-yl}-1,3-diethyl-1H-imidazol-3-ium,
- salts of 2-{2-[4-(5-{4-[2-(1,3-dimethyl-1H-imidazol-3-ium-2-yl)diazen-1-yl]-2,5-dimethylphenyl}-1,5-diazocan-1-yl)-2,5-dimethylphenyl]diazen-1-yl}-1,3-dimethyl-1H-imidazol-3-ium,
- salts of 2-{2-[4-(5-{4-[2-(1,3-diethyl-1 H-imidazol-3-ium-2-yl)diazen-1-yl]-2,5-dimethylphenyl}-1,5-diazocan-1-yl)-2,5-dimethylphenyl]diazen-1-yl}-1,3-diethyl-1H-imidazol-3-ium,
- salts of 5-{2-[4-(5-{4-[2-(1,4-dimethyl-1H-1,2,4-triazol-4-ium-5-yl)diazen-1-yl]phenyl}-1,5-diazocan-1-yl)phenyl]diazen-1-yl}-1,4-dimethyl-1H-1,2,4-triazol-4-ium,
- salts of 5-{2-[4-(5-{4-[2-(1,4-diethyl-1H-1,2,4-triazol-4-ium-5-yl)diazen-1-yl]phenyl}-1,5-diazocan-1-yl)phenyl]diazen-1-yl}-1,4-diethyl-1H-1,2,4-triazol-4-ium,
- salts of 5-{2-[4-(5-{4-[2-(1,4-dimethyl-1H-1,2,4-triazol-4-ium-5-yl)diazen-1-yl]-2-methylphenyl}-1,5-diazocan-1-yl)-3-methylphenyl]diazen-1-yl}-1,4-dimethyl-1H-1,2,4-triazol-4-ium,
- salts of 5-{2-[4-(5-{4-[2-(1,4-diethyl-1H-1,2,4-triazol-4-ium-5-yl)diazen-1-yl]-2-methylphenyl}-1,5-diazocan-1-yl)-3-methylphenyl]diazen-1-yl}-1,4-diethyl-1H-1,2,4-triazol-4-ium,
- salts of 5-{2-[4-(5-{4-[2-(1,4-dimethyl-1H-1,2,4-triazol-4-ium-5-yl)diazen-1-yl]-3-methylphenyl}-1,5-diazocan-1-yl)-2-methylphenyl]diazen-1-yl}-1,4-dimethyl-1H-1,2,4-triazol-4-ium,
- salts of 5-{2-[4-(5-{4-[2-(1,4-diethyl-1H-1,2,4-triazol-4-ium-5-yl)diazen-1-yl]-3-methylphenyl}-1,5-diazocan-1-yl)-2-methylphenyl]diazen-1-yl}-1,4-diethyl-1H-1,2,4-triazol-4-ium,
- salts of 5-{2-[4-(5-{4-[2-(1,4-dimethyl-1H-1,2,4-triazol-4-ium-5-yl)diazen-1-yl]-2-methoxy phenyl}-1,5-diazocan-1-yl)-3-methoxyphenyl]diazen-1-yl}-1,4-dimethyl-1H-1,2,4-triazol-4-ium,
- salts of 5-{2-[4-(5-{4-[2-(1,4-diethyl-1H-1,2,4-triazol-4-ium-5-yl)diazen-1-yl]-2-methoxyphenyl}-1,5-diazocan-1-yl)-3-methoxyphenyl]diazen-1-yl}-1,4-diethyl-1H-1,2,4-triazol-4-ium,
- salts of 5-{2-[4-(5-{4-[2-(1,4-dimethyl-1H-1,2,4-triazol-4-ium-5-yl)diazen-1-yl]-3-methoxyphenyl}-1,5-diazocan-1-yl)-2-methoxyphenyl]diazen-1-yl}-1,4-dimethyl-1H-1,2,4-triazol-4-ium,
- salts of 5-{2-[4-(5-{4-[2-(1,4-diethyl-1H-1,2,4-triazol-4-ium-5-yl)diazen-1-yl]-3-methoxyphenyl}-1,5-diazocan-1-yl)-2-methoxyphenyl]diazen-1-yl}-1,4-diethyl-1H-1,2,4-triazol-4-ium,
- salts of 5-{2-[4-(5-{4-[2-(1,4-dimethyl-1H-1,2,4-triazol-4-ium-5-yl)diazen-1-yl]-2,5-dimethylphenyl}-1,5-diazocan-1-yl)-2,5-dimethylphenyl]diazen-1-yl}-1,4-dimethyl-1H-1,2,4-triazol-4-ium, and/or
- salts of 5-{2-[4-(5-{4-[2-(1,4-diethyl-1H-1,2,4-triazol-4-ium-5-yl)diazen-1-yl]-2,5-dimethylphenyl}-1,5-diazocan-1-yl)-2,5-dimethylphenyl]diazen-1-yl}-1,4-diethyl-1H-1,2,4-triazol-4-ium.

8. The agent according to one of claims 1 to 7, **characterized in that** it contains, based on the total weight of the agent, one or more direct dyes (a) of formula (I) in a total amount of from 0.01 to 4.5 wt.%, preferably from 0.05 to 2.8 wt.%, more preferably from 0.1 to 2.2 wt.% and particularly preferably from 0.2 to 1.2 wt.%.

9. The agent according to one of claims 1 to 8, **characterized in that** it contains (b) at least one at least one anionic surfactant selected from
- linear and branched fatty acids having 8 to 30 C atoms,
- ether carboxylic acids of formula R-O-(CH₂-CH₂O)ₓH₂-COOM, in which
R represents a linear alkyl group having 8 to 30 C atoms,
x represents an integer from 0 to 16,
M represents hydrogen or a metal such as an alkali metal, in particular sodium, potassium, lithium, an alkaline earth metal, in particular ½ magnesium, ½ calcium, ½ zinc, or an ammonium ion (NH4⁺),
- acyl sarcosides having 8 to 24 C atoms in the acyl group,
- acyl taurides having 8 to 24 C atoms in the acyl group,
- acyl isethionates having 8 to 24 C atoms in the acyl group, which can be obtained by esterification of C₈-C₂₄ fatty acids with the sodium salt of 2-hydroxyethanesulfonic acid (isethionic acid),
- amide ether carboxylic acids, R¹-CO-NR²-CH₂CH₂-O-(CH₂CH₂O)_{y}CH₂COOM, in which
R¹ represents a C2-C30 alkyl group,
y represents an integer from 1 to 20,
R² represents hydrogen, a methyl, ethyl, propyl, isopropyl, n-butyl, t-butyl or iso-butyl functional group, and
M represents hydrogen or a metal such as an alkali metal, in particular sodium, potassium, lithium, an alkaline earth metal, in particular ½ magnesium, ½ calcium, ½ zinc, or an ammonium ion (NH4⁺).

10. The agent according to one of claims 1 to 9, **characterized in that** at least one at least one anionic surfactant (b) from the group of ether carboxylic acids of formula (B1) contains R-
O-(CH2-CH2O)x-CH2-COOM (B1),
in which
R represents a linear alkyl group having 8 to 30 C atoms,
x represents an integer from 0 to 16,
M represents hydrogen or a metal such as an alkali metal, in particular sodium, potassium, lithium, an alkaline earth metal, in particular ½ magnesium, ½ calcium, ½ zinc, or an ammonium ion (NH4⁺).

11. The agent according to one of claims 1 to 10, **characterized in that** it contains, based on the total weight of the agent, one or more anionic surfactants (b) in a total amount of from 0.05 to 9.5 wt.%, preferably from 0.1 to 3.1 wt.%, more preferably from 0.15 to 2.5 wt.% and very particularly preferably from 0.2 to 0.9 wt.%.

12. The agent according to one of claims 1 to 11, **characterized in that** the weight ratio of all the anionic surfactants of formula (B1) contained in the agent with respect to the total amount of the anionic surfactants contained in the agent is 0.5, preferably 0.6, more preferably 0.75 and particularly preferably 0.9, the anionic surfactants of formula (B1) being the following surfactants:
R-O-(CH2-CH2O)x-CH2-COOM (B1),
where
R is equal to a linear alkyl group having 8 to 30 C atoms,
x is equal to an integer from 0 to 16,
M is equal to hydrogen or a metal such as an alkali metal, in particular sodium, potassium, lithium, an alkaline earth metal, in particular ½ magnesium, ½ calcium, ½ zinc, or an ammonium ion (NH4⁺).

13. The agent according to one of claims 1 to 12, **characterized in that** it contains, based on the total weight of the agent, 0.5 to 12.5 wt.%, preferably 2.5 to 10 wt.% and particularly preferably 4.0 to 9.0 wt.%, hydrogen peroxide.

14. The agent according to one of claims 1 to 13, **characterized in that** it contains at least one persulfate from the group of ammonium peroxodisulfate, potassium peroxodisulfate and sodium peroxodisulfate.

15. The agent according to one of claims 1 to 14, **characterized in that** it contains, based on the total weight of the agent,
- 0.2 wt.% to 13.5 wt.%, preferably 0.5 wt.% to 1.5 wt.%, ammonium peroxodisulfate
- 0.5 wt.% to 4.5 wt.%, preferably 1.5 wt.% to 2.5 wt.%, potassium peroxodisulfate and
- 0.2 wt.% to 1.8 wt.%, preferably 0.4 wt.% to 0.8 wt.%, sodium peroxodisulfate.

## Revendications

1. Agent de modification de la couleur des cheveux humains, contenant dans un support cosmétique (a) au moins un colorant direct de formule (I), 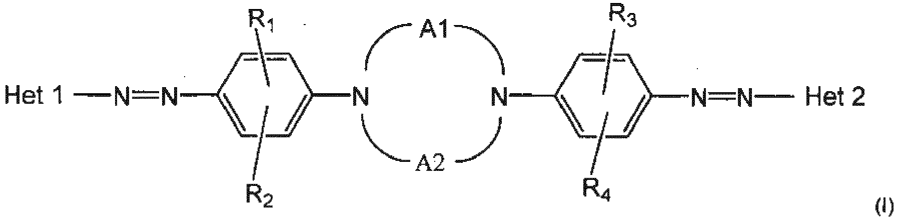 dans laquelle
Het1 et Het2 représentent indépendamment l'un de l'autre, l'une des structures (II), (III), (IV), (V), (VI) ou (VII), 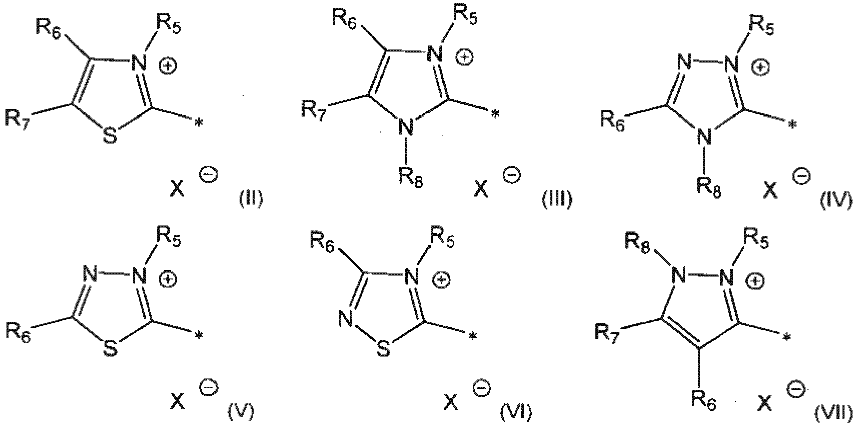
R1 et R3 représentent indépendamment l'un de l'autre, un atome d'hydrogène, un groupe alkyle en C₁ à C₆, un atome d'halogène choisi dans le groupe comprenant le chlore, le brome, le fluor ou l'iode, un groupe alcoxy en C₁ à C₆ ou un groupe nitro,
R2 et R4 représentent indépendamment l'un de l'autre, un atome d'hydrogène, un groupe alkyle en C₁ à C₆, un atome d'halogène choisi dans le groupe comprenant le chlore, le brome, le fluor ou l'iode, un groupe alcoxy en C₁ à C₆ ou un groupe nitro,
R5 et R8 représentent chacun, indépendamment l'un de l'autre, un groupe alkyle en C₁ à C₆ ou un groupe alcényle en C₂ à C₆,
R6 et R7 représentent chacun, indépendamment l'un de l'autre, un atome d'hydrogène, un groupe alkyle en C₁ à C₆, un atome d'halogène choisi dans le groupe comprenant le chlore, le brome, le fluor ou l'iode, un groupe alcoxy C₁ à C₆ ou un groupe nitro,
A1 et A2 représentent indépendamment l'un de l'autre, un groupe de formule (VIII) ou (IX),
*-(CH₂)n-* (VIII)
*-(CH₂)m-O-(CH₂)p-* (IX)
n représente un nombre entier situé dans la plage allant de 2 à 6,
m, p représentent chacun, indépendamment l'un de l'autre, le nombre 2 ou le nombre 3,
X- représente un anion physiologiquement compatible, de préférence dans le groupe comprenant le chlorure, le bromure, l'iodure, le méthylsulfate, le méthylsulfonate, le p-toluènesulfonate, l'acétate, le sulfate d'hydrogène, un demi-sulfate ou un demi-tétrachlorozincate, et
(b) au moins un tensioactif.

2. Agent selon la revendication 1, **caractérisé en ce qu'**il contient (a) au moins un colorant direct de formule générale (I), dans laquelle
Het1 et Het2 représentent indépendamment l'un de l'autre, l'une des structures (II), (III) ou (IV).

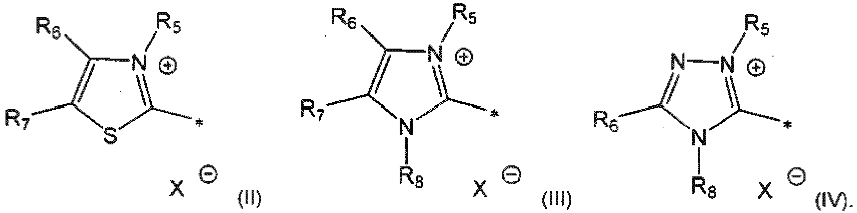

3. Agent selon l'une des revendications 1 à 2, **caractérisé en ce qu'**il contient (a) au moins un colorant direct de formule générale (I), dans laquelle
R1 et R3 représentent indépendamment l'un de l'autre, un atome d'hydrogène, un groupe alkyle en C₁ à C₆ ou un groupe alcoxy en C₁ à C₆.

4. Agent selon l'une des revendications 1 à 3, **caractérisé en ce qu'**il contient (a) au moins un colorant direct de formule générale (I), dans laquelle
R2 et R4 représentent indépendamment l'un de l'autre, un atome d'hydrogène, un groupe alkyle en C₁ à C₆ ou un groupe alcoxy en C₁ à C₆, de manière particulièrement préférée un atome d'hydrogène.

5. Agent selon l'une des revendications 1 à 4, **caractérisé en ce qu'**il contient (a) au moins un colorant direct de formule générale (I), dans laquelle
R5 et R8 représentent chacun, indépendamment l'un de l'autre, un groupe alkyle en C₁ à C₆, et
R6 et R7 représentent chacun, indépendamment l'un de l'autre, un atome d'hydrogène, un groupe alkyle en C₁ à C₆ ou un groupe alcoxy en C₁ à C₆.

6. Agent selon l'une des revendications 1 à 5, **caractérisé en ce qu'**il contient (a) au moins un colorant direct de formule générale (I), dans laquelle
A1 et A2 représentent tous deux un groupe de formule (VIII),
*-(CH₂)n-* (VIII).
dans laquelle
n représente les nombres 2 ou 3, de préférence le nombre 2,

7. Agent selon l'une des revendications 1 à 6, **caractérisé en ce qu'**il contient (a) au moins un colorant direct de formule générale (I), choisi parmi les
- Sels de 3-méthyl-2-{2-[4-(4-{4-[2-(3-méthyl-1,3-thiazole-3-ium-2-yl)diazène-1-yl]phényl}pipérazine-1-yl)phényl]diazène-1-yl}-1,3-thiazole-3-ium,
- Sels de 3-éthyl-2-{2-[4-(4-{4-[2-(3-éthyl-1,3-thiazole-3-ium-2-yl)diazène-1-yl]phényl}pipérazine-1-yl)phényl]diazène-1-yl}-1,3-thiazole-3-ium,
- Sels de 3-méthyl-2-{2-[3-méthyl-4-(4-{2-méthyl-4-[2-(3-méthyl-1,3-thiazole-3-ium-2-yl)diazène-1-yl]phényl}pipérazine-1-yl)phényl]diazène-1-yl}-1,3-thiazole-3-ium,
- Sels de 3-éthyl-2-{2-[4-(4-{4-[2-(3-éthyl-1,3-thiazole-3-ium-2-yl)diazène-1-yl]-2-méthylphényl}pipérazine-1-yl)-3-méthylphényl]diazène-1-yl}-1,3-thiazole-3-ium,
- Sels de 3-méthyl-2-{2-[2-méthyl-4-(4-{3-méthyl-4-[2-(3-méthyl-1,3-thiazole-3-ium-2-yl)diazène-f-yl]phényl}pipérazine-1-yl)phényl]diazène-1-yl}-1,3-thiazole-3-ium,
- Sels de 3-éthyl-2-{2-[4-(4-{4-[2-(3-éthyl-1,3-thiazole-3-ium-2-yl)diazène-1-yl]-3-méthylphényl}pipérazine-1-yl)-2-méthylphényl]diazène-1-yl}-1,3-thiazole-3-ium,
- Sels de 2-{2-[3-méthoxy-4-(4-{2-méthoxy-4-[2-(3-méthyl-1,3-thiazole-3-ium-2-yl)diazène-1-yl]phényl}pipérazine-1-yl)phényl]diazène-1-yl}-3-méthyl-1,3-thiazole-3-ium,
- Sels de 3-éthyl-2-{2-[4-(4-{4-[2-(3-éthyl-1,3-thiazole-3-ium-2-yl)diazène-1-yl]-2-méthoxyphényl}pipérazine-1-yl)-3-méthoxyphényl]diazène-1-yl}-1,3-thiazole-3-ium,
- Sels de 2-{2-[2-méthoxy-4-(4-{3-méthoxy-4-[2-(3-méthyl-1,3-thiazole-3-ium-2-yl)diazène-1-yl]phényl}pipérazine-1-yl)phényl]diazène-1-yl}-3-méthyl-1,3-thiazole-3-ium,
- Sels de 3-éthyl-2-{2-[4-(4-{4-[2-(3-éthyl-1,3-thiazole-3-ium-2-yl)diazène-1-yl]-3-méthoxyphényl}pipérazine-1-yl)-2-méthoxyphényl]diazène-1-yl}-1,3-thiazole-3-ium,
- Sels de 2-{2-[4-(4-{2,5-diméthyl-4-[2-(3-méthyl-1,3-thiazole-3-ium-2-yl)diazène-1-yl]phényl}pipérazine-1-yl)-2,5-diméthylphényl]diazène-1-yl}-3-méthyl-1,3-thiazole-3-ium,
- Sels de 3-éthyl-2-{2-[4-(4-{4-[2-(3-éthyl-1,3-thiazole-3-ium-2-yl)diazène-1-yl]-2,5-diméthylphényl}pipérazine-1-yl)-2,5-diméthylphényl]diazène-1-yl}-1,3-thiazole-3-ium,
- Sels de 2-{2-[4-(4-{4-[2-(1,3-diméthyl-1H-imidazol-3-ium-2-yl)diazène-1-yl]phényl}pipérazine-1-yl)phényl]diazène-1-yl}-1,3-diméthyMH-imidazole-3-ium,
- Sels de 2-{2-[4-(4-{4-[2-(1,3-diéthyl-1 H-imidazole-3-ium-2-yl)diazène-1-yl]phényl}pipérazine-1-yl)phényl]diazène-1-yl}-1,3-diéthyl-1H-imidazole-3-ium,
- Sels de 2-{2-[4-(4-{4-[2-(1,3-diméthyl-1H-imidazole-3-ium-2-yl)diazène-1-yl]-2-méthyl-phényl}pipérazine-1-yl)-3-méthylphényl]diazène-1-yl}-1,3-diméthyl-1H-imidazole-3-ium,
- Sels de 2-{2-[4-(4-{4-[2-(1,3-diéthyl-1H-imidazole-3-ium-2-yl)diazène-1-yl]-2-méthyl-phényl}pipérazin-1-yl)-3-méthylphényl]diazène-1-yl}-1,3-diéthyl-1H-imidazole-3-ium,
- Sels de 2-{2-[4-(4-{4-[2-(1,3-diméthyl-1H-imidazole-3-ium-2-yl)diazène-1-yl]-3-méthyl-phényl}pipérazine-1-yl)-2-méthylphényl]diazène-1-yl}-1,3-diméthyl-1H-imidazole-3-ium,
- Sels de 2-{2-[4-(4-{4-[2-(1,3-diéthyl-1H-imidazole-3-ium-2-yl)diazène-1-yl]-3-méthyl-phényl}pipérazine-1-yl)-2-méthylphényl]diazène-1-yl}-1,3-diéthyl-1H-imidazole-3-ium,
- Sels de 2-{2-[4-(4-{4-[2-(1,3-diméthyl-1H-imidazole-3-ium-2-yl)diazène-1-yl]-2-méthoxy-phényl}pipérazin-1-yl)-3-méthoxyphényl]diazène-1-yl}-1,3-dimethyl-1H-imidazole-3-ium,
- Sels de 2-{2-[4-(4-{4-[2-(1,3-diéthyl-1H-imidazole-3-ium-2-yl)diazène-1-yl]-2-méthoxy-phényl}pipérazine-1-yl)-3-méthoxyphényl]diazène-1-yl}-1,3-diéthyl-1H-imidazole-3-ium,
- Sels de 2-{2-[4-(4-{4-[2-(1,3-diméthyl-1H-imidazole-3-ium-2-yl)diazène-1-yl]-3-méthoxy-phényl}pipérazine-1-yl)-2-méthoxyphényl]diazène-1-yl}-1,3-dimethyl-1H-imidazole-3-ium,
- Sels de 2-{2-[4-(4-{4-[2-(1,3-diéthyl-1H-imidazole-3-ium-2-yl)diazène-1-yl]-3-méthoxy-phényl}pipérazine-1-yl)-2-méthoxyphényl]diazène-1-yl}-1,3-diéthyl-1H-imidazole-3-ium,
- Sels de 2-{2-[4-(4-{4-[2-(1,3-diméthyl-1H-imidazole-3-ium-2-yl)diazène-1-yl]-2,5-diméthyl-phényl}pipérazine-1-yl)-2,5-diméthylphényl]diazène-1-yl}-1,3-diméthyl-1H-imidazole-3-ium,
- Sels de 2-{2-[4-(4-{4-[2-(1,3-diéthyl-1H-imidazole-3-ium-2-yl)diazène-1-yl]-2,5-diméthyl-phényl}pipérazine-1-yl)-2,5-diméthylphényl]diazène-1-yl}-1,3-diéthyl-1H-imidazole-3-ium,
- Sels de 5-{2-[4-(4-{4-[2-(1,4-diméthyl-1H-1,2,4-triazole-4-ium-5-yl)diazène-1-yl]phényl}pipérazine-1-yl)phényl]diazène-1-yl}-1,4-diméthyl-1H-1,2,4-triazole-4-ium,
- Sels de 5-{2-[4-(4-{4-[2-(1,4-diéthyl-1H-1,2,4-triazole-4-ium-5-yl)diazène-1-yl]phényl}pipérazine-1-yl)phényl]diazène-1-yl}-1,4-diéthyl-1H-1,2,4-triazole-4-ium,
- Sels de 5-{2-[4-(4-{4-[2-(1,4-diméthyl-1H-1,2,4-triazole-4-ium-5-yl)diazène-1-yl]-2-méthylphényl}pipérazine-1-yl)-3-méthylphényl]diazène-1-yl}-1,4-diméthyl-1H-1,2,4-triazole-4-ium,
- Sels de 5-{2-[4-(4-{4-[2-(1,4-diéthyl-1H-1,2,4-triazole-4-ium-5-yl)diazène-1-yl]-2-méthylphényl}pipérazine-1-yl)-3-méthylphényl]diazène-1-yl}-1,4-diéthyl-1H-1,2,4-triazole-4-ium,
- Sels de 5-{2-[4-(4-{4-[2-(1,4-diméthyl-1H-1,2,4-triazole-4-ium-5-yl)diazène-1-yl]-3-méthylphényl}pipérazine-1-yl)-2-méthylphényl]diazène-1-yl}-1,4-diméthyl-1H-1,2,4-triazole-4-ium,
- Sels de 5-{2-[4-(4-{4-[2-(1,4-diéthyl-1H-1,2,4-triazole-4-ium-5-yl)diazène-1-yl]-3-méthylphényl}pipérazine-1-yl)-2-méthylphényl]diazène-1-yl}-1,4-diéthyl-1H-1,2,4-triazole-4-ium,
- Sels de 5-{2-[4-(4-{4-[2-(1,4-diméthyl-1H-1,2,4-triazole-4-ium-5-yl)diazène-1-yl]-2-méthoxyphényl}pipérazine-1-yl)-3-méthoxyphényl]diazène-1-yl}-1,4-diméthyl-1H-1,2,4-triazole-4-ium,
- Sels de 5-{2-[4-(4-{4-[2-(1,4-diéthyl-1H-1,2,4-triazole-4-ium-5-yl)diazène-1-yl]-2-méthoxyphényl}pipérazine-1-yl)-3-méthoxyphényl]diazène-1-yl}-1,4-diéthyl-1H-1,2,4-triazole-4-ium,
- Sels de 5-{2-[4-(4-{4-[2-(1,4-diméthyl-1H-1,2,4-triazole-4-ium-5-yl)diazène-1-yl]-3-méthoxyphényl}pipérazine-1-yl)-2-méthoxyphényl]diazène-1-yl}-1,4-diméthyl-1H-1,2,4-triazole-4-ium,
- Sels de 5-{2-[4-(4-{4-[2-(1,4-dyléthyl-1H-1,2,4-triazole-4-ium-5-yl)diazène-1-yl]-3-méthoxyphényl}pipérazine-1-yl)-2-méthoxyphényl]diazène-1-yl}-1,4-diéthyl-1H-1,2,4-triazole-4-ium,
- Sels de 5-{2-[4-(4-{4-[2-(1,4-diméthyl-1H-1,2,4-triazole-4-ium-5-yl)diazène-1-yl]-2,5-diméthyl-phényl}pipérazine-1-yl)-2,5-diméthylphényl]diazène-1-yl}-1,4-dimethyl-1H-1,2,4-triazole-4-ium,
- Sels de 5-{2-[4-(4-{4-[2-(1,4-diéthyl-1H-1,2,4-triazole-4-ium-5-yl)diazène-1-yl]-2,5-diméthyl-phényl}pipérazine-1-yl)-2,5-diméthylphényl]diazène-1-yl}-1,4-diéthyl-1H-1,2,4-triazole-4-ium,
- Sels de 3-méthyl-2-{2-[4-(5-{4-[2-(3-méthyl-1,3-thiazole-3-ium-2-yl)diazène-1-yl]phényl}-1,5-diazocane-1-yl)phényl]diazène-1-yl}-1,3-thiazole-3-ium,
- Sels de 3-éthyl-2-{2-[4-(5-{4-[2-(3-éthyl-1,3-thiazole-3-ium-2-yl)diazène-1-yl]phényl}-1,5-diazocane-1-yl)phényl]diazène-1-yl}-1,3-thiazole-3-ium,
- Sels de 3-méthyl-2-{2-[3-méthyl-4-(5-{2-méthyl-4-[2-(3-méthyl-1,3-thiazole-3-ium-2-yl)diazène-1-yl]phényl}-1,5-diazocane-1-yl)phényl]diazène-1-yl}-1,3-thiazole-3-ium,
- Sels de 3-éthyl-2-{2-[4-(5-{4-[2-(3-éthyl-1,3-thiazole-3-ium-2-yl)diazène-1-yl]-2-méthylphényl}-1,5-diazocane-1-yl)-3-méthylphényl]diazène-1-yl}-1,3-thiazole-3-ium,
- Sels de 3-méthyl-2-{2-[2-méthyl-4-(5-{3-méthyl-4-[2-(3-méthyl-1,3-thiazole-3-ium-2-yl)diazène-1-yl]phényl}-1,5-diazocane-1-yl)phényl]diazène-1-yl}-1,3-thiazole-3-ium,
- Sels de 3-éthyl-2-{2-[4-(5-{4-[2-(3-éthyl-1,3-thiazole-3-ium-2-yl)diazène-1-yl]-3-méthylphényl}-1,5-diazocane-1-yl)-2-méthylphényl]diazène-1-yl}-1,3-thiazole-3-ium,
- Sels de 2-{2-[3-méthoxy-4-(5-{2-méthoxy-4-[2-(3-méthyl-1,3-thiazole-3-ium-2-yl)diazène-1-yl]phényl}-1,5-diazocane-1-yl)phényl]diazène-1-yl}3-méthyl-1,3-thiazole-3-ium,
- Sels de 3-éthyl-2-{2-[4-(5-{4-[2-(3-éthyl-1,3-thiazole-3-ium-2-yl)diazène-1-yl]-2-méthoxyphényl}-1,5-diazocane-1-yl)-3-méthoxyphényl]diazène-1-yl}-1,3-thiazole-3-ium,
- Sels de 2-{2-[2-méthoxy-4-(5-{3-méthoxy-4-[2-(3-méthyl-1,3-thiazole-3-ium-2-yl)diazène-1-yl]phényl}-1,5-diazocane-1-yl)phényl]diazène-1-yl}3-méthyl-1,3-thiazole-3-ium,
- Sels de 3-éthyl-2-{2-[4-(5-{4-[2-(3-éthyl-1,3-thiazole-3-ium-2-yl)diazène-1-yl]-3-méthoxyphényl}-1,5-diazocane-1-yl)-2-méthoxyphényl]diazène-1-yl}-1,3-thiazole-3-ium,
- Sels de 2-{2-[4-(5-{2,5-diméthyl-4-[2-(3-méthyl-1,3-thiazole-3-ium-2-yl)diazène-1-yl]phényl}-1,5-diazocane-1-yl)-2,5-diméthylphényl]diazène-1-yl}-3-méthyl-1,3-thiazole-3-ium,
- Sels de 3-éthyl-2-{2-[4-(5-{4-[2-(3-éthyl-1,3-thiazole-3-ium-2-yl)diazène-1-yl]-2,5-diméthylphényl}-1,5-diazocane-1-yl)-2,5-diméthylphényl]diazène-1-yl}-1,3-thiazole-3-ium,
- Sels de 2-{2-[4-(5-{4-[2-(1,3-diméthyl-1H-imidazole-3-ium-2-yl)diazène-1-yl]phényl}-1,5-diazocane-1-yl)phénylldiazène-1-yl}-1,3-diméthyl-1H-imidazole-3-ium,
- Sels de 2-{2-[4-(5-{4-[2-(1,3-diéthyl-1H-imidazole-3-ium-2-yl)diazène-1-yl]phényl}-1,5-diazocane-1-yl)phényl]diazène-1-yl}-1,3-diéthyl-1H-imidazole-3-ium,
- Sels de 2-{2-[4-(5-{4-[2-(1,3-diméthyl-1H-imidazole-3-ium-2-yl)diazène-1-yl]-2-méthylphényl}-1,5-diazocane-1-yl)-3-méthylphényl]diazène-1-yl}-1,3-diméthyl-1H-imidazole-3-ium,
- Sels de 2-{2-[4-(5-{4-[2-(1,3-diéthyl-1H-imidazole-3-ium-2-yl)diazène-1-yl]-2-méthylphényl}-1,5-diazocane-1-yl)-3-méthylphényl]diazène-1-yl}-1,3-diéthyl-1H-imidazole-3-ium,
- Sels de 2-{2-[4-(5-{4-[2-(1,3-diméthyl-1H-imidazole-3-ium-2-yl)diazène-1-yl]-3-méthylphényl}-1,5-diazocane-1-yl)-2-méthylphényl]diazène-1-yl}-1,3-diméthyl-1H-imidazole-3-ium,
- Sels de 2-{2-[4-(5-{4-[2-(1,3-diéthyl-1H-imldazole-3-ium-2-yl)diazène-1-yl]-3-méthylphényl}-1,5-diazocane-1-yl)-2-méthylphényl]diazène-1-yl}-1,3-diéthyl-1H-imidazole-3-ium,
- Sels de 2-{2-[4-(5-{4-[2-(1,3-diméthyl-1H-imidazole-3-ium-2-yl)diazène-1-yl]-2-méthoxyphényl}-1,5-diazocane-1-yl)-3-méthoxyphényl]diazène-1-yl}-1,3-diméthyl-1H-imidazole-3-ium,
- Sels de 2-{2-[4-(5-{4-[2-(1,3-diéthyl-1H-imidazole-3-ium-2-yl)diazène-1-yl]-2-méthoxyphényl}-1,5-diazocane-1-yl)-3-méthoxyphényl]diazène-1-yl}-1,3-diethyl-1H-imidazole-3-ium,
- Sels de 2-{2-[4-(5-{4-[2-(1,3-diméthyl-1H-imidazole-3-ium-2-yl)diazène-1-yl]-3-méthoxyphényl}-1,5-diazocane-1-yl)-2-méthoxyphényl]diazène-1-yl}-1,3-diméthyl-1H-imidazole-3-ium,
- Sels de 2-{2-[4-(5-{4-[2-(1,3-diéthyl-1H-imidazole-3-ium-2-yl)diazène-1-yl]-3-méthoxyphényl}-1,5-diazocane-1-yl)-2-méthoxyphényl]diazène-1-yl}-1,3-diéthyl-1H-imidazole-3-ium,
- Sels de 2-{2-[4-(5-{4-[2-(1,3-diméthyl-1H-imidazol-3-ium-2-yl)diazène-1-yl]-2,5-diméthylphényl}-1,5-diazocane-1-yl)-2,5-diméthylphényl]diazène-1-yl]-1,3-diméthyl-1H-imidazole-3-ium,
- Sels de 2-{2-[4-(5-{4-[2-(1,3-diéthyl-1H-imidazole-3-ium-2-yl)diazène-1-yl]-2,5-diméthylphényl}-1,5-diazocane-1-yl)-2,5-diméthylphényl]diazène-1-yl}-1,3-diéthyl-1H-imidazole-3-ium,
- Sels de 5-{2-[4-(5-{4-[2-(1,4-diméthyl-1H-1,2,4-triazole-4-ium-5-yl)diazène-1-yl]phényl}-1,5-diazocane-1-yl)phényl]diazène-1-yl}-1,4-diméthyl-1H-1,2,4-triazole-4-ium,
- Sels de 5-{2-[4-(5-{4-[2-(1,4-diéthyl-1H-1,2,4-triazol-4-ium-5-yl)diazène-1-yl]phényl}-1,5-diazocane-1-yl)phényl]diazène-1-yl}-1,4-dièthyl-1H-1,2,4-triazole-4-ium,
- Sels de 5-{2-[4-(5-{4-[2-(1,4-diméthyl-1H-1,2,4-trlazole-4-ium-5-yl)dlazène-1-yl]-2-méthylphényl}-1,5-diazocane-1-yl)-3-méthylphényl]diazène-1-yl}-1,4-diméthyl-1H-1,2,4-triazole-4-ium,
- Sels de 5-{2-[4-(5-{4-[2-(1,4-diéthyl-1H-1,2,4-triazole-4-ium-5-yl)diazène-1-yl]-2-méthylphényl}-1,5-diazocane-1-yl)-3-méthylphényl]diazène-1-yl}-1,4-diéthyl-1H-1,2,4-triazole-4-ium,
- Sels de 5-{2-[4-(5-{4-[2-(1,4-diméthyl-1H-1,2,4-triazole-4-ium-5-yl)diazène-1-yl]-3-méthylphényl}-1,5-diazocane-1-yl)-2-méthylphényl]diazène-1-yl}-1,4-diméthyl-1H-1,2,4-triazole-4-ium,
- Sels de 5-{2-[4-(5-{4-[2-(1,4-diéthyl-1H-1,2,4-triazol-4-ium-5-yl)diazène-1-yl]-3-méthylphényl}-1,5-diazocane-1-yl)-2-méthylphényl]diazène-1-yl}-1,4-diéthyl-1H-1,2,4-triazole-4-ium,
- Sels de 5-{2-[4-(5-{4-[2-(1,4-diméthyl-1H-1,2,4-triazole-4-ium-5-yl)diazène-1-yl]-2-méthoxyphényl}-1,5-diazocane-1-yl)-3-méthoxyphényl]diazène-1-yl}-1,4-diméthyl-1H-1,2,4-triazole-4-ium,
- Sels de 5-{2-[4-(5-{4-[2-(1,4-diéthyl-1H-1,2,4-triazole-4-ium-5-yl)diazène-1-yl]-2-méthoxyphényl}-1,5-diazocane-1-yl)-3-méthoxyphényl]diazène-1-yl]-1,4-diéthyl-1H-1,2,4-triazole-4-ium,
- Sels de 5-{2-[4-(5-{4-[2-(1,4-diméthyl-1H-1,2,4-triazole-4-ium-5-yl)diazène-1-yl]-3-méthoxyphényl}-1,5-diazocane-1-yl)-2-méthoxyphényl]diazène-1-yl}-1,4-diméthyl-1H-1,2,4-triazole-4-ium,
- Sels de 5-{2-[4-(5-{4-[2-(1,4-diéthyl-1H-1,2,4-triazole-4-ium-5-yl)diazène-1-yl]-3-méthoxyphényl}-1,5-diazocane-1-yl)-2-méthoxyphényl]diazène-1-yl}-1,4-diéthyl-1H-1,2,4-triazole-4-ium,
- Sels de 5-{2-[4-(5-{4-[2-(1,4-diméthyl-1H-1,2,4-triazole-4-ium-5-yl)diazène-1-yl]-2,5-diméthyl-phényl}-1,5-diazocane-1-yl)-2,5-diméthylphényl]diazène-1-yl}-1,4-diméthyl-1H-1,2,4-triazole-4-ium et/ou
- Sels de 5-{2-[4-(5-{4-[2-(1,4-diéthyl-1H-1,2,4-triazole-4-ium-5-yl)diazène-1-yl]-2,5-diméthyl-phényl}-1,5-diazocane-1-yl)-2,5-diméthylphényl]diazène-1-yl}-1,4-diéthyl-1H-1,2,4-triazole-4-ium.

8. Agent selon l'une des revendications 1 à 7, **caractérisé en ce qu'**il contient, par rapport au poids total de l'agent, un ou plusieurs colorants directs (a) de formule (I) en une quantité totale située dans la plage allant de 0,01 à 4,5 % en poids, de préférence de 0,05 à 2,8 % en poids, de manière davantage préférée de 0,1 à 2,2 % en poids et de manière particulièrement préférée de 0,2 à 1,2 % en poids.

9. Agent selon l'une des revendications 1 à 8, **caractérisé en ce qu'**il contient (b) au moins un tensioactif anionique, choisi parmi les
- acides gras linéaires et ramifiés ayant 8 à 30 atomes de carbone,
- acides éther carboxyliques de formule R-O-(CH₂-CH₂O)ₓ-CH₂-COOM, dans laquelle
R représente un groupe alkyle linéaire ayant 8 à 30 atomes de carbone,
x représente un nombre entier situé dans la plage allant de 0 à 16,
M représente l'hydrogène ou un métal tel qu'un métal alcalin, en particulier le sodium, le potassium, le lithium, un métal alcalino-terreux, en particulier le demi-magnésium, le demi-calcium, le demi-zinc ou un ion ammonium (NH4⁺),
- acylsarcosides ayant 8 à 24 atomes de carbone dans le groupe acyle,
- acyltaurates ayant 8 à 24 atomes de carbone dans le groupe acyle,
- acyliséthionates ayant 8 à 24 atomes de carbone dans le groupe acyle, qui sont obtenus par estérification des acides gras en C₈-C₂₄ avec du sel de sodium de l'acide 2-hydroxyéthane-sulfonique (acide iséthionique),
- acides amidoéthercarboxyliques de formule R¹-CO-NR²-CH₂CH₂-O-(CH₂CH₂O)_{y}CH₂COOM, dans laquelle
R¹ représente un groupe alkyle en C2-C30,
y représente un nombre entier situé dans la plage allant de 1 à 20,
R² est un atome d'hydrogène, un groupe méthyle, éthyle, propyle, isopropyle, n-butyle, t-butyle ou isobutyle, et
M représente l'hydrogène ou un métal tel qu'un métal alcalin, en particulier le sodium, le potassium, le lithium, un métal alcalino-terreux, en particulier le demi-magnésium, le demi-calcium, le demi-zinc ou un ion ammonium (NH4⁺).

10. Agent selon l'une des revendications 1 à 9, **caractérisé en ce qu'**au moins un agent tensioactif anionique (b) du groupe des acides éthercarboxyliques de formule (B1) contient R-
O-(CH₂-CH₂O)ₓ-CH₂-COOM (B1),
dans laquelle
R représente un groupe alkyle linéaire ayant 8 à 30 atomes de carbone,
x représente un nombre entier situé dans la plage allant de 0 à 16,
M représente l'hydrogène ou un métal tel qu'un métal alcalin, en particulier le sodium, le potassium, le lithium, un métal alcalino-terreux, en particulier le demi-magnésium, le demi-calcium, le demi-zinc ou un ion ammonium (NH4⁺).

11. Agent selon l'une des revendications 1 à 10, **caractérisé en ce qu'**il contient, par rapport au poids total de l'agent, un ou plusieurs colorants directs (b) en une quantité totale située dans la plage allant de 0,05 à 9,5 % en poids, de préférence de 0,1 à 3,1 % en poids, de manière davantage préférée de 0,15 à 2,5 % en poids et de manière particulièrement préférée de 0,2 à 0,9 % en poids.

12. Agent selon l'une des revendications 1 à 11, **caractérisé en ce que** le rapport en poids de tous les agents tensioactifs anioniques de formule (B1) contenus dans l'agent à la quantité totale des agents tensioactifs anioniques contenus dans l'agent s'élève à une valeur de 0,5, de préférence de 0,6, de manière davantage préférée de 0,75 et de manière particulièrement préférée de 0,9, les agents tensioactifs anioniques de formule (B1) étant les tensioactifs suivants
R-O-(CH₂-CH₂O)ₓ-CH₂-COOM (B1),
où
R est identique à un groupe alkyle linéaire ayant 8 à 30 atomes de carbone,
x est identique à un entier situé entredans la plage allant de 0 à 16,
M est identique à l'hydrogène ou à un métal tel qu'un métal alcalin, en particulier le sodium, le potassium, le lithium, un métal alcalino-terreux, en particulier le demi-magnésium, le demi-calcium, le demi-zinc ou un ion ammonium (NH4⁺).

13. Agent selon l'une des revendications 1 à 12, **caractérisé en ce qu'**il contient, par rapport au poids total de l'agent, 0,5 à 12,5 % en poids, de préférence 2,5 à 10 % en poids et de manière particulièrement préférée 4,0 à 9,0 % en poids de peroxyde d'hydrogène.

14. Agent selon l'une des revendications 1 à 13, **caractérisé en ce qu'**il contient au moins un persulfate du groupe constitué par le peroxodisulfate d'ammonium, le peroxodisulfate de potassium et le peroxodisulfate de sodium.

15. Agent selon l'une des revendications 1 à 14, **caractérisé en ce qu'**il contient, par rapport au poids total de l'agent,
- 0,2 à 13,5 % en poids, de préférence 0,5 à 1,5 % en poids de peroxodisulfate d'ammonium,
- 0,5 à 4,5 % en poids, de préférence 1,5 à 2,5 % en poids de peroxodisulfate de potassium, et
- 0,2 à 1,8 % en poids, de préférence 0,4 à 0,8 % en poids de peroxodisulfate de sodium.
